# EUROPEAN PATENT SPECIFICATION

(11) **EP 1 595 949 B1**
(45) Date of publication and mention of the grant of the patent: **22.06.2011**
(21) Application number: 03809435.5
(22) Date of filing: 17.09.2003
(51) Int. Cl.: C12N 15/09, C08J 11/10, C08J 11/18, B29B 17/00, C12N 1/00, C12N 1/15, C12N 9/30, C12N 15/55, C12R 1/465, C12R 1/66, C12R 1/685, C12R 1/69

(54) **METHOD OF DEGRADING PLASTIC AND PROCESS FOR PRODUCING USEFUL SUBSTANCE USING THE SAME**
VERFAHREN ZUM ABBAU VON KUNSTSTOFF UND HERSTELLUNGSPROZESS FÜR EINEN NÜTZLICHEN STOFF UNTER VERWENDUNG DAVON
PROCEDE DE DEGRADATION DU PLASTIQUE ET PROCEDE DE PRODUCTION DE SUBSTANCE UTILE DANS LEQUEL LEDIT PROCEDE EST UTILISE

(30) Priority: 23.10.2002 JP 2002308884; 24.12.2002 JP 2002371246
(43) Date of publication of application: 16.11.2005
(73) Proprietor: Tohoku Techno Arch Co., Ltd., Sendai-shi, Miyagi 980-8577 (JP); National Institute of Advanced Industrial Science and Technology, Tokyo 100-8921 (JP)
(72) Inventor: ABE, Keietsu, Shiogama-shi, Miyagi 985-0043 (JP); GOMI, Katsuya, Sendai-shi, Miyagi 989-3201 (JP); YAMAGATA, Yohei, Sendai-shi, Miyagi 981-0935 (JP); HASEGAWA, Fumihiko, Semdai-shi, Miyagi 981-0911 (JP); MAEDA, Hiroshi, Room 203, Rest Famil, Sendai-shi, Miyagi 981-0931 (JP); NAKAJIMA, Tasuku, Sendai-shi, Miyagi 981-3122 (JP); MACHIDA, M, Nat.Inst.of Adv.Indust.Science&Techn., 1-1, Higashi 1-chome, Tsukuba-shi (JP)
(74) Representative: Woods, Geoffrey Corlett
(86) International application number: PCT/JP2003/011861
(87) International publication number: WO 2004/038016

(56) References cited:
- WO-A1-01/14524
- WO-A1-98/36086
- JP-A- 10 096 174
- JP-A- 10 306 098
- JP-A- 11 216 355
- JP-A- 52 082 773
- TAKAHASHI TORU ET AL: "The fungal hydrophobin RolA recruits polyesterase and laterally moves on hydrophobic surfaces" MOLECULAR MICROBIOLOGY, vol. 57, no. 6, September 2005 (2005-09), pages 1780-1796, XP002355614 ISSN: 0950-382X
- TETSUYA DEGUCHI: 'Lignin Bunkaikin (IZU-154 Kabu) ni yoru Nylon oyobi Polyethylene no Seibunkai' POLYMER PREPRINTS vol. 42, no. 2, 1993, JAPAN, page 427, XP002976222
- ZANGI R. ET AL.: 'Molecular dynamics study of the folding of hydrophobin SC3 at a hydrophilic/hydrophobic interface' BIOPHYS J. vol. 83, no. 1, 2002, pages 112 - 124, XP002976223
- LUGONES LG. ET AL.: 'A hydrophobin (ABH3) specifically secreted by vegetatively growing hyphae of Agaricus bisporus(common white button mushroom)' MICROBIOLOGY vol. 144, no. 8, 1998, pages 2345 - 2353, XP002976224
- WOSTEN H. ET AL.: 'Interfacial self-assembly of a fungal hydrophobin into a hydrophobic rodlet layer' PLANT CELL vol. 5, no. 11, 1993, pages 1567 - 1574, XP002976225
- STRINGER MA. ET AL.: 'dewA encodes a fungal hydrophobin component of the Aspergillus spore wall' MOL. MICROBIOL vol. 16, no. 1, 1995, pages 33 - 44, XP000770242
- PARTA M. ET AL.: 'HYP1, a hydrophobin gene from Aspergillus fumigatus, complements the rodletless phenotype in Aspergillus nidulans' INFEST.IMMUN vol. 62, no. 10, 1994, pages 4389 - 4395, XP000770240

### SEQUENCE LISTING

<110> Tohoku Techno Arch Co. Ltd. and National Institute of Advanced Industrial Science and Technology
<120> Method of Degrading Plastic and Process for Producing Useful Substance Using the Same
<130> N.94531 GCW/SJB
<140> EP 03809435.5
   <141> 2003-09-17
<150> PCT/JP03/11861
   <151> 2003-09-17
<150> JP 2002-308884
   <151> 2002-10-23
<150> JP 2002-371246
   <151> 2002-12-24
<160> 7
<170> PatentIn version 3.1
<210> 1
   <211> 497
   <212> DNA
   <213> Aspergillus oryzae
<220>
   <221> CDS
   <222> (1)..(453)
   <223>
<400> 1
   atg cag ttc tcc gtc gcc gct gtt ctt gct ctg gct act gcc gtt gcc       48
   Met Gln Phe Ser Val Ala Ala Val Leu Ala Leu Ala Thr Ala Val Ala
   1               5                   10                  15
   gct ctt cct cct gcc tct ggc act ggc gct ggc cag caa gtc gga cac       96
   Ala Leu Pro Pro Ala Ser Gly Thr Gly Ala Gly Gln Gln Val Gly His
               20                  25                  30
   tcc aag aac gac ttc cct ctc cct aag gag ttg acc acc aag cag gcc      144
   Ser Lys Asn Asp Phe Pro Leu Pro Lys Glu Leu Thr Thr Lys Gln Ala
           35                  40                  45
   gcc gac aag tgt ggt gac cag gct cag ctc acc tgc tgc aac aag acc      192
   Ala Asp Lys Cys Gly Asp Gln Ala Gln Leu Thr Cys Cys Asn Lys Thr
       50                  55                  60
   gtc aag acc ggt gac ttc acc cag gtt gag gag ggt ctc ctt gct ggc      240
   Val Lys Thr Gly Asp Phe Thr Gln Val Glu Glu Gly Leu Leu Ala Gly
   65                  70                  75                  80
   ctc ctc tcc aac ctc ctc ggt gcc gga cag ggc tcc cag ggt ctt ggt      288
   Leu Leu Ser Asn Leu Leu Gly Ala Gly Gln Gly Ser Gln Gly Leu Gly
                   85                  90                  95
   ctc ttg gat gag tgc acc aac atc cct gtt atc ccc atc atc tcc atc      336
   Leu Leu Asp Glu Cys Thr Asn Ile Pro Val Ile Pro Ile Ile Ser Ile
               100                 105                 110
   gcc tct cct cag gag aag tgc aag cag ccc atc tct tgc tgc cag aac      384
   Ala Ser Pro Gln Glu Lys Cys Lys Gln Pro Ile Ser Cys Cys Gln Asn
           115                 120                 125
   acc aag tcc agc gcc gat ggc gac ctc gtc ggt att ggt ctt cct tgc      432
   Thr Lys Ser Ser Ala Asp Gly Asp Leu Val Gly Ile Gly Leu Pro Cys
       130                 135                 140
   atc gct ctc ggc tct ctc ctg taagcgattg cattcgcgaa aatggtagct         483
   Ile Ala Leu Gly Ser Leu Leu
   145                 150
   cgaggagcac ggga                                                       497
<210> 2
   <211> 438
   <212> DNA
   <213> Aspergillus oryzae
<220>
   <221> CDS
   <222> (1)..(438)
   <223>
<400> 2
   atg aag ttc ttc gct gtc gct gct ctc ttc gcc acc gcc gcc atg gcc      48
   Met Lys Phe Phe Ala Val Ala Ala Leu Phe Ala Thr Ala Ala Met Ala
   1               5                   10                  15
   gct cct ggt tcc gct cct gtg ccc ggt gct gct gct gct gcc ggc aac      96
   Ala Pro Gly Ser Ala Pro Val Pro Gly Ala Ala Ala Ala Ala Gly Asn
               20                  25                  30
   ggt aac gcc ccg gtt atc aac cag acc cag cag aag gcc ttc aca gac     144
   Gly Asn Ala Pro Val Ile Asn Gln Thr Gln Gln Lys Ala Phe Thr Asp
           35                  40                  45
   gct tgc agt gct aag ggc aac cac ccc gtc tgc tgc gac cag atc gac     192
   Ala Cys Ser Ala Lys Gly Asn His Pro Val Cys Cys Asp Gln Ile Asp
       50                  55                  60
   acc tcc aag acc aca act gtg aac gag gga ctt ctc ggt ggc ctc ctc     240
   Thr Ser Lys Thr Thr Thr Val Asn Glu Gly Leu Leu Gly Gly Leu Leu
   65                  70                  75                  80
   ggt gag ggc ctg ggt ggc gtt ctt aac aac ctt gtt ggt ggc gag cct     288
   Gly Glu Gly Leu Gly Gly Val Leu Asn Asn Leu Val Gly Gly Glu Pro
                   85                  90                  95
   ggc gct tgc agt ggt ctt gtt tct gcc ctc aac aag caa tgc cag acc     336
   Gly Ala Cys Ser Gly Leu Val Ser Ala Leu Asn Lys Gln Cys Gln Thr
               100                 105                 110
   agc atc ggt tgc tgc cag cag aac gcc aag ggc gac aac tac cag agc     384
   Ser Ile Gly Cys Cys Gln Gln Asn Ala Lys Gly Asp Asn Tyr Gln Ser
           115                 120                 125
   ggc ctc ctc aac ctc aac ctc cag gct cct tgc ctt ctc agc aat ggt     432
   Gly Leu Leu Asn Leu Asn Leu Gln Ala Pro Cys Leu Leu Ser Asn Gly
       130                 135                 140
   ctg taa                                                             438
   Leu
   145
<210> 3
   <211> 318
   <212> DNA
   <213> Aspergillus oryzae
<220>
   <221> CDS
   <222> (1)..(318)
   <223>
<400> 3
   atg aag gtt tct acc ttg ttg acc acc ctt tgc gta ctg ggt gtt gct      48
   Met Lys Val Ser Thr Leu Leu Thr Thr Leu Cys Val Leu Gly Val Ala
   1               5                   10                  15
   agt tcg gcg gat cca acc gca caa tgc gat aac gga ccc gtt caa tgc      96
   Ser Ser Ala Asp Pro Thr Ala Gln Cys Asp Asn Gly Pro Val Gln Cys
               20                  25                  30
   tgc gca acg gtc ggt tta ccg act gac agt gta gtc tca cct tta ctt      144
   Cys Ala Thr Val Gly Leu Pro Thr Asp Ser Val Val Ser Pro Leu Leu
           35                  40                  45
   ggc ttg ctc gga gta gtt gtt cca gac atg agc act cca gtt gga ttg      192
   Gly Leu Leu Gly Val Val Val Pro Asp Met Ser Thr Pro Val Gly Leu
       50                  55                  60
   acc tgt aat cct att gtc cag ggt gga tct tgc cca ggc cac ccc gtt      240
   Thr Cys Asn Pro Ile Val Gln Gly Gly Ser Cys Pro Gly His Pro Val
   65                  70                  75                  80
   tgc tgt aca gga aat gta ggc cat gct tct gcg aca cat tgt tcc agc      288
   Cys Cys Thr Gly Asn Val Gly His Ala Ser Ala Thr His Cys Ser Ser
                   85                  90                  95
   tac cta atg tcc atc gga aat gat tac tga                              318
   Tyr Leu Met Ser Ile Gly Asn Asp Tyr
               100                 105
<210> 4
   <211> 675
   <212> DNA
   <213> Aspergillus oryzae
<220>
   <221> CDS
   <222> (1)..(675)
   <223>
<400> 4
   atg gtg atg ctc cgc tcc ctc ctc gtc tct gcc ctg gct gct ctg gca      48
   Met Val Met Leu Arg Ser Leu Leu Val Ser Ala Leu Ala Ala Leu Ala
   1               5                   10                  15
   gca ggc agc ccc atc gct gag cca gcg gac cag tca ctg gaa gcc aga        96
   Ala Gly Ser Pro Ile Ala Glu Pro Ala Asp Gln Ser Leu Glu Ala Arg
               20                  25                  30
   cag ctc ggg tct tcc aat gac ctc acc aac ggt gca tgc aag gat gtg       144
   Gln Leu Gly Ser Ser Asn Asp Leu Thr Asn Gly Ala Cys Lys Asp Val
           35                  40                  45
   acc ctc att ttc gct cga ggc tcc acc gaa atg ggc aac atg ggc act       192
   Thr Leu Ile Phe Ala Arg Gly Ser Thr Glu Met Gly Asn Met Gly Thr
       50                  55                  60
   gtc atc gga ccc cct ctc tgc tcg tcg ctg aag agc aag ctc ggt gcc       240
   Val Ile Gly Pro Pro Leu Cys Ser Ser Leu Lys Ser Lys Leu Gly Ala
   65                  70                  75                  80
   gac aag gtc gct tgc caa ggt gtg ggt ggc ttg tac aca gga gga ttg       288
   Asp Lys Val Ala Cys Gln Gly Val Gly Gly Leu Tyr Thr Gly Gly Leu
                   85                  90                  95
   atg caa aat gct ctc cct cag aac acc gac ccc ggc gct atc tcc acc       336
   Met Gln Asn Ala Leu Pro Gln Asn Thr Asp Pro Gly Ala Ile Ser Thr
               100                 105                 110
   gcg aag tcg ctc ttt gaa caa gcc agc acc aag tgc cct aac acc cag       384
   Ala Lys Ser Leu Phe Glu Gln Ala Ser Thr Lys Cys Pro Asn Thr Gln
           115                 120                 125
   atc gtg gcc ggt gga tac agt caa ggt agt gcc gtc atc gac aac gcc       432
   Ile Val Ala Gly Gly Tyr Ser Gln Gly Ser Ala Val Ile Asp Asn Ala
       130                 135                 140
   gtc caa caa ctc tcc gcc gaa gtc aag gac aag gtc aag ggt gtt gtt       480
   Val Gln Gln Leu Ser Ala Glu Val Lys Asp Lys Val Lys Gly Val Val
   145                 150                 155                 160
   ttc ttc ggc ttc act cgt aac ctc cag gac aag ggt cag atc ccc aac       528
   Phe Phe Gly Phe Thr Arg Asn Leu Gln Asp Lys Gly Gln Ile Pro Asn
                   165                 170                 175
   tac ccc aag gac aat gtt aag gtc ttt tgt gcg atg gga gat ctt gtc       576
   Tyr Pro Lys Asp Asn Val Lys Val Phe Cys Ala Met Gly Asp Leu Val
               180                 185                 190
   tgc gat ggc acg ctt att gtc acc gcc gcc cat ttg aca tat acc att       624
   Cys Asp Gly Thr Leu Ile Val Thr Ala Ala His Leu Thr Tyr Thr Ile
           195                 200                 205
   aat gcg ccg gag gct gcc tct ttc ctt gcg tcg aag gtt cag tca gct       672
   Asn Ala Pro Glu Ala Ala Ser Phe Leu Ala Ser Lys Val Gln Ser Ala
       210                 215                 220
   tag                                                                   675
<210> 5
   <211> 780
   <212> DNA
   <213> Aspergillus oryzae
<220>
   <221> CDS
   <222> (1)..(780)
   <223>
<400> 5
   atg aat ctc cgc ctg ttg acc ctt gct ctc gct gct gtg gcg gct gca        48
   Met Asn Leu Arg Leu Leu Thr Leu Ala Leu Ala Ala Val Ala Ala Ala
   1               5                   10                  15
   agt cca gta gat atc cag gaa cgc cag ctc tcc ggc gga aac gag ctg        96
   Ser Pro Val Asp Ile Gln Glu Arg Gln Leu Ser Gly Gly Asn Glu Leu
               20                  25                  30
   cga gat ggc tct tgc aag ccg atc acc ttc atc ttt gcc cgc gct tcc       144
   Arg Asp Gly Ser Cys Lys Pro Ile Thr Phe Ile Phe Ala Arg Ala Ser
           35                  40                  45
   aca gag cca gga ctt ttg ggt ata tca act ggc cct gca gtc tgc aat       192
   Thr Glu Pro Gly Leu Leu Gly Ile Ser Thr Gly Pro Ala Val Cys Asn
       50                  55                  60
   ggc ttg aag atg gcc aaa gcg ggc caa gta gct tgc cag ggt gtt gga       240
   Gly Leu Lys Met Ala Lys Ala Gly Gln Val Ala Cys Gln Gly Val Gly
   65                  70                  75                  80
   ccc aag tac acg gca gac ctc gca tca aat gct ttg ccc gag aac acg       288
   Pro Lys Tyr Thr Ala Asp Leu Ala Ser Asn Ala Leu Pro Glu Asn Thr
                   85                  90                  95
   tcc cca gct gcc atc caa gag gca caa gac ctg ttc cag cag gcc gtc       336
   Ser Pro Ala Ala Ile Gln Glu Ala Gln Asp Leu Phe Gln Gln Ala Val
               100                 105                 110
   acc aaa tgc cct gac acc caa att gtt gcc ggt ggt tat agc caa ggc       384
   Thr Lys Cys Pro Asp Thr Gln Ile Val Ala Gly Gly Tyr Ser Gln Gly
           115                 120                 125
   aca gcc gtg atg gat gac tcc atc aag cgt cta cca gac aac gtg aaa       432
   Thr Ala Val Met Asp Asp Ser Ile Lys Arg Leu Pro Asp Asn Val Lys
       130                 135                 140
   gag aag atc aag ggt gtc gtg ctc ttc ggc tac acc cgc aac gca cag       480
   Glu Lys Ile Lys Gly Val Val Leu Phe Gly Tyr Thr Arg Asn Ala Gln
   145                 150                 155                 160
   gaa cac ggt cag att gcc aac ttt ccc aag gac aag gtc aag gtc tac       528
   Glu His Gly Gln Ile Ala Asn Phe Pro Lys Asp Lys Val Lys Val Tyr
                   165                 170                 175
   tgt gcc gta ggc gat atg gtc tgt gac ggc acc ttg atc gtg ggg ccc       576
   Cys Ala Val Gly Asp Met Val Cys Asp Gly Thr Leu Ile Val Gly Pro
               180                 185                 190
   gcc cat ttc act tac ctg ggt aac acc ggc gaa gca act cag ttc ttg       624
   Ala His Phe Thr Tyr Leu Gly Asn Thr Gly Glu Ala Thr Gln Phe Leu
           195                 200                 205
   ctt ggt aag ctg agt gct tcg tct tcg tct tcg tct tca tcc ggt tct       672
   Leu Gly Lys Leu Ser Ala Ser Ser Ser Ser Ser Ser Ser Ser Gly Ser
       210                 215                 220
   tcg gat act tcg tcc gca tca aca tcc gct gct gct gac tct tct tcg       720
   Ser Asp Thr Ser Ser Ala Ser Thr Ser Ala Ala Ala Asp Ser Ser Ser
   225                 230                 235                 240
   tct tcg tct tct tca tcg tcg cct ttt ggg aat ctt ggt aat ctc ttt      768
   Ser Ser Ser Ser Ser Ser Ser Pro Phe Gly Asn Leu Gly Asn Leu Phe
                   245                     250                 255
   ggt gga ctc tag                                                      780
   Gly Gly Leu
<210> 6
   <211> 525
   <212> DNA
   <213> Aspergillus oryzae
<220>
   <221> CDS
   <222> (1)..(525)
   <223>
<400> 6
   atg ctt gcc aaa cac gtc ctt gct gtc ctt ctg tct gtt ggc gct tcc        48
   Met Leu Ala Lys His Val Leu Ala Val Leu Leu Ser Val Gly Ala Ser
   1               5                   10                  15
   gcc att cct ttc gac aag cga gat gca tcc gct gtc ctc gct gat ttt        96
   Ala Ile Pro Phe Asp Lys Arg Asp Ala Ser Ala Val Leu Ala Asp Phe
               20                  25                  30
   aac aca ctc tca acc gac ctg tcg gct ctc ggc tct gct att tcg agc       144
   Asn Thr Leu Ser Thr Asp Leu Ser Ala Leu Gly Ser Ala Ile Ser Ser
           35                  40                  45
   ttc gat gga acg ctc aat ggt gcc ttg ggc gtc cag cag aag gaa ggc       192
   Phe Asp Gly Thr Leu Asn Gly Ala Leu Gly Val Gln Gln Lys Glu Gly
       50                  55                  60
   caa gta gag acg gcg ttg aag caa acc gtc agc gat gtg aaa gcg tct       240
   Gln Val Glu Thr Ala Leu Lys Gln Thr Val Ser Asp Val Lys Ala Ser
   65                  70                  75                  80
   act gcg ttc agt gct gct gac agc aca agc gtg acc aat gct gtg act       288
   Thr Ala Phe Ser Ala Ala Asp Ser Thr Ser Val Thr Asn Ala Val Thr
                   85                  90                  95
   ggt ctg gag cct agc att gtg aac gtt ctc aac gat ctc gtt tcc aag       336
   Gly Leu Glu Pro Ser Ile Val Asn Val Leu Asn Asp Leu Val Ser Lys
               100                 105                 110
   aaa tcc ggt ttt gac tct gtt ggc gtt acc agc att gtc gta tcg gat       384
   Lys Ser Gly Phe Asp Ser Val Gly Val Thr Ser Ile Val Val Ser Asp
           115                 120                 125
   ctc aac tcc ctc cat gat ctt act ggc cag ctg tca act gag ctc cag       432
   Leu Asn Ser Leu His Asp Leu Thr Gly Gln Leu Ser Thr Glu Leu Gln
       130                 135                 140
   tcg aag gtc act tcg ggt gac gca tcc act att tct gat gag gct gcg       480
   Ser Lys Val Thr Ser Gly Asp Ala Ser Thr Ile Ser Asp Glu Ala Ala
   145                 150                 155                 160
   cga ctt gat gcg gaa tac aag aag gcc atc gct gcc tat tcc tag           525
   Arg Leu Asp Ala Glu Tyr Lys Lys Ala Ile Ala Ala Tyr Ser
                   165                 170
<210> 7
   <211> 561
   <212> DNA
   <213> Aspergillus oryzae
<220>
   <221> CDS
   <222> (1)..(561)
   <223>
<400> 7
   atg cac ttc cac cgt ctc ctg gtc ttg gcc gcc ggg ctc ctg aca acg       48
   Met His Phe His Arg Leu Leu Val Leu Ala Ala Gly Leu Leu Thr Thr
   1               5                   10                  15
   gca aca tca gca ccc acc gaa ctc ttc aaa cgc gat gcc gca ggc gta       96
   Ala Thr Ser Ala Pro Thr Glu Leu Phe Lys Arg Asp Ala Ala Gly Val
               20                  25                  30
   gta gat gct gtg gca gag atc gct gac aaa atg acg acc cta aat acc      144
   Val Asp Ala Val Ala Glu Ile Ala Asp Lys Met Thr Thr Leu Asn Thr
           35                  40                  45
   aca gtc acg ggt tac caa ggc gga gtg ctc gga acc gga aca gcc ctg      192
   Thr Val Thr Gly Tyr Gln Gly Gly Val Leu Gly Thr Gly Thr Ala Leu
       50                  55                  60
   aag att gag ttc cag tcc atc caa ctc agc cac gcc ctg aaa gat gcc      240
   Lys Ile Glu Phe Gln Ser Ile Gln Leu Ser His Ala Leu Lys Asp Ala
   65                  70                  75                  80
   atc tcg acc act gaa gat tcc caa aac ttc acc ggc gat gag tcc aat      288
   Ile Ser Thr Thr Glu Asp Ser Gln Asn Phe Thr Gly Asp Glu Ser Asn
                   85                  90                  95
   aag gtt gcc gct gct ttt att gat ctg caa ccc aag atc tct tca act      336
   Lys Val Ala Ala Ala Phe Ile Asp Leu Gln Pro Lys Ile Ser Ser Thr
               100                 105                 110
   ctc aat aac att gtc tcc aag aaa cct cag ttc gac aca ggg ctc ctc      384
   Leu Asn Asn Ile Val Ser Lys Lys Pro Gln Phe Asp Thr Gly Leu Leu
           115                 120                 125
   ggc atc ggg tcc gtc tca ttc ttg gtc aag tgg aac ttg caa cag gag      432
   Gly Ile Gly Ser Val Ser Phe Leu Val Lys Trp Asn Leu Gln Gln Glu
       130                 135                 140
   aag gac ttg tcg gcg gac ttg ggg cag gct gtt gtt gct aag ctg gcg      480
   Lys Asp Leu Ser Ala Asp Leu Gly Gln Ala Val Val Ala Lys Leu Ala
   145                 150                 155                 160
   gag ccg tat gcc tcg gtt gcg cca ttg ctg aac gat cag att gcc gct      528
   Glu Pro Tyr Ala Ser Val Ala Pro Leu Leu Asn Asp Gln Ile Ala Ala
                   165                 170                 175
   gct ttt gag aag gca ctt gct gct tat aat tag                          561
   Ala Phe Glu Lys Ala Leu Ala Ala Tyr Asn
               180                 185

## Description

### [Technical Field]

This invention is related to a method of degrading plastic in the presence of a plastic-binding protein, especially to a method of degrading of plastic with the use of *Aspergillus oryzae* or *Aspergillus sojae,* to a method of degrading biodegradable plastic by making the plastic in contact with a microorganism and converting carbon in the plastic into a useful substance, and to various proteins and genes encoding them used in the above methods.

### [Background]

According to the statistics of 1997, an amount of plastic demanded in Japan amounted to 14-15 millions of tons and more than 9 millions of tons of them were discharged as discarded plastic. It is therefore required to cope with such discarded plastic in view of an environmental aspect and reduction in consumption of fossil fuel ("Basic knowledge about recycle of plastics", Incorporated Association of Plastic Waste Management Institute). Biodegradable plastics have been developed as a countermeasure against these problems in order to reduce the generation of carbon dioxide gas and dioxins due to the burnout of petrochemical plastics and to suppress the discharge of non-biodegradable plastics into the environment. It is estimated that an amount of the biodegradable plastics circulated in the market will be 500 thousands - one million tons per year, i.e., about 3∼7 % of the total plastics ("Scenario of increase of the market of biodegradable plastics", Ed., Fuji Chimera General Institute p.23). However, it is pointed out that a capacity of the present technology for the degradation of biodegradable plastics in natural soil is limited and the cost for the production of the biodegradable plastics is relatively high.

Eukaryotic filamentous fungi, especially *Aspergillus* genus including *Aspergillus oryzae,* have been utilized in a brewing industry in Japan for a long time in order to produce sake, soybean paste, soy sauce, and Japanese sweet rice wine for cooking. They are therefore considered safe genetic resources and are actually listed as Generally Recognized as Safe (GRAS) in Department of Agriculture in USA (USDA).

As the *Aspergillus* fungi are superior in their ability of secreting proteins outside their fungal forms, they are used in the production of various useful substances.

Patent Document 1 discloses a method of degrading polybutylene succinate resin with the use of a species of *Actinomycetes, Amycolatopsis mediterranei* HT-6.

Patent Document 2 discloses a method for degrading biodegradable plastic with the use of enzymes such as lipase and cutinase.

These documents, however, neither describe the coexistence of a biosurfactant in a reaction system, nor suggest the production of useful substances other than degraded components of the plastic.
Patent Document 1: Japanese Patent Publication Hei 9 (1997) - 25271
Patent Document 2: Japanese Patent Publication 2001 - 512504

The present technology used in degradation of the biodegradable plastic is based on a simple degradation into water and carbon dioxide by means of microorganisms in soil, but not intend to convert it into substances having useful added values. Such situation can be seen worldwide. The cost for the production of the biodegradable plastic is 3-5 times higher than that of petrochemical plastic ("The present ('01) situation and a new development of biodegradable plastics", Ed. of DIA RESEARCH MARTECH INC. and Chuo Research Center Co., Ltd., p.513-521). It is therefore required to reduce the cost for the production of the biodegradable plastic, or to develop any means for recovering the cost.

Furthermore, degradation of the biodegradable plastic is now carried out only in an area in the vicinity of surface of earth (30 -50 cm in depth). However, as density of microorganisms in soil is low, and is much lower in deeper area of the soil, the rate of the degradation of the biodegradable plastic in soil shall be slow. If the demand of the biodegradable plastic is increased as expected in the future, it will be impossible to deal with the load of the discarded plastic in a large scale only by means of the above degradation in soil. Further, it would be difficult to secure a huge space for reclamation in such a small country as Japan, especially in city areas where a large amount of amount of the plastic is discarded. The biodegradable plastic is now already used as packing material, agricultural material, packing material for compost. As it will be further utilized in mass production items such as interior parts in cars, packing material for computer and home electric appliances, a facility will be necessary for a highly efficient treatment of the biodegradable plastic. According to questionnaire about the use of the biodegradable plastic, it is pointed out that the problems are the establishment of infrastructure for a large-scaled facility as well as the high cost ("The present ('01) situation and a new development of biodegradable plastics", Ed. of DIA RESEARCH MARTECH INC. and Chuo Research Center Co., Ltd., p.513-521).

The present inventors have intensively studied the above problems, and invented a novel method where by using a microorganism producing a surfactant such as a biosurfactant and a plastic-degrading enzyme, or these substances themselves, useful substances such as enzymes and antibiotics may be produced by a microorganism (filamentous fungi or *Actinomycetes*) having a high productivity of the useful substances simultaneously with performing the degradation of plastics with a high density in a large scale

### [Summary of the Invention]

This invention is based on the findings that a biosurfactant such as a plastic-binding protein attaches to a hydrophobic surface of plastic and effectively promotes degradation of the plastic in cooperation with a plastic-degrading enzyme.

The present invention provides a transformant prepared by recombination with the use of a DNA comprising a gene encoding a biosurfactant which is a plastic binding protein and a DNA comprising a gene encoding a plastic-degrading enzyme which is a cutinase.

The invention also provides a method of degrading biodegradable plastic using such a transformant.

The invention also provides a method of producing a useful substance from biodegradable plastic comprising degrading the plastic using the transformant of the invention and further converting a component of the degraded plastic into a useful substance.

The present invention further provides a method degrading biodegradable plastic which is characterized by co-culture of a transformant prepared by recombination with the use of a DNA comprising a gene encoding a biosurfactant which is a plastic binding protein, and a transformant prepared by recombination with the use of a DNA comprising a gene encoding a plastic-degrading enzyme which is a cutinase.

Also provided is a method of producing a useful substance from biodegradable plastic comprising degrading the plastic and further converting a component of the degraded plastic into a useful substance, the method being characterized by using triple co-culture of: (i) a transformant prepared by recombination with the use of a DNA comprising a gene encoding a biosurfactant which is a plastic binding protein; (ii) a transformant prepared by recombination with the use of a DNA comprising a gene encoding a plastic-degrading enzyme which is a cutinase; and (iii) a transformant prepared by recombination with the use of a DNA comprising a gene encoding an enzyme involved in biosynthesis.

Also referred to is a method of degrading plastic in the presence of a biosurfactant such as a plastic-binding protein, especially to the method with the use of a plastic-degrading enzyme

A second aspect of the present invention relates to a method of producing a useful substance from plastic comprising degrading the plastic by making the plastic in contact with a microorganism and further converting a component of the degraded plastic into a useful substance with the use of a microorganism.

The above method is useful as a recycling system where waste plastics are degraded into a substrate available for fermentation by a microorganism, which will then converted into a useful substance such as an enzyme, medicine, chemical product with a high added value.

There is no limitation on the kind of the useful substance, which includes any material known to those skilled in the art, such as a protein involved in a biosynthesis such as an enzyme, first metabolite (e.g., organic acid), second metabolite (e.g., antibiotics and precursor of the chemical products), and biosurfactant. Especially, a useful substance that is extracellularly secreted may be easily and advantageously subjected to a post-production operation such as purification in view of effectiveness and economical efficiency. Further, monomers and oligomers which are obtained as a result of the degradation of the plastic will be useful as a starting material for reproduction of plastics, and are therefore included in the useful substance of the present invention.

The microorganism that degrades the plastic and that converts the degraded plastic into the useful substance may be the same or different from each other.

The microorganism that may be used for converting the degraded plastic into the useful substance includes, for example, a variant highly expressing the useful substance, which may be obtained by mutation with radiation of ultraviolet, treatment with an agent such as N-methyl-N'-nitor-N-nitrosoguanidine (NTG). Furthermore, it is preferable to use a transformant highly expressing various useful substances such as the enzyme involved in a biosynthesis, which has been obtained by recombination with the use of a gene encoding the above substances.

A third aspect of the present invention relates to a method of degrading plastic by making the plastic in contact with a microorganism in the coexistence of a biosurfactant and/or a plastic-degrading enzyme, and degrading the plastic with the use of the microorganism.

In a further aspect, the present invention provides for the user of a transformant prepared by recombination with the use of a DNA comprising a gene encoding a biosurfactant which is a plastic binding protein or a DNA comprising a gene encoding a plastic degrading enzyme which is a cutinase in a method of degrading biodegradable plastic with the plastic degrading enzyme in the presence of the biosurfactant.

There is no limitation on the microorganism used in the above aspects of the present invention as long as it is capable of degrading the plastic or converting the degraded plastic to produce the useful substance, filamentous bacteria and eucaryotic filamentous fungi being mentioned as a representative example of the above microorganism.

The filamentous bacteria include *Actinomycetes* such as *Streptomyces* genus, for example, *Streptomyces griseus,* and *Streptomyces sericara.*

The eucaryotic filamentous fungi include genera of *Aspergillus, Penicillium, Trichodera, Rhizopus, Mucor, Humicola, Magnaporthe, Metarhisium, Neurospora, Monascus, Acremonium* and *Fusarium. Aspergillus genus* includes *Aspergillus oryzae, Aspergillus soyae,* and *Aspergillus nidulans.*

In one instance, a transformant of the invention is a eukaryotic filamentous fungus selected from the genera of *Aspergillus, Penicillium, Trichodera, Rhizopus, Magnaporthe, Metarhisium, Neurospora, Monascus, Acremonium* and *Mucor.* In one instance, the transformant is of the genus *Aspergillus.* In another instance, it is *Aspergillus oryzae*

The *Aspergillus oryzae* RIB 40 strain was deposited at the International Patent Organism Depository of National Institute of Advanced Industrial Science and Technology (1-3, Higashi 1-chome, Tsukuba-shi, Ibaraki-ken 305-8566 JAPAN) on March 28, 2001 with an acceptance No. FERM P-18273.

The *Aspergillus oryzae* pN-cha strain (a strain highly triple-coexpressing cutinase, hydrophobin and amylase together) was deposited on September 11, 2003 under the terms of the Budapest Treaty on the International Recognition of the Deposit of Microorganisms for the Purposes of Patent Procedure and Regulation under Accession No. FERM BP-08486 with the International Patent Organism Depository, the National Institute of Advanced Industrial Science and Technology.

Other various transformants that may be obtained by using a genetic engineering known to those skilled in the art from the above filamentous bacteria and eucaryotic filamentous fungi may be also advantageously utilized in the present invention.

The transformants may be prepared by recombination with the use of DNA comprising at least one DNA selected from the group consisting of DNA comprising a gene encoding the biosurfactant or plastic-binding protein such as hydrophobin according to the present invention, DNA comprising a gene encoding the plastic-degrading enzyme which is a cutinase, and DNA comprising a gene encoding the useful substance (for example, enzymes involved in production of the useful substances by biosynthesis, degradation of high-molecular substances into low molecular ones or various translocation reaction, such as hydroxilase, oxidase, polyketide synthetase, α-amylase, cellulase, lipase and dportease). The above transformants are capable of highly expressing the surfactant, plastic-degrading enzyme and/or the enzymes involved in the biosynthesis of the useful substances.

A further aspect of the present invention therefore relates to the above transformants.

A microorganism which may be used for the preparation of the above transformants includes *Aspergillus niger, Aspergillus awamori, Aspergillus kawachii, Aspergillus fumigatus, Aspergillus flavus, Aspergillus parasiticus Aspergillus nomius, Aspergillus tamari* and *Aspergillus repens* in addition to the above-mentioned microorganisms.

The present invention also relates to a novel plastic-binding protein and plastic-degrading enzyme which may be used in the above methods, and a gene encoding these substances, especially those derived from *Aspergillus oryzae.*

### [Explanation of Drawings]

Figure 1 is a photo showing a PBS-converting capability of *Aspergillus* filamentous fungi such as *A. oryzae.*
Figure 2 is a photo showing a PLA-converting capability of *Aspergillus* filamentous fungi such as *A. oryzae.*
Figure 3 shows a combination of culture media for a DNA microarray analysis.
Figure 4 shows schematically a group of genes that are specifically expressed in the presence of PBS.
Figure 5 shows a result of visual analysis of DNA microarray of Su3PP5, Su3PE5 and Su3Bu5, in which each number means a ratio of Cy5/Cy3 in gene expression.
Figure 6 shows a base sequence of JZ3981 comprising a full length of hydrophobin.
Figure 7 shows schematically the construction of a high-expression plasmid of hydrophobin for *Aspergillus* strain.
Figure 8 is a photo of SDS-PAGE showing that the hydrophobin high-expression Aspergillus strain secretes the hydrophobin into a culture medium.
Figure 9 a photo of SDS-PAGE (left) showing that a protein is detected only in a supernatant from a culture medium of the *Aspergillus oryzae* transformant having PBS-degrading enzyme (cutinase) gene inserted in its genomic DNA at the same position as a purified sample of the PBS-degrading enzyme, and a photo (right) showing degradation of PBS by the same transformant.
Figure 10 shows the construction of pPTR-gla-hyp having glaA 142 promoter fused upstream of a desired gene.

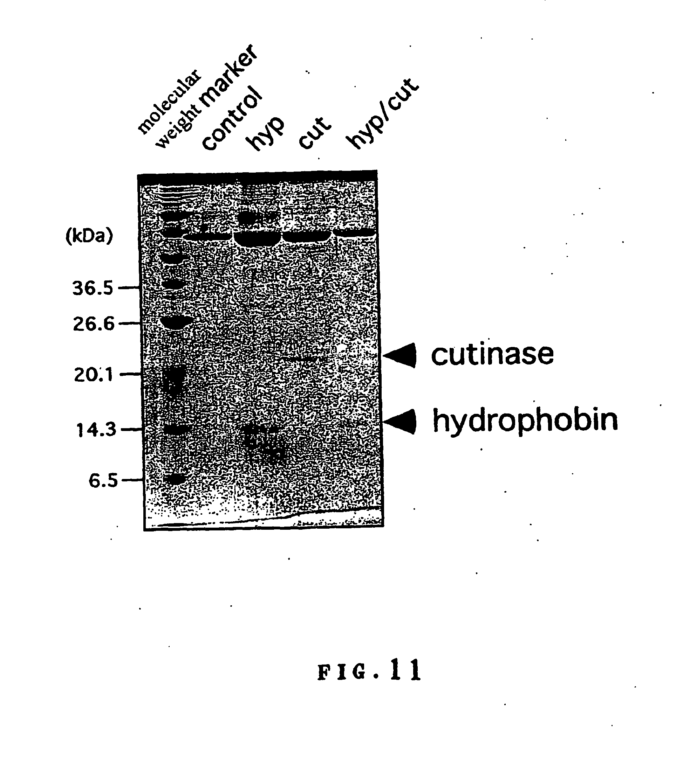

Figure 11 is a photo of SDS-PAGE showing the preparation of a strain highly coexpressing cutinase and hydrophobin.
Figure 12 shows the construction of pPTR-eno-hyp, a plasmid highly expressing hydrophobin.

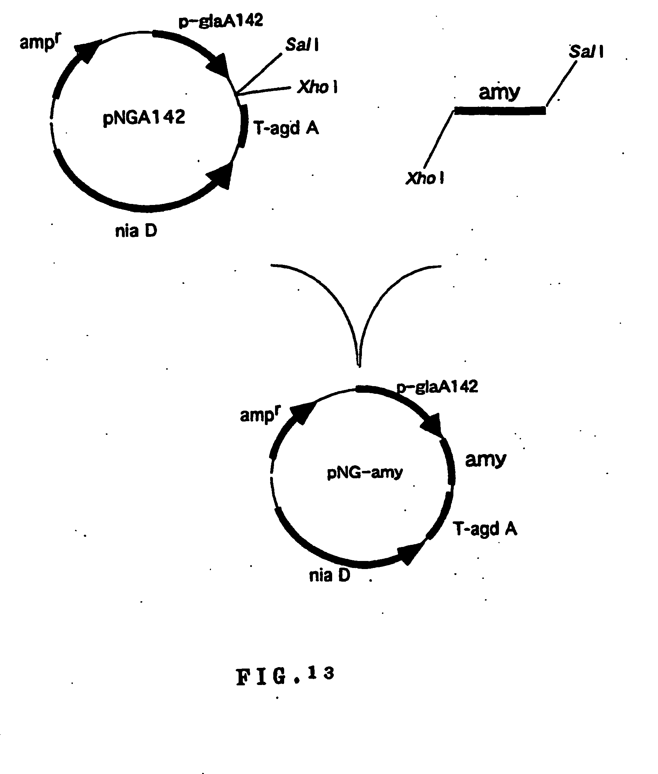

Figure 13 shows the construction of pNG-amy, a plasmid highly expressing α-amylase for *Aspergillus* strain.
Figure 14 is a photo of SDS-PAGE showing that a strain highly triple-coexpressing cutinase, hydrophobin and α-amylase highly expresses hydrophobin.
Figure 15 shows the construction of pNG-gla-hypB having glaA 142 promoter fused upstream of hypB gene.
Figure 16 shows the construction of pNG-gla-hydrophobin-315 having glaA 142 promoter fused upstream of hydrophobin-315 gene.
Figure 17 shows a phylogenic tree of hydrophobins derived from filamentous microorganisms and their analogues.
Figure 18 shows the adsorption of hydrophobin to various biodegradable plastics.
Figure 19 shows a phylogenic tree of protein-degrading enzymes derived from filamentous microorganisms and their analogues.
Figure 20 shows a graph showing the concentration of protein in an eluate fraction from octylcellulofine column of the supernatant of PBS degradation culture, and a photo of SDS-PAGE showing the existence of each fraction.
Figure 21 shows the construction of a high expression system of PBS-binding protein.
Figure 22 is a photo of SDS-PAGE showing that a band of a desired protein is observed at about 14 kDa only in the supernatant of a culture medium of *Aspergillus oryzae* transformant.
Figure 23 is a photo showing that a strain expressing the 14 kDa protein and hydrophobin grows faster than a wild strain.
Figure 24 is a photo of SDS-PAGE showing the adsorption of PbpA to PBS in vitro.
Figure 25 shows a phylogenic tree of PbpA and PbpB homologous sequences.
Figure 26 is a photo showing the degradation of platinum string covered with PBS by cutinase.
Figure 27 is a photo showing promotion of cutinase-degradation of PBS film by RoIA.
Figure 28 shows a promoting effect of RoIA for the degradation of PBS emulsion by cutinase in vitro.
Figure29 shows promotion of degradation by the adsorption of PbpA to PBS.
Figure 30 shows the comparison in cutinase-degradation of PBS film between hydrophobin and a synthetic surfactant.

### [Best Mode for Carrying Out the Invention]

The biosurfactant used in the present invention generally means a surfactant produced by an organism, especially is a kind of a plastic-binding factor that is produced and secreted extracellularly by a microorganism.

The biosurfactant includes in addition to the plastic-binding protein any substance known to those skilled in the art, for example, a glycolipid in which a phospholipid or hydrophilic sugar is bound to a hydrophobic fatty acid, including such as glycolipid ester such as mannosilerythritol lipid and rhamnolipid; cyclolipopeptide; cyclopolypeptide; and amphiphatic protein such as surfectin.

The plastic-binding protein includes hydrophobin and its homologues specifically described in the following examples, and other protein-binding proteins. They may be obtained from any origin, including the above filamentous bacteria and filamentous fungi, especially *Aspergillus* strains such as *Aspergillus oryzae.*

The plastic-degrading enzyme used in the present invention is a cutinase . The enzyme may be derived from the above filamentous bacteria and filamentous fungi, especially from *Aspergillus* strains such as *Aspergillus oryzae.*

Thus, hydrophobin and cutinase from *Aspergillus oryzae* may be used as the plastic-binding protein and plastic-degrading enzyme, respectively. Hence, in one instance the invention provides a transformant of the invention wherein the biosurfactant is hydrophobin or a hydrophobin homologue derived from *Aspergillus oryzae.*

The present invention also provides a transformant further prepared by recombination with the use of DNA comprising a gene encoding a useful substance.

The plastic to be degraded according to the method of the present invention is so called "biodegradable plastic". Thus, the invention provides a method of degrading biodegradable plastic using a transformant of the invention.

The biodegradable plastic is defined as a substance that keeps its function sufficiently enough for its during a used state, and will be degraded to a simpler molecular level by the function of microorganism in water or soil after having been discharged. It may be classified into "completely degradation- type biodegradable plastic" and "partially degradation (disintegration)-type biodegradable plastic" depending on a degree of degradation, and into "microorganism production type", "natural polymer type", and "chemical synthesis-type" in view of their production method and material. Any type of the above biodegradable plastics may be used in the present invention.

Thus, examples of the plastic used in the present invention may be selected from the group consisting of polyester, polyurethane, polypropylene, polyvinyl chloride, nylon, polystyrene, starch, and any combination thereof. Polystyrene includes poly butylene succinate (PBS), poly butylsuccinate adipate (PBSA), poly lactic acid (PLA), aliphatic polyester, polycaprolactone and any combination thereof.

The plastic to be degraded in the present invention may be comprised as one element of a composite material. The composite material consists of two or more kinds of substances, being, for example, that of plastic, any metal and other inorganic substances. Such composite material is widely used as industrial material for various purposes in various fields.

According to the present invention, the plastic comprised in the composite material may be selectively degraded and recovered as monomer or oilgomer, while the other parts such as metal parts containing substantially no plastic may be recovered as well.

The degradation reaction according to the present invention may be performed in any reaction system (e.g., aqueous solution or solid system) and any conditions known to those skilled in the art depending on its purpose and scale. For example, it may be carried out in any culture system known to those skilled in the art including a liquid culture system comprising plastic emulsion and solid culture system comprising plastic solid pellet or powder.

The plastic to be degraded may therefore take any form such as emulsion and solid pellet depending the type of culture system.

In the aspect of the present invention in which the plastic is degraded in the presence of the biosurfactant, an amount, timing, means and method with respect to the addition of the biosurfactant to the reaction system may be optionally selected by those skilled in the art.

It is preferable to perform the above method in such a condition that a hydrophobic interaction between the biosurfactant and the plastic will be strengthened in order for the biosurfactant to effectively attach to the plastic and to obtain an advantage of the present invention.

A preferable example of the present method therefore comprises a step of mixing the biosurfactant and the plastic in a low water activity condition, and a step of degrading the plastic with the use of the plastic-degrading enzyme in a high water activity condition.

The term "water activity" is a factor well known to those skilled in the art, and is defined as a ratio between the vapor pressure of a solution comprising a solute and that of pure one (John A. Troller & J.B. Christian, "Water Activity and Food", Academic Press, Inc.). The term "low water activity condition" in this specification means the condition wherein the biosurfactant can significantly attach to a hydrophobic surface of the plastic, specifically 0.97 or less, preferably 0.95 or less, more preferably 0.9 or less of the water activity. On the other hand, the term "high water activity condition" in this specification means other conditions other than the low water activity condition. The solute may be salts, sugars, and alcohols.

One specific example therefore comprises mixing the biosurfactant and the plastic in film or pellets in the low water activity condition so that an effective amount of the biosurfactant may attach to the plastic, and increasing the water activity to promote the degradation of the plastic with use of the plastic-degrading enzyme.

Alternatively, another preferable example wherein the hydrophobic interaction between the biosurfactant and the plastic will be strengthened comprises a step of mixing the biosurfactant and the plastic in a high salt concentration condition, and a step of degrading the plastic with the use of the plastic-degrading enzyme in a low salt concentration condition. The tem "high salt concentration condition" means in this specification a condition wherein the biosurfactant can significantly attach to a hydrophobic surface of the plastics, specifically 4% or more, preferably 8% or more, more preferably 14 % or more when NaCl is used as salt. On the other hand, the term "low salt concentration condition" in this specification means other conditions other than the "high salt concentration condition. However, the plastic-degrading enzyme does not necessarily have to be reacted in the low solute concentration, but may be reacted directly after the adsorption of the surfactant in the high solute concentration.

The reaction of the method according to the present invention may be performed at a temperature of 0∼100°C, preferably 0∼80°C, more preferably 15∼80°C.

Since a pH value of the reaction system can deviate from an optimum pH range of the plastic-degrading enzyme as a result of acidification of a reaction solution due to acidic substances such as the monomer or oligomer generated by the degradation of the plastic, it is preferable to keep an appropriate neutral pH condition, for example, pH of 4∼11, preferably of 6∼10, more preferably of 7∼9 by performing the degradation reaction in a buffer solution having a high buffering function, or by adding a basic substance into the system during the reaction.

The plastic may be degraded with the use of the biosurfactant and the plastic-degrading enzyme in a protein preparation form, or with those as produced *in situ* by *Aspergillus oryzae* or *Aspergillus sojae.*

The degradation of the plastic may be promoted by making the plastic in contact with the microorganism in the coexistence of the biosurfactant and/or the plastic-degrading enzyme in the culture medium. Those substances may be produced by the microorganisms themselves, or they may be further added from an outside of the culture system. An amount, ratio, and timing with respect to their addition may be optionally selected by those skilled in the art. It is not necessary to add simultaneously those substances, but they may be added sequentially in each step of the method according to the present invention.

The coexistence of the biosurfactant and/or the plastic-degrading enzyme in the culture medium will strengthen the contact of the plastic with the microorganisms and promote the degradation efficiency of the plastic.

The biosurfactant and the plastic-degrading enzyme that may coexist in the above aspects are the same as those referred to in the first aspect of the present invention.

As already described in the above, it is possible in the present invention to degrade the plastic and convert the degraded plastic into the useful substance with the use of the transformant capable of highly expressing the biosurfactant, plastic-degrading enzyme and/or useful substances.

A gene encoding each of the above substances may be introduced into a separate microorganism, and the thus prepared transformants may be used in the method according to the present invention. Thus, it is possible to carry out the present invention with the use of any combination of the transformants selected from a transformant prepared by recombination with the use of a gene encoding the biosurfactant, a transformant prepared by recombination with the use of a gene encoding the plastic-degrading enzyme, and a transformant prepared by recombination with the use of a gene encoding the useful substance.

Two or more kinds of the genes encoding the biosurfactant, plastic-degrading enzyme or useful substances, respectively, may be used for the recombination to prepare the above transformants.

Alternatively, as will be described in the example, a transformant highly coexpressing the biosurfactant and the plastic-degrading enzyme may be used in the present invention, which has been transformed with both the gene encoding the biosurfactant and the gene encoding the plastic-degrading enzyme. In this case, the microorganism producing the plastic-degrading enzyme itself will attach to the hydrophobic surface of the plastic so as to promote the degradation of the plastic.

It is also possible to make the thus obtained transformant capable of degrading the plastic and a microorganism to be used for the production of the useful substance co-exist in the culture system in order to finally produce the useful substance.

The transformant of the invention may be one further prepared by recombination with the use of DNA comprising a gene encoding a useful substance.

Also, as will be described in the example, a transformant highly triple-coexpressing simultaneously the biosurfactant, the plastic-degrading enzyme and the useful substance may be used in the present invention, which has been transformed with the genes encoding each of the above substances, respectively.

By utilizing the variant highly expressing the useful substance, or the transformant highly expressing the useful substance, which has been obtained by recombination with the use of a gene encoding it, it is possible to introduce the gene encoding the useful substance into the microorganism having a high capacity of degrading the plastic. The useful substance may be effectively produced simultaneously with the degradation of the plastic with the use of the thus obtained transformant highly triple-coexpressing them.

When plural kinds of the microorganisms are used in the present invention, they may be simultaneously cultured in a co-culture system. Or they may be sequentially added in each step to the culture medium obtained in the previous step when the present method consists of sequential culture steps for degradation and conversion.

Even when the above transformants are used in the present invention, the biosurfactant, plastic-degrading enzyme and/or useful substances may be further added from the outside to co-exist in the system so as to further promote the degradation and conversion of the plastic.

Any surfactant known to those skilled in the art may be used in the present invention, the biosurfactant that is produced by the microorganism on its surface or extracellularly being preferred from an environmental point of view.

In the methods of degrading the plastic with the use of the microorganism and for producing the useful substance from the plastic according to the present invention, degradation and conversion reaction may be carried out in any culture system known to those skilled in the art, including a liquid culture system comprising plastic emulsion and solid culture system comprising plastic solid pellet or powder.

The plastics to be degraded may therefore take any form such as emulsion and solid pellet depending the type of culture system. The culture medium and its composition, and culture conditions such as temperature and pH may be optionally selected by those skilled in the art depending on the kinds of the plastic and the microorganism to be used.

As already described in the above, It is, however, preferable to perform the above method in such a condition that a hydrophobic interaction between the biosurfactant and the plastic will be strengthened so that the biosurfactant may first effectively attach to the plastic and function together with the plastic-degrading enzyme to degrade the plastic.

For example, after an appropriately low water activity state has been obtained by adding buffer to the plastic, the fungal form (cell, spore, mycelia, etc) of the microorganism such as the transformant according to the present invention is inoculated and cultured in an incubator with a controlled temperature and humidity to produce "koji" (rice malt) where the biosurfactant produced by the microorganism will react with the plastic. The water activity state is then increased by adding an appropriate buffer so that the plastic may be degraded by the plastic-degrading enzyme. In this case, the addition of the surfactant from the outside is done preferably in the low water activity condition, and the addition of the plastic-degrading enzyme from the outside is done preferably in the high water activity condition.

As already mentioned, in order to prevent the influence by the acidic substances such as the monomer or oligomer generated by the degradation of the plastics, it is preferable to keep an appropriate neutral pH condition by using a buffer solution having a high buffering function, or by adding a basic substance into the system during the reaction.

An example of the gene of the hydrophobin and its homologue derived from *Aspergillus oryzae* is a DNA comprising a base sequence encoding the following polypeptide (a) or (b):
(a) polypeptide having an amino acid sequence that is the same or substantially the same as that represented by SEQ ID No.1, No.2 or No.3,
(b) polypeptide having an amino acid sequence of (a) wherein a part of amino acid residues are replaced, deleted, or added, and having substantially the same function as the hydrophobin.

Another example is a DNA of the following (a) or (b):
(a) DNA comprising a base sequence represented by SEQ ID No.1, No.2 or No.3 or its partial sequence,
(b) DNA being hybridized with a base sequence complementary to the DNA comprising the base sequence in (a) under stringent conditions, and having substantially the same function as the DNA (a).

An example of the gene of the plastic-degrading enzyme derived from *Aspergillus oryzae* is a DNA comprising a base sequence encoding the following polypeptide (a) or (b):
(a) polypeptide having an amino acid sequence that is the same or substantially the same as that represented by SEQ ID No.4 or No.5,
(b) polypeptide having an amino acid sequence of (a) wherein a part of amino acid residues are replaced, deleted, or added, and having substantially the same function as the plastic-degrading enzyme.

Another example is a DNA of the following (a) or (b):
(a) DNA comprising a base sequence represented by SEQ ID No.4 or No.5 or its partial sequence,
(b) DNA being hybridized with a base sequence complementary to the DNA comprising the base sequence in (a) under stringent conditions, and having substantially the same function as the DNA (a).

An example of the gene of the plastic-binding protein derived from *Aspergillus oryzae* is a DNA comprising a base sequence encoding the following polypeptide (a) or (b):
(a) polypeptide having an amino acid sequence that is the same or substantially the same as that represented by SEQ ID No.6 or No.7,
(b) polypeptide having an amino acid sequence of (a) wherein a part of amino acid residues are replaced, deleted, or added, and having substantially the same function as the plastic-binding protein.

Another example is a DNA of the following (a) or (b):
(a) DNA comprising a base sequence represented by SEQ ID No.6 or No.7 or its partial sequence,
(b) DNA being hybridized with a base sequence complementary to the DNA comprising the base sequence in (a) under stringent conditions, and having substantially the same function as the DNA (a).

The "function" of the polypeptide means in this specification a biological function or activity shown inside or outside the cell (fungal form). The term "substantially the same" means the functions (or activities) in question can be considered equivalent as function inside or outside the cell to each other although there may be some difference in degree.

The genes according to the present invention may be prepared by any method known to those skilled in the art. For example, they may be easily cloned from the strains deposited in accordance with the methods described in the examples. Alternatively, they may be prepared with a chemical synthesis known to those skilled in the art or by PCR using the primers of the present invention based on the information about the base or amino acid sequence of the present DNA.

The term "stringent conditions" means in this specification, for example, that a hybrid may be formed only between the base sequences that have such a high degree of homology between them as of about 80% or more, preferably about 90% or more, more preferably 95% or more on a total average. Specifically, it means, for example, sodium concentration of 150∼900mM, preferably 600∼900mM, pH of 6∼8 at 60°C∼68°C.

The hybridization may be performed in accordance with a method known in the art, for example, that described in Current protocols in molecular biology (edited by Frederick M. Ausubel et al., 1987). When a commercially available library is used, the hybridization may be done according to instructions attached to it.

The amino acid sequence that is substantially the same as the specific amino acid sequence in the present specification means that 80% or more, preferable 90% or more, more preferably 99% or more of the amino acids are the same on a total average when they are aligned and compared to each other. Accordingly, it is considered that they have substantially the same function.

The polypeptide having the particular amino acid sequence wherein a part of amino acid residues are replaced, deleted, or added means in this specification that preferably 1∼20 amino acids, more preferably 1∼10 amino acids, further more preferably a few amino acids are replaced, deleted, and/or added, as long as it has substantially the same function as its original amino acid sequence.

The polypeptide having the particular amino acid sequence, or that wherein a part of amino acid residues are replaced, deleted, or added may be easily prepared by any combination of methods known to those skilled in the art, such as site-specific mutagenesis, homologous recombination, primer extension method, and PCR.

Replacement between amino acids belonging to the same group (polar or non-polar amino acids, hydrophobic or hydrophilic amino acids, positively- or negatively charged amino acids, aromatic amino acids, etc.) may be selected in order to maintain substantially the same function as that of the original protein.

On the other hand, amino acids within a functional domain of the protein should preferably be kept for the above purpose.

The various transformants, which are already described in the above as the examples of the microorganism to be advantageously used in the method of the present invention, may be prepared by constructing a recombinant vector comprising the DNA or gene, and introducing the vector into filamentous bacteria or fungi or their variant strains with a recombination method known to those skilled in the art such as protoplast-PEG method, electro-poration, T-plasmid method, and particle-gun method.

As already mentioned, it is preferable to release a suppressed induction of the production of the substances. For that purpose, the genes encoding these substances may be controlled by a promoter for constitutive expression or various inducible promoters. As a result, the above substances are highly expressed and produced extracellularly or on the cell surface so that the degradation of the plastic and the production of the useful substances may be promoted.

Thus, the invention also provides a transformant wherein at least one of the DNA comprising the gene encoding the biosurfactant and the DNA comprising the gene encoding the plastic-degrading enzyme is expressed under the control of a promoter derived from another gene.

The above promoters are well known in the art, including constitutive expression promoters for *Aspergillus oryzase* such as enoA promoter, pgkA promoter and tef1 promoter; and inducible expression promoters for *Aspergillus oryzase* such as amylase promoter and α-amylase promoter using maltose as an inducing substance, and xylanase promoter using xylose as an inducing substance.

### [Example]

The present invention will be explained more in detail by referring to the Examples, which will not limit the scope of the present invention. The procedures in the examples were done in accordance with those described in Current Protocols in Molecular Biology (edited by Frederick M. Ausubel et al., 1987).

### Example 1

(1) Isolation of a gene of hydrophobin-fungal plastic-attaching factor
   (1-1) Confirmation of the conversion of biodegradable plastic with the use of *A. oryzae*

### [Materials]

1. Agent
   Unless otherwise noted, a high quality agent of Nacalai Tesque, Inc. was used. Polybutylene succinate (PBS) was selected as the biodegradable plastic, and "Bionolle Emulsion OLX-07527" from SHOWA HIGHPOLYMER CO., LTD., was used as PBS emulsion. The agents were prepared with the use of Milli-Q.
2. Fungus
   *Aspergillus oryzae* RIB40 was used.
3. Culture medium
   Culture medium shown in Table 1 was used.

**[TABLE 1]**

| 10 × stook solution (pH 6.5) (/litter) | | PBS emulsion minimal medium (1/litter) | |
|---|---|---|---|
| | | upper medium | |
| NaNO, | 60 g | 10 × stock solution | 100ml |
| KCI KH₂PO₄ | 5.2 g 15.2 g | 1000 × trace elements solution | 1 ml |
| | | PBS emulsion liquid | 10ml |
| adjust to pH 6.5 with 10 N KOH | | 1M MgSO₄ | 2ml |
| | | agarose | 5_{g} |
| 1000 × trace elements solution (/litter) | | lower medium (1/litter) | |
| FeSO₄ · 7H₂O | 1.0 g | 10 × stock solution | 100ml |
| Zn · 7H₂O | 8.8 g | 1000 × trace elements solution | 1ml |
| Cu · 5H₂O | 0.4 g | 1M MgSO₄ | 2ml |
| Mn · 4H₂O | 0.15 g | agarose | 15g |
| Na₂B₄O₇ · 10H₂O | 0.1 g | pour the medium on the lower medium | |
| (NH₄)6Mo₇O₂₄ · 7H₂O | 0.05 g | | |

### [Methods]

A spore suspension of various kinds of *Aspergillus* strains (*A.oryzae, A.soyae, A.kawachi, A. awamori, A.niger, A.nidulans*) was spotted on a PBS emulsion minimal agar medium and cultured for 7 days at 37°C. Similarly, a spore emulsion of *A. oryzae* was inoculated at a final concentration of 0.5 x 10⁵ spores/ml in a PBS emulsion minimal liquid medium (5 ml) wherein carbon source was limited only to PBS, and cultured in a test tube with shaking for 5 days at 30°C.

### [Results]

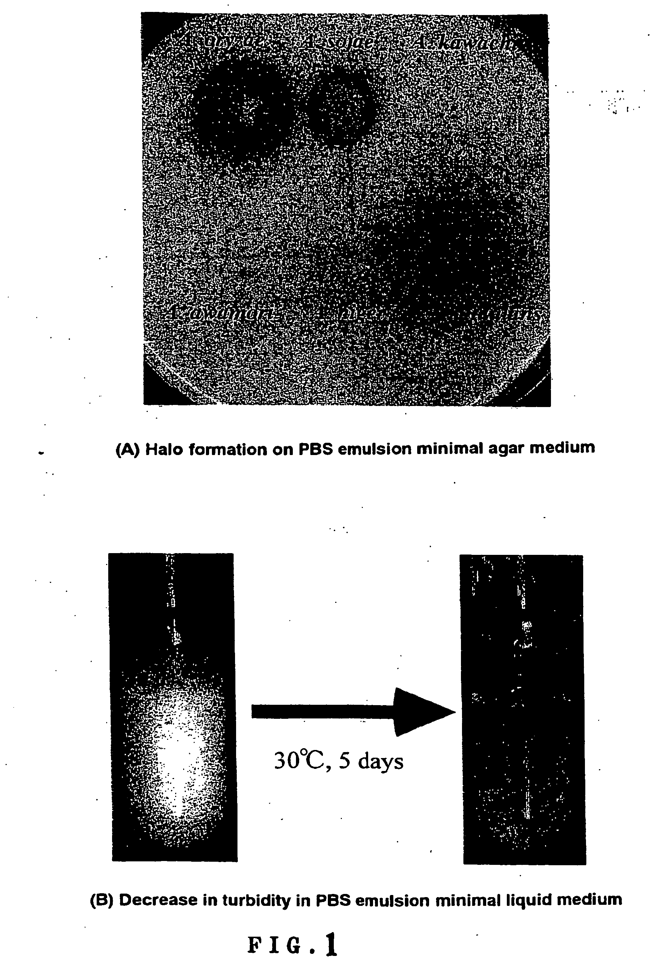

A halo with about 1.9 cm in width and about 1.5 cm in width were observed in the solid culture of *A.oryzae* and *A*.*soyae*, respectively (Fig.1 (A)). And a halo was also observed in the culture of *A.nidulans.* Turbidity of PBS was significantly decreased in the liquid culture of *A.oryzae* (Fig.1 (B)). These facts confirmed that the filamentous fungi of *Aspergillus* such as *A*.*oryzae* had a PBS-converting activity

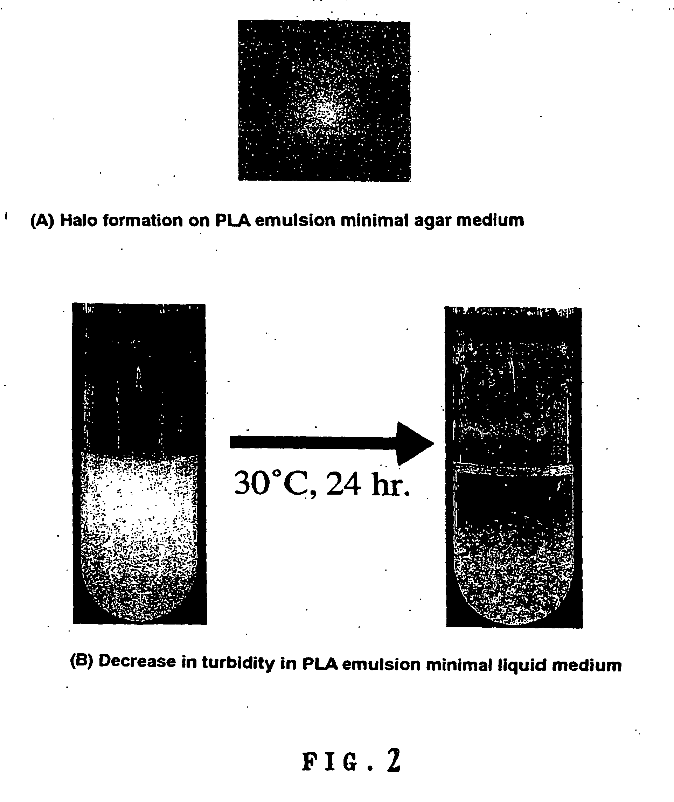

Furthermore, the degradation of plastic with the use of *A*.*oryzae* was also confirmed in the culture system in which poly lactic acid (PLA) was used instead of PBS. The results are shown in Fig.2.

### (1-2) Analysis of gene expression by AND microarray

### (1-2-1) Preparation of total RNA of Aspergillus fungus

### [Materials]

### 1. Agent

The total RNA from the fungus was prepared by means of Sepasol-RNAISuper (Nacarai Tesque Inc.).

### 2. Culture Medium

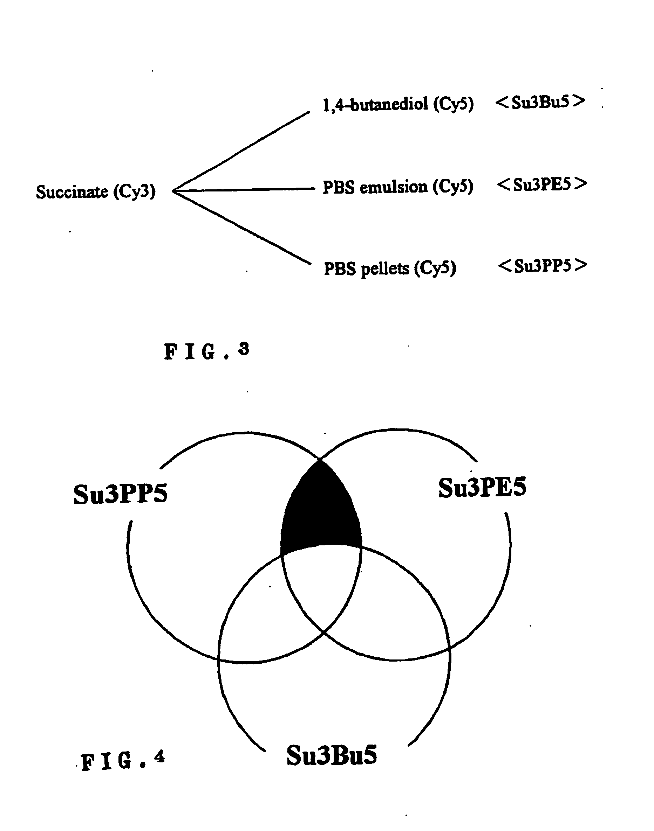

The composition, culture conditions and their combination were summarized in Table 2 and Fig.3.

**[TABLE 2]**

| C source | Succinate | 1.4-butanediol | PBS emulsion | PBS pellets |
|---|---|---|---|---|
| the quantity of C sources | 10g | 10ml | 4.3ml | 100g |
| the volume of cultures | 150ml | 150ml | 450ml | 1500ml |
| cultivating times | 24h | 24h | 60h | 120h |

### [Methods]

A spore solution was inoculated at a final concentration of 0.5x10⁷ spores/ml to Czapek-Dox medium (150 ml) in a conical flask with a blade (500 ml) and cultured for 12 hours at 30°C. The fungus was collected by means of MIRACLOTH (Calbiochem) and washed with sterilized water, followed by removal of extra water. The collected fungus was transferred to a minimal medium containing PBS solid pellet, PBS emulsion, succinic aid and 1,4-butanediol, respectively, as an only carbon source, cultured with shaking at 30°C for 120 hours (PBS solid pellet), 70 hours (PBS emulsion), 12 hours (succinic acid) and 24 hours (1,4-butanediol). After the culture, the fungus was collected by means of the MIRACLOTH and washed with sterilized water, followed by removal of extra water. The fungus was separated from the PBS solid pellet by means of a tee strainer. After the thus obtained wet fungus was measured with respect to its wet weight, it was crushed into powder in a triturator while liquid nitrogen was being poured. The pulverized fungus was transferred into a tube (50 ml) containing Sepasol-RNAISuper with an amount four times that of the wet weight, stirred vigorously and allowed to stand for 5 min at a room temperature. Chloroform in an amount one fifth that of Sepasol-RNAISuper was added to the tube, stirred vigorously and allowed to stand still for 5 min at a room temperature. After centrifugation at 10,000xg for 15 min at 4°C, an aqueous layer was transferred to a 15 ml tube and mixed with water-saturated acidic phenol-chloroform (phenol/chloroform=1:1). After centrifugation at 12,000 x g for 10 min at 4°C, the resulting aqueous layer was transferred to another 15 ml tube. After addition of an equal amount of isopropanol, the mixture was allowed to stand for 10 min at a room temperature and centrifuged at 12,000 x g for 10 min at 4°C. The resulting supernatant was discarded and the precipitate was rinsed with 70% ethanol. After drying with air, it was resolved into an appropriate amount of DEPC (diehtylpirocarbonate)-treated water.

### (1-2-2) Purification of mRNA

### [Materials]

### mRNA was purified with Message Marker (Giboco BRL).

### [Methods]

Purification was done in accordance with a manual attached to the Message Marker. The procedures are described below with the use of 1 mg of total RNA.

A 15 ml tube was filled up with one mg of total RNA and 1.8 ml of DEPC-treated water to a final concentration of 0.55 mg/ml, incubated for 5 min at 65°C, and cooled rapidly on ice. After addition of 200 µl of 5 M NaCl followed by vigorous stirring, and addition of 1 ml of oligo(dT) Cellulose Suspension followed by vigorous stirring, the mixture was incubated for 10 min at 37°C. The resulting sample was put into a syringe and pressed out by means of a plunger. Wash buffer 1 of 3 ml in a disposable cup was sucked into the syringe. The resulting solution was mixed well and liquid was pressed out by means of the plunger. The same procedure was done with 3 ml of Wash buffer 2. One ml of DEPC-treated water kept at 65°C was then sucked into the syringe, and the resulting solution was mixed well and pressed into a 15 ml tube. After the same procedure was repeated with anther 1 ml of the above DEPC-treated water, the resulting eluate was put together, centrifuged at 12,000 x g for 3 min at 4 ° C to removed the oligo(dT) Cellulose Suspension. The resulting supernatant (ca. 2 ml) was mixed with 20 µl of glycogen solution (5mg/ml) and 200 µl of 7.5 M ammonium acetate (pH5.2), and dispensed evenly into five Eppendorf tubes. The dispensed solution was mixed with ice-cooled ethanol with an amount twice that of the solution and subjected to ethanol precipitation at -20°C for overnight. After centrifugation at 12,000 x g, for 30 min at 4°C, the supernatant was discarded and the precipitate was rinsed with 75% ethanol. After drying with air, the precipitate was dissolved into DEPC-treated water.

### (1-2-3) Labeling of mRNA

### [Materials]

The purified mRNA was labeled with Cy3-dUTP, Cy5-dUTP (Amersham Biosciences).

### [Methods]

mRNA (0.5-1.0 µg), 1 µl of random primer (9 mer, 2 µg/µl), 1 µl of oligo(dT) primer (12-18 mer, 0.5 µg/µl), and DEPC-treated water were added to an Eppendorf tube to a final volume of 10 µl. The mixture was incubated for 10 min at 70°C, allowed to stand for 10 min at a room temperature and for 5 min or more on ice so as to anneal mRNA with the primers. To this mixture, 4 µl of 5 x First Strand Buffer (250 mM Tris-HCl, pH 8.3, 375 mM KCI, 15 mM MgCl₂), 2 µl of 0.1 M DTT, 0.4 µl of 50 x dNTPs (25 mM of dATP, GTP and CTP, 10 mM of TTP), 2 µl of Cy-dye (3 or 5) and 1.5 µl of Super Script II RT (200 U/µl) were added, mixed with a pipette and allowed to stand for 1 hour at 42°C for reverse transcription reaction. After this procedure, attention had been paid in order to protect the samples from a direct light. Then, the resulting solution was mixed with another 1.5 µl of Super II RT (200 U/µl) and incubated for 1 hour at 42°C, for 10 min at 70°C and for 5 min at 37°C, and mixed with 0.3 µl of RNase H (10 U/µl) and incubated for 15 min at 37°C and for 10 min at 70°C to decompose unreacted mRNA. Each combination of the solution labeled with Cy3 or Cy5 (Fig.3) was mixed in a Microcon-30 cup amounted with a specialized tube, mixed with 300 µl of TE and centrifuged for concentration at 10,000 x g for 20 min at a room temperature. The resulting solution was then mixed with 60 µl of Human Cot1-DA (1 mg/ml) and 300 µl of TE, and centrifuged for concentration at 10,000 x g for 30 min at a room temperature. The Microcon-30 cup was picked up, inserted upside down into another tube and centrifuged at 3,000 x g for 3 min at a room temperature to recover the solution in the cup. The recovered solution was filled up to 28 µl with TE, and mixed with 4 µl of yeast tRNA (10mg/ml), 16 µl of poly dA (1 mg/ml) and 10.2 µl of 20 x SSC and 1.8 µl of 10 % SDS. The resulting mixture (60µl) was boiled for 1 min, cooled back to a room temperature and subjected to hybridization.

### (1-2-4) Hybridization, Washing, Detection

### [Materials]

The following devices were used: cDNA microarray (or "DNA chip") (Asahi Technoglass Corp.) carrying 2,146 EST clones of *A*. *oryzae*, a gap cover glass (25 x 50 mm; MATSUNAMI GLASS IND., LTD.), Array IT for hybridization cassette, Gene Pix 4000B microarray scanner (Axon) for scanner analysis, and Gene Pix Pro 3.0 software package (Axon) and Array Pro program (Cybernetics) for image analysis.

### [Methods]

The gap cover glass was mounted on a slide glass so that an area wherein genes had been spotted would be surround, and 30 µl of the labelled solution prepared above was infused by means of a capillary action. After the slide glass was set in a hybridization cassette, 30 µl of purified water was dropped into depression in the cassette and 5 µl of 3 x SSC was dropped onto two points far from the cover glass on the slide glass for the purpose of prevention of drying. Hybridization was carried out with gentle shaking for 8 hours at 55°C. After the completion of hybridization, washing of the slide glass was done as follows. The hybridization cassette was taken out and soaked in 2 x SSC/0.1 % SDS in order to quickly slip away the slide glass in the same liquid. The slide glass was shook in 0.2 X SSC/0.1 % SDS kept at 40°C for 5 min and allowed to stand in 0.2 X SSC for 3 min. After centrifugation at 300 x g for 1 min to remove moisture from the slide glass, the detection was made. In the case where the expression amount of a control (succinic aid) was more than that of the samples (PBS solid pellet, PBS emulsion, and 1,4-butanediol), a spot of the DNA chip would become green. While the spot would become yellow when their expression amounts were the same. It would become red when the expression amount of the samples was more than that of the control. That is, genes that expressed specifically in the presence of PBS would become red in Su3PP5 and Su3PE5, but would not be colored in Su3Bu5 (Fig.4). Accordingly, the genes that expressed specifically in the presence of PBS were searched by subtracting the genes that were colored in red in Su3Bu5 from the genes that were colored in red in both Su3PP5 and Su3PE5. Signal intensity (Cy5/Cy3) of histone gene (control) that was expressed commonly in Su3PP5, Su3PE5 and Su3Bu5 was adjusted to about 1 (yellow).

### [Results]

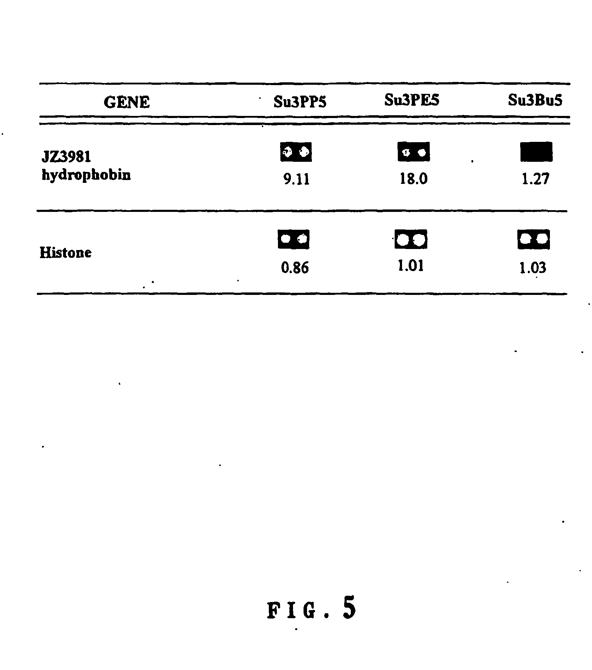

Visual analysis of the results obtained in the DNA array of Su3PP5, Su3PE5 and Su3Bu5 indicated that JZ3981 was colored in red only in Su3PP5 and Su3PE5 (Fig.5). The numbers shown below the spots in Fig.5 represent a ratio of the expression (Cy5/Cy3). The above result showed that JZ3981 gene was involved in the degradation of PBS.

### (1-2-5) Investigation of JZ3981 with the use of EST data base

Information about a gene having a high homology (identity) to JZ3981 gene was searched with the use of BLAST network service. It was shown that the JZ3981 gene had homology to RODLET PROTEIN PRECURSOR *of Emericella nidulans* (4e-457), and HYDROPHOBIN PRECURSOR of *Aspergillus fumigatus* (9e-57). It was also shown that the JZ3981 gene contained eight cysteine resides conserved in hydrophobin. As a result, the JZ3981 gene was identified to be a hydrophobin gene (hyp) of *A oryzae.*

### (1-3) Isolation of the hydrophobin gene (hyp) of A. oryzae

### (1-3-1) Analysis of the base sequence of JZ3981

As information of the EST clone JZ3981 contained only its partial sequence, its full base sequence was determined in this example.

### [Methods]

Sequencing was performed by using ABI PRISM 310 Genetic Analyzer.

| | |
|---|---|
| JZ3981 | 4 µl |
| M 13 Universal primer (F or R) | 4 µl |
| Terminator Ready Reaction ¥mix | 8 µl |
| DDW | fill up to 20 µl |

PCR was carried out in the above reaction system by means of TaKaRa PCR Thermal Cycler PERSONAL (TAKARA SHUZO CO., LTD.) repeating 30 cycles of 96°C for 3 min, 96°C for 10 min, 50°C for 5 min and 60°C for 4 min. After the completion of PCR, the reaction mixture was precipitated with ethanol, dissolved in 15 µl of loading dye, boiled for 3 min, and subjected to ABI PRISM 310 Genetic Analyzer for analysis with ABI PRISM Sequencing Analysis version 3.0.

### [Result]

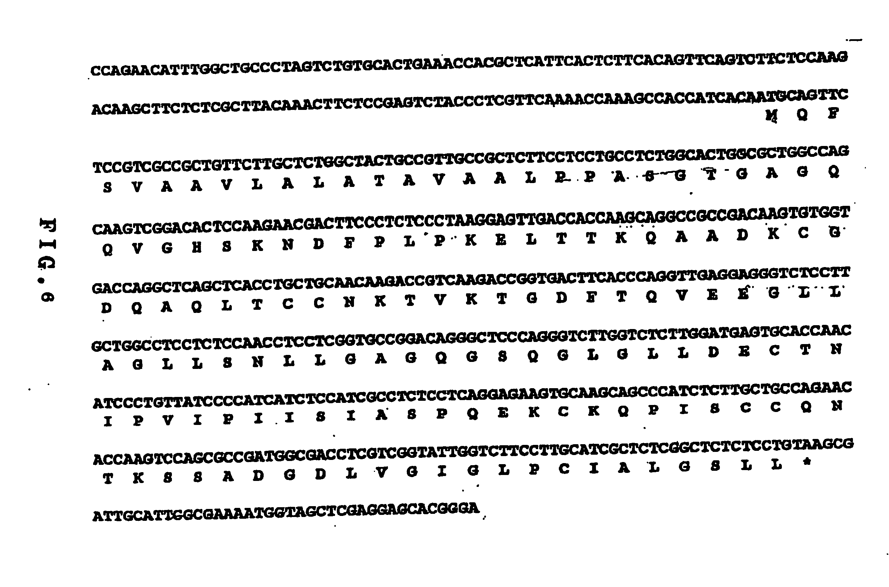

It was confirmed that the JZ3981 contained a full length of hydrophobin (Fig.6).

### Example 2

### (2) Preparation and Growth of an Aspergillus strain highly expressing hydrophobin.

### (2-1) Construction of a plasmid highly expressing hydrophobin.

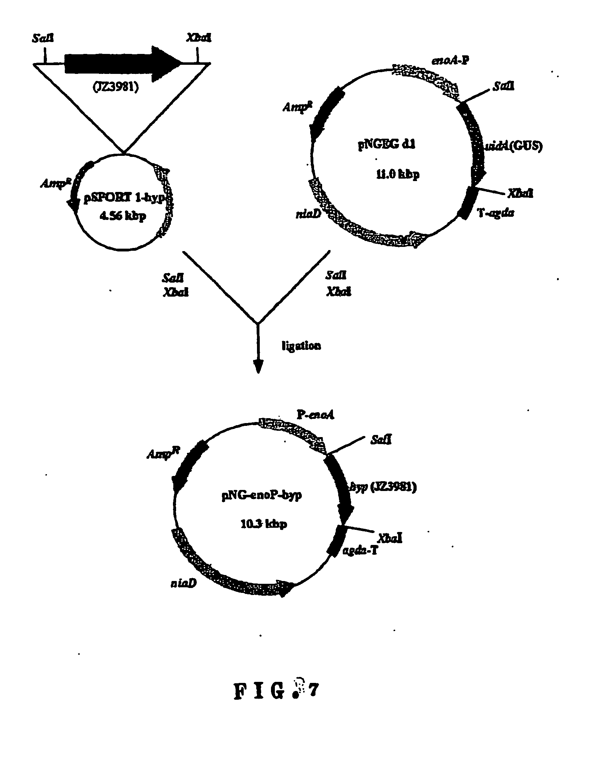

For the preparation and growth of an *Aspergillus* strain highly expressing hydrophobin, a plasmid highly expressing hydrophobin for *Aspergillus* was constructed by using an expression vector for *Aspergillus* fungi, pNGA142 vector ("Fundamental Science and Biotechnology Fungus and Mould", Kazuo Shishido, IPC, p.534). As a constitutive promoter was necessary for the high-expression of hydrophobin. enoA promoter was fused upstream of a desired gene (hyp) to give pNG-enoP-hyp (Fig.7).

### [Materials]

### 1. Agent

Restriction enzymes and modification enzymes were purchased from TAKARA SHUZO CO., LTD, Boehringer Mannheim Yamanouchi Co., New England Biolabs. The agents were prepared with MilliQ and sterilized in an autoclave for 20 min at 121°C unless otherwise noted.

### 2. Strains

*Escherichia coli* XL1-Blue (Stratagene Inc.) and *A. oryzae* RIB40 were used.

### 3. Culture medium

Culture medium for *E*. *coli* was prepared according to a manual attached to the above XL 1-Blue.

### 4. Vector

A vector, pNGEG-d1 (Toda T., et al., Curr. Genet. 40:260-267, 2001) comprising the enoA promoter was used. JZ3981 comprising an insert portion (pSPORT 1-hyp) was furnished from National Institute of Brewing (3-7-1, Kagamiyama, Higashihiroshima-shi, Hiroshima).

### [Methods]

### 1. Cleavage of DNA by restriction enzyme

pNGEG-d1 and pSPORT 1-hyp were cut by *Xba*I and *Sal*I, respectively.

### 2. Preparation of vector DNA and insert DNA

A vector DNA and an insert DNA were completely digested with the restriction enzymes and subjected to agarose gel electrophoresis. DNA fragments were excised under UV (366 nm) radiation and recovered with the use of prep A gene (Bio-Rad- Laboratories) to be used as the vector DNA and insert DNA.

### 3. Ligation

Ligation reaction was carried out for 16 hours at 16°C using the thus prepared vector DNA and insert DNA, T4 DNA ligase (GIBCO BRL Lifetechnology), 5 x ligation buffer (250 mM Tris-HCl; pH 7.6, 50 mM MgCI₂, 5 mM ATP, 5 mM DTT, 25% PEG-8000) and sterilized water to a final volume of 20 µl.

### 4. Transformation of E. coli

To the ligation solution (10 µl) were added 10 µl of 10 x KCM (1 M KCI, 0.3 M CaCl₂, .5 M MgCl₂), 7 µl of 30 % polyethyleneglycol (#6000), and 73 µl of sterilized water, followed by sufficient stirring. After sufficient cooling on ice, competent cells (100 µl) thawed on ice were added to the solution and stirred gently, and allowed to stand for 20 min on ice and 10 min at a room temperature. This mixture was mixed with 200 µl of LB liquid culture medium and poured on a LB liquid culture plate containing 100 µg/ml of ampicillin, and incubated for overnight at 37°C.

### 5. Preparation of plasmid DNA

A single colony of the *E*. *coli* transformed with the desired plasmid DNA was inoculated on the LB liquid culture plate (3 ml) containing 100 µg/ml of ampicillin, and cultured with shaking for overnight at 37°C. The culture medium (1.5 ml) was transferred into a 2 ml Eppendorf tube, centrifuged at 15,000 x g for 1 min. The resulting precipitate was suspended in 100 µl of TEG (25 mM Tris-HCl, 10 mM EDTA, 50 mM Glucose, pH8.0) cooled with ice, mixed with 200 µl of 0.2 M NaOH-1% SDS and stirred gently, then mixed with 150 µl of 3 M NaOAc, pH 5.2. The resulting mixture was centrifuged at 15,000 x g for 5 min at 4°C to collect a supernatant, to which was added 450 µl of phenol/chloroform/isoamylalcohol (25:24:1), stirred vigorously and subjected to centrifugation at 15,000 x g for 5 min at a room temperature to collect a supernatant. The resulting supernatant was mixed with 900 µl of ethanol cooled at -20°C, allowed to stand for 10 min at -20°C, and centrifuged at 15,000 x g for 5 min at 4°C. The resulting precipitate was rinsed with 50 µl of 70% ethanol, dried and dissolved in 50 µl of TE (10 mM Tris-HCl, 1 mM EDTA, pH8.0) containing RNaseH (100 µg/ml). The resulting plasmid was digested by *Xba*I and *Sal*I and subjected to agarose electrophoresis to confirm existence of the inserted fragment, and completion of the construction of the pNG-enoP-hyp.

### (2-2) Transformation and growth of an Aspergillus strain highly expressing hydrophobin

*Aspergillus oryzae* was transformed by the above plasmid, pNG-enoP-hyp and pNG-enoP (only vector, lacking the hydrophobin gene contained in the pNG-enoP-hyp) according to a modified protoplast PEG method. These plasmids were completely digested by *Mun*I*,* extracted with phenol and precipitated with ethanol according to a standard method, and dissolved in 10 µl of TE to serve as a DNA transformation solution.

A spore solution of *A. oryzae* niaD300 strain (nitrate reductase (niaD)-deficient variant derived from RIB40 strain) furnished from National Institute of Brewing was added to YPD liquid culture medium, and cultured with shaking for 12 hours at 30°C. The fungi were harvested by means of a glass filter. The fungi were transferred into a 50 ml centrifuge tube, suspended with 10 ml of a solution for preparing protoplasts (0.6 M KCI, 0.2 M NaH₂PO₄, pH5.5, 5mg/ml Lysing enzyme (Sigma Chemical Co.,), 10 mg/ml Cellulose Onozuka R-10 (Yakult Pharmaceutical Inc. Co.Ltd.), 10 mg/ml Yatalase (TaKaRa)), and shook for 3 hours at 90 rpm and 30 °C to prepare protoplasts. The resulting suspension were filtered by a sterilized MIRACLOTH (CALBIOCHEM), and the protoplats contained in the resulting filtrate were collected as precipitate by centrifugation at 3,000 × g, for 5 min at 4°C. The resulting precipitate was washed three times with Soll (1.2 M sorbitol, 50 mM CaCl₂, Tris-HCl buffer, pH7.5) and centrifuged at 3,000 × g, for 5 min at 4°C to give the protoplasts. The resulting protoplasts were suspended in Soll at a concentration of 1 × 10⁹ protoplasts/ml. Each of the above DNA transformation solution (10 µl) and 12.5 µl of Soll (50 (w/v)%) of PEG #4000, 50 mM CaCl₂, 10 mM Tris-HCl buffer, ,pH7.5 were mixed with 100 µl of the protoplast suspension and allowed to stand for 30 min on ice. The resulting mixture was transferred to a 50 ml centrifuge tube, mixed with 1 ml of Soll solution and 2 ml of 1.2 M sorbitol, 50 mM CaCl₂, Tris-HCl buffer, pH7.5. The resulting protoplast suspension was mixed with Czapek-Dox soft agar medium kept at 55°C, placed on Czapek-Dox agar medium and cultured at 30°C until spore was formed.

After the spore was formed, conidiophore was scraped away by means of a platinum needle and suspended in 0.01 (v/v)% Tween 80. The suspension was diluted, spreaded onto Czapek-Dox agar medium and cultured at 30° C. Monospore was separated by repeating the above procedures. The separation of the monospore was confirmed by a modified Hondel method (Spore PCR). The conidium was inoculated in a 1.5 ml micro tube containing 200 µl of YPD culture medium by means of the platinum needle and cultured for 40 hours at 30°C. The cultured fungus was transferred into another 1.5 ml micro tube, suspended with 50 µl of a solution for preparing protoplasts (0.8 M KCI, 10 mM citric acid, pH6.5, 2.5mg/ml Lysing enzyme (Sigma Chemical Co.,), 2.5 mg/ml Yatalase (TaKaRa)), allowed to stand for one hour at 37°C, heated for 3 min at 95°C, and then allowed to stand for 5 min or more on ice to precipitate the fungus. PCR was done using a 5 ml of the resulting supernatant as a template, and the following synthesized primers:
5'-ATTCGCGAAAATGGTAGCTCGAGGA-3'and
5'-GTAGAATCACGAATGAGACCTTTGACGACC-3' for pNG-enoP-hyp; and
5'-GTAGAATCACGAATGAGACCTTTGACGACC-3'and
5'-GTTAGTCGACTGACCAATTCCGCAG-3' for pNG-enoP.

PCR was carried out using the plasmid DNA for the transformation as a template for a positive control by means of TaKaRa PCR Thermal Cycler PERSONAL (TAKARA SHUZO CO., LTD.) by denaturing the template DNA for 3 min 95°C and repeating 30 cycles of 94°C for 1 min, 55.5°C for 1 min and 72° C for 90 seconds, followed by 72°C for 95 seconds for a complete extension and kept at 4° C for amplification of pNG-enoP-hyp, or by denaturing the template DNA for 3 min 95°C and repeating 30 cycles of 94°C for 1 min, 57°C for 1 min and 72°C for 90 seconds, followed by 72°C for 95 seconds for a complete extension and kept at 4°C for amplification of pNG-enoP. Agarose gel electrophoresis of the resulting PCR amplified fragment revealed its amplification at the same position as the positive control, confirming the existence of the sequence wherein hydrophobin gene was inserted downstream of the enolase promoter in the genomic DNA of the *Aspergillus* strain.

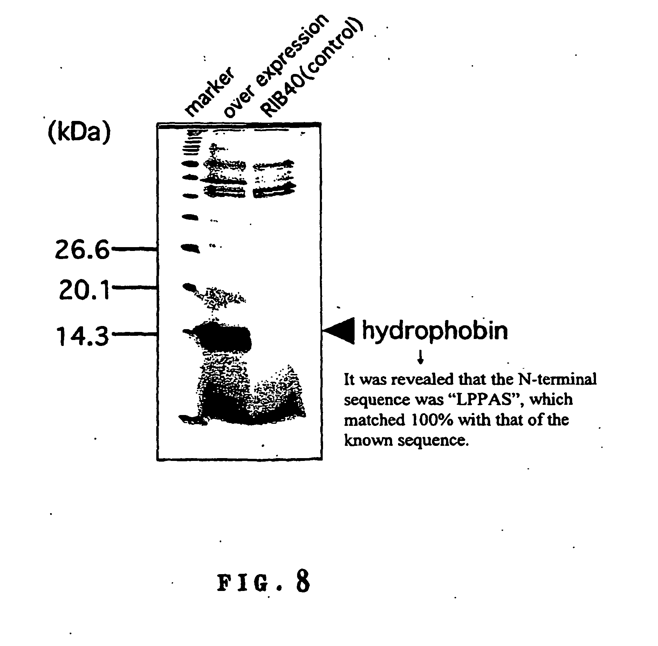

The thus transformed *Aspergillus* strain highly expressing hydrophobin and *Aspergillus* strain having no inserted hudriphobin gene (pNG-enoP) were inoculated into 200 ml of YPD culture medium (1 (w/v)% yeast extract, 2 (w/v)% peptone, 2 (w/v)% glucose) at a concentration of 2,500 spores/ml, cultured for 24 hours at 30°C with shaking, filtered with a glass filter to give culture supernatant. The supernatant (1 ml) was mixed well with 500µl of 100 (w/v)% cold trichloroacetic acid, and kept in ice for 12-16 hours. The mixture was then centrifuged at 15,000 x g, for 20 min at 4°C. After the supernatant was completely removed, the resulting precipitate was dissolved in 15µl of SDS solution (0.063M Tris-HCl buffer, pH6.8 2 (w/v)% SDS, 1 (v/v)% 2-mercaptoethanol, 10 (w/v)% glycerol, 0.05 (w/v)% Bromo Phenol Blue) and allowed to stand on a boiling bath for 5 min for SDS treatment to give a sample. SDS-PAGE and blotting to PVDF membrane were carried out according to a method of Schagger, H., et al. (1987) Anal. Biochem., 166, 368-379). A phoresis plate (160mm x 160mm x 1mm) was used. A constant current of 10 mA was applied to start electrophoresis and kept until a glycerol front line reached an end of the phoresis plate. The resulting electrophoresis gel was transferred to the PVDF membrane, and a fragment of 14.3 kDa was excised from the membrane and subjected to N-terminal amino acid sequence analysis. The result of the analysis revealed the terminal amino acid sequence of LPPAS, which was identified by homology search to be the same as that deduced from the base sequence of the *Aspergillus* fungus hydrophobin EST clone, showing the existence of hydrophobin in the supernatant. Further, CBB stain simultaneously done with the transfer to the PVDF membrane showed that no band was observed at 14.3 kDa from the culture supernatant of the *Aspergillus* strain having no inserted hydrophobin gene. As a result, it was confirmed that the high expression *Aspergillus* strain of hydrophobin secreted hydrophobin not only on its surface but also into the culture supernatant (Fig.8).

The high expression *Aspergillus* strain of hydrophobin transformed by the plasmid DNA (pNG-enoP-hyp) was deposited at the International Patent Organism Depository of National Institute of Advanced Industrial Science and Technology (1-1, Higashi 1-chome, Tsukuba-shi, Ibaraki Japan) on 4 October, 2002 with an accession No. FERM P-19055.

### Example 3

### (3) Isolation of gene of biodegradable plastic-degrading enzyme, cutinase, and growth of an Aspergillus strain highly expressing the same enzyme

A degrading enzyme may be one of degrading factors of biodegradable plastic. A gene of biodegradable plastic-degrading enzyme was therefore obtained and an *Aspergillus* strain highly expressing the same enzyme was cultured using PBS as biodegradable plastic.

### (3-1) Purification of PBS-degrading enzyme form Aspergillus oryzae

RIB40 spore solution was inoculated at a final concentration of 0.5x10⁶ spores/ml in Czapek-Dox medium containing 34 µg/ml of chloramphenicol (Nkajima, K., et al. 2000, Curr. Genet., 37, 322-327) in a conical flask (500 ml) containing 100 ml of 1 (v/w)% PBS emulsion (SHOWA HIGHPOLYMER CO., LTD.) as an only carbon source, and cultured for 5 days at 30°C and 125 rpm. The culture medium was filtered by means of MIRACLOTH (Calbiochem) and the resulting filtrate was centrifuged at 8,000 g for 20 min at 4°C to give a supernatant to be used as a crude enzyme solution. The crude enzyme solution was then mixed with ammonium sulfate up to 20 % saturation and centrifuged at 8,000 g for 20 min at 4°C to give a supernatant fraction. The resulting supernatant fraction was applied to Octyl-Cellulofine column (SEIKAGAKU CORPORATION) equilibrated with 10 mM Tris-NCI buffer, pH6.8 and 20% saturated ammonium sulfate and an adsorbed fraction was eluted with a linear concentration gradient of 20 - 0 % saturated ammonium sulfate. To the resulting fractionated solution was added PBS emulsion to a final concentration of 0.1 (v/v)%, and an active fraction was monitored according to decrease in turbidity (degradation of PBS). The active fraction thus obtained was dialysed against 10 mM Tris-HCl buffer, pH8.0 and applied to DEAE-TOYOPEAL 650S column (TOSOH CORPORATION) equilibrated with the same buffer. An adsorbed fraction was eluted with a linear concentration gradient of 0 - 0.3 M NaCl. The resulting active fraction was dialysed against 10 mM MES buffer, pH5.5 and applied to HiTrap SP column (Amershampharmacia Biotech) equilibrated with the same buffer. An adsorbed fraction was eluted with a linear concentration gradient of 0 - 0.3 M NaCl. The resulting active fraction was subjected to SDS-PAGE (Laemmli, U.K. (1970) Nature, 227, 680-685) to show electrophoretic homogeneity, confirming that the resulting active fraction was a purified sample of PBS-degrading enzyme.

### (3-2) Determination of an internal amino acid sequence of PBS-degrading enzyme

The purified enzyme sample (250 µl: 0.4 mg/ml) obtained above was mixed well with 100 (w/v) % of cold trichloroacetic acid (200 µl) and allowed to stand on ice for 12- 16 hours. The resulting sample was centrifuged at 15,000 g for 20 min at 4°C. After the supernatant was completely removed, the precipitate was dissolved in 50 µl of SDS solution (0.063M Tris-HCl buffer, pH6.8, 2 (w/v)% SDS, 1 (v/v)% 2-mercaptoethanol, 10 (w/v)% glycerol) and allowed to stand in a boiling bath for 5 min for SDS treatment to give a sample.

V8 protease (6.3 µg) was dissolved in 25µl of solution containing 0.05 (w/v)% Bromo Phenol Blue, 0.019M Tris-HCl buffer, pH6.8, 0.6 (w/v)% SDS, 0.3 (v/v)% 2-mercaptoethanol, 3 (w/v)% glycerol to give V8 protease solution. SDS-PAGE and blotting to a PVDF membrane were carried out according to a method of Schagger, H., et al. (1987) Anal. Biochem., 166, 368-379). A phoresis plate (160mm x 160mm x 1mm) was used. The sample solution (50 µl) was applied on a well and 25 µl of the V8 protease solution was placed on it. The electrophoresis was kept with a constant current of 15 mA until the dye (Bromo Phenol Blue) reached about a half point of a concentration gel. After the gel was kept for one hour at a room temperature for a partial degradation by V8 protease within the gel, the electrophoresis was then re-started. After the completion of the electrophoresis, proteins in the electrophoresis gel were transferred to the PVDF membrane, and fragments of 7.9 kDa, 10.3 kDa, 11.9 kDa were excised from the membrane and subjected to a terminal amino acid sequence analysis (Matsudaira, P. (1987) J. Biol. Chem., 262, 10035-10038). The results of the analysis of the fragments of 10.3 kDa and 11.9 kDa revealed that a terminal amino acid sequence of both the fragments were AQGLFEQAVS. The resulting amino acid sequence was subjected to BLAST search in National Center for Biotechnology Information (NCBl;http://www.ncbi.nim.nih.gov/) (Zhang, J., et al.,(1997) Genome Res., 7, 649-656) for homology research to reveal that the above terminal amino acid sequence coincided with an internal amino acid sequence of cutinase of *Aspergillus oryzae* (Ohnishi, k., et al. (1995) FEMS Microbiol. Lett., 126 (2), 145-150).

### (3-3) Cloning of a genomic DNA comprising a gene of the degrading enzyme

A genomic DNA was prepared from mycelia of *Aspergillus oryzae* in order to obtain a genomic DNA comprising a gene of the degrading enzyme. RIB40 spore solution was inoculated at a final concentration of 0.5x10⁶ spores/ml in YPD liquid culture medium (1 (w/v)% yeast extract, 2 (w/v)% peptone, 2 (w/v)% glucose) in a conical flask (500 ml), and cultured for 16 hours at 30°C and 125 rpm with shaking, filtered with a glass filter to collect mycelia. The resulting mycelia was washed with distilled water, poured into a triturator cooled at -20°C and frozen with liquid nitrogen and finely crushed by means of a cooled pestle. The crushed mycelia was taken with a spatula into a micro tube (1.5 ml), suspended with 0.4 ml of TE (10 mM Tris-HCl buffer pH 8.0. 1mM EDTA), mixed gently with 0.4 ml of a bacteriolysis solution (2 (w/v)% SDS, 0.1 M NaCl, 10 mM EDTA, 50 mM Tris-HCl buffer, pH 7.0) and was allowed to stand for 15 min at a room temperature. After a centrifugation at 15,000 x g for 10 min, the resulting supernatant was taken into another micro tube (1.5 ml). After the addition of an equal amount of phenol-chloroform-isoamylalcohol (25:24:1), the tube was shaken gently upside down and centrifuged at 15,000 x g, 5 min to collect the resulting supernatant in another micro tube (1.5 ml). After the addition of ethanol cooled at -20 ° C in an amount 2.5 times as much as that of the supernatant, the mixture was allowed to stand for 10 min at -20°C, centrifuged at 15,000 x g for 15 min at 4 ° C to completely separate precipitate from supernatant. The precipitate was dissolved in 0.5 ml of TE, mixed with 5 µl of RNase A solution (10mg/ml) and to be allowed to stand for 30 min at 37°C to decompose RNA. After the addition of 0.5 ml of phenol-chloroform-isoamylalcohol (25:24:1), the tube was shaken gently upside down and centrifuged at 15,000 x g, 5 min to collect the resulting supernatant in another micro tube (1.5 ml). These procedures were repeated once. After the addition of 0.5 ml of chloroform-isoamylalcohol (24:1), the tube was shaken gently upside down and centrifuged at 15,000 x g, 5 min to collect the resulting supernatant in another micro tube (1.5 ml). After the addition of 50 µl of 5 M NaCl and 1 ml of ethanol cooled at -20°C, the mixture was allowed to stand for 10 min at -20°C, centrifuged at 15,000 x g for 15 min at 4°C to completely separate precipitate from supernatant. The resulting DNA was washed with 70 (V/v)% ethanol, centrifuged at 15,000 x g for 10 min at 4°C to completely separate precipitate from supernatant. The resulting precipitate was dissolved in 0.1 ml of TE to serve as a genomic DNA solution.

An oligonucleotide of 30 base pairs:
5'-GCACAAGGACTGTTTGAACAAGCTGTTTCC-3'), and
an oligonucleotide of 30 base pairs:
(5'-CCAGGCAGACAAGATCTCCCACGGCGCAAT-3')
were synthesized on the basis of the above internal amino acid sequence and the base sequence of known cutinase, respectively. PCR was carried out using the genomic DNA (100 ng) as a template, Ex taq polymerase (TAKARA SHUZO CO., LTD.) and the resulting pair of primer set with TaKaRa PCR Thermal Cycler PERSONAL (TAKARA SHUZO CO., LTD.) to amplify a probe for southern hybridization. The amplification was done denaturing the template DNA for 3 min at 95°C, and repeating 30 cycles of 95°C for 1 min, 60°C for 1 min, 72°C for 30 seconds, and 72°C for 1 min for a complete extension, followed by being kept at 4°C. Agarose gel electrophoresis of the resulting PCR amplified fragments revealed amplification of a PCR fragment having 300 base pairs.

Southern hybridization and colony hybridization were carried out using NEN RandomPrimer Fluorescein Labeling Kit with Antifluorescein-AP (Eizo Diagnostics, Inc.). SSC solution was prepared by optionally diluting 20 x SSC (autoclaved 1 l aqueous solution containing 175.4 g of NaCl and 88.2 g of Sodium Citrate Dihydrate). The genomic DNA (10 µg) was completely digested by 50 units of EcoR V (TAKARA SHUZO CO., LTD.) and subjected to agarose gel electrophoresis. A glass plate was placed in a tray containing an alkali transfer buffer (0.4 N NaOH), and a filter paper (ADVANTEC TOYO) cut into such a size that it could reach the transfer buffer was then placed on the glass plate and got wet with it. The gel obtained after the electrophoresis was then placed on the paper, and nylon membrane (Hybond-N+; Amershampharmacia Biotech) was then placed on the gel, and three sheets moisturized with sterilized water were further placed on the membrane. Finally paper towel was pile up on the filter paper to 5 cm high and a weighting was put on the top of the paper towel. After blotting for 12 hours, the membrane was washed with 4 x SSC and subjected to hybridization.

The above PCR solution was mixed with NaCl (5 M) in an amount one tenth that of the PCR solution and cooled ethanol in an amount 2.5 times as much as that of the same solution, allowed to stand for 10 min at -20°C and centrifuged at 15,000 x g, for 15 min at 4°C to completely separate precipitate from supernatant. The DNA precipitate was washed with 70 (v/v)% ethanol and centrifuged at 15,000 x g, for 10 min at 4°C to completely separate precipitate from supernatant. The precipitate was dissolved in TE at a final concentration of 1µg/µl. The resulting DNA solution (20 µl) was mixed well by pipetting with Random Primers and Reaction Buffer Mix 5 µl, Fluorescein Nucleotide Mix 5 µl and Klenow Fragment 1 µl of NEN RandomPrimer Fluorescein Labeling Kit with Antifluorescein-AP. After reaction for one hour at 37°C, the mixture was mixed with 0.5 µl of 0.1 M EDTA, pH 8.0 and hybridization buffer (2 x SSC, 0.1 (w/v)% SDS, 5 (w/v) % Dextran sulphate, 0.5 (v/v)% Blocking Reagent) to give a probe solution with a concentration of 20 ng/ml.

The membrane after blotting was rinsed with 2 x SSC, transferred to a hybridization bag, mixed with the hybridization buffer to give 0.1 ml/cm², and with Carrier DNA to give a final concentration of 50 µg/ml, and finally allowed to stand for one hour at a room temperature. The hybridization buffer (200 µl), the probe solution (20 µl) and 35 µl of Carrier DNA solution (10 mg/ml) were mixed in a micro tube, boiled for 5 min at 100°C and rapidly cooled and kept in ice. For 5 min The resulting solution, which had been pre-heated at 60°C, was replaced with the solution in the hybridization bag and kept for 16 hours at 60°C. The membrane was picked up from the hybridization bag, washed with vigorous shaking by 1 ml/cm² of the membrane or more of 2 x SSC, 1 (w/v)% SDS for 15 min at 60°C, then by 1 ml/cm² of the membrane or more of 0.2 x SSC, 0.1 (w/v)% SDS for 15 min at 60°C. The following procedures were done at a room temperature. The membrane was washed with vigorous shaking by 1 ml/cm² of the membrane or more of 0.1 M Tris-HCl buffer, pH 7.5, 0.15 M NaCl for 5 min. Then the membrane was washed in a plastic bag with shaking by 0.1 ml/cm² of the membrane or more of 0.1 M Tris-HCl buffer, pH 7.5, 0.15 M NaCl and 0.5 (v/v)% Blocking Reagent for one hour, and then washed with shaking by 0.1 ml/cm² of the membrane or more of 0.1 M Tris-HCl buffer, pH 7.5, 0.15 M NaCl, 0.5 (v/v)% Blocking Reagent and 1/1000 (v/v) Antifluorescein-AP Conjugate for one hour. The membrane was picked up, and washed four times with vigorous shaking by 0.1 M Tris-HCl buffer, pH 7.5, 0.15 M NaCl for 5 min each, and two times with vigorous shaking by 0.1 M Tris-HCl buffer, pH 9.5, 0.1 M NaCl for 5 min each. The membrane was transferred into another plastic bag, mixed with 1 ml of ECF substrate Auto Phos (Eizo Diagnostics, Inc.) to react for 30 min under light shielding. The membrane was picked up from the plastic bag, kept under light shielding for further 4 hours, and subjected to a fluorescence analyzer (FLA-2000: FUJIFILM). As a result, it was detected that a band consisting of about 1,300 base pairs strongly hybridized.

The genomic DNA (10 µg) was completely digested by 50 units of *Eco*R V and subjected to agarose gel electrophoresis. After staining with ethidium bromide, a part of gel around 1,300 base pairs was excised under UV irradiation. DNA was extracted from the gel using prep A gene (BioRad) to be used as an insertion DNA fragment.

A plasmid, pBluescript II KS+DNA (Stratagene), 2.5 µg, was digested completely by EcoR V (TAKARA SHUZO CO., LTD.) and subjected to conventional phenol extraction and ethanol precipitation, followed by removal of phosphoric acid at the 5' end by alkaline phosphatase (TAKARA SHUZO CO., LTD.). The resulting solution was subjected to conventional phenol extraction and ethanol precipitation and dissolved in TE to be used as a vector DNA solution.

The insertion DNA fragment (1.5 µg) was ligated with the vector DNA (1 µg) by T4 DNA ligase (TAKARA SHUZO CO., LTD.) to give a ligated DNA solution. Competent *E. coli* DH5α (TAKARA SHUZO CO., LTD.) 100 µl was added to a mixture of the ligated DNA solution (10 µl) and 10 µl of 10 × KCM (1 M KCI, 0.3 M CaCI₂, 0.5 M MgCl₂), 7 µl of 30 (w/v)% PEG#6000, and 73 µl of sterilized water, allowed to stand for 20 min on ice and for 10 min at a room temperature to give a transformed *E. coli* suspension. The transformed E. coli suspension was inoculated on a nylon membrane (Hybond-N+; Amershampharmacia Biotech) placed on LB agar medium containing 50 µg/ml of ampicillin, and cultured for 16 hours at 37°C. The membrane was peeled away from the medium, soaked in 10 (w/v) % SDS for 3 min, in 0.5 N NaOH and in 1.5 M NaCl for 10 min, and in 1 M Tris-HCl buffer pH 8.0, 1.5 M NaCl to remove the solution, followed by washing with 2 × SSC. The membrane was then soaked in 0.4 N NaOH for one hour and washed with 2 × SSC. The resulting membrane was hybridized with the probe in accordance with the same procedures as in southern hybridization to give a positive signal.

*E. coli* having a plasmid showing the positive signal was scraped away from the agar medium by means of a platinum needle, transferred in to LB liquid culture medium (50 µg of ampicillin) and cultured with shaking for 16 hours at 37°C. The culture medium (1.5 ml) was transferred into a 1.5 ml micro tube and centrifuged at 15,000 × g, 1 min to remove the supernatant. The resulting bacteria was suspended in 100 µl of TEG (25 mM Tris-HCl buffer, pH 8.0, 10 mM EDTA, 50 mM glucose), mixed gently with 200 µl of 1 (w/v) % SDS, 0.2 N NaOH and allowed to stand on ice for 5 min. The mixture was mixed gently with 150 µl of 3 M sodium acetate, pH 5.2 and allowed to stand on ice for 5 min. The mixture was then mixed gently with 150 µl of 10 M ammonium acetate and centrifuged at 15, 000 × g, for 10 min at 4°C. The resulting supernatant was transferred to another 1.5 ml micro tube, mixed well with 600 µl of 2-propanol and centrifuged at 15, 000 × g, for 10 min at 4°C to remove supernatant. The resulting DNA was washed with 70 (v/v)% ethanol and centrifuged at 15, 000 × g, for 10 min at 4°C to completely separate supernatant from precipitate. The precipitate was dissolved in TE to be used as a plasmid solution. The base sequence of the insertion DNA fragment comprised in the plasmid DNA was analyzed by means of ABI PRISM^{™} 377 DNA sequencing system according to a protocol of ABI PRISM^{™} 377 DNA sequencer Long Read (PE Biosystem). As a result, an open reading frame comprising the base sequence of the probe was found in the EcoR V digested fragment (1334 base pairs) of the genomic DNA. It was also revealed that the open reading frame comprised three intron base sequences, "ATG" encoding an initiation methionine and a stop codon, showing that it contained a full length of a gene of the PBS-degrading enzyme (Ohnishi K. et al., (1995) FEMS Microbiol Lett., 162(2), 145-150).

### (3-4) Construction of a high expression system of the PBS-degrading enzyme

The plasmid DNA (5 µg) comprising the full length of the gene of the PBS-degrading enzyme was completely digested by 50 units of EcoR V and subjected to agarose gel electrophoresis. After staining with ethidium bromide, a fragment having 1,334 base pairs was excised from the gel under UV irradiation. DNA was extracted using prep A gene (BioRad) to be used as an insertion DNA fragment. A plasmid, pNGA142, DNA comprising a glucoamylase promoter (PglaA 142) sequence (TAKARA SHUZO CO., LTD.), 5 µg, was digested by *Pma*C I (TAKARA SHUZO CO., LTD.) at a PmaC I recognition site just after the promoter sequence, and subjected to conventional phenol extraction and ethanol precipitation, followed by removal of phosphoric acid at the 5' end by alkaline phosphatase (TAKARA SHUZO CO., LTD.). The resulting solution was subjected to conventional phenol extraction and ethanol precipitation and dissolved in TE to be used as a vector DNA solution.

The insertion DNA fragment (1.5 µg) was ligated with the vector DNA (1 µg) by T4 DNA ligase (TAKARA SHUZO CO., LTD.) to give a ligated DNA solution. Competent *E. coli* DH5α (TAKARA SHUZO CO., LTD.) 100 µl, was added to a mixture of the ligated DNA solution (10 µl) and 10 µl of 10 x KCM (1 M KCI, 0.3 M CaCI₂, 0.5 M MgCI₂), 7 µl of 30 (w/v)% PEG#6000, and 73 µl of sterilized water, allowed to stand for 20 min on ice and for 10 min at a room temperature to give a transformed E. coli suspension. The transformed *E*. *coli* suspension was inoculated on LB agar medium containing 50 µg/ml of ampicillin, and cultured for 16 hours at 37°C. Colony on the medium was scraped away by means of a platinum needle, transferred in to LB liquid culture medium (50 µg of ampicillin) and cultured with shaking for 16 hours at 37°C. The plasmid DNA was extracted from the cultured strains in the same manners as above to give a plasmid DNA for the transformation of *Aspergillus oryzae* (pNG-cut). The resulting plasmid DNA (10 µg) was digested by *Mun*I and subjected to conventional phenol extraction and ethanol precipitation, and dissolved in 10 µl of TE to be used as a DNA solution for transformation. The transformation of *Aspergillus oryzae* was carried out according to a modified protoplast-PEG method of Vollmer et al (Vollmer, S.J., et al. (1986) Proc. Natl. Acad. Sci. USA, 83, 4869-4873), and as described more in detail in the Example 2 (2-2) of the present specification.

After the spore was formed, conidiophore was scraped away by means of a platinum needle and suspended in 0.01 (v/v%) Tween 80. The suspension was appropriately diluted, poured onto Czapek-Dox agar medium and cultured at 30° C. Monospore was separated repeating the above procedures. The separation of the monospore was confirmed by a modified Hondel method (Spore PCR method: van, Zeiji, C.M., et al. (1997) J. Biotechnol., Jan 3; 59 (3), 221-224) and as described more in detail in the Example 2 (2-2) of the present specification. The following two oligomers were synthesized as the primers for spore PCR:
5'-TGCAGTGGCGGATCCGGTGGAC-3', and
5'-GTAGAATCACGAATGGAGCCTTTGACGACC-3'.

PCR was carried out by means of TaKaRa PCR Thermal Cycler PERSONAL (TAKARA SHUZO CO., LTD.) using the plasmid DNA for the transformation of *Aspergillus oryzae* (pNG-cut) as a template in a positive control and Ex Taq polymerase (TAKARA SHUZO CO., LTD.). Amplification was done by denaturing the template DNA for 3 min at 94 °C and repeating 30 cycles of 94°C for 1 min, 55.5°C for 1 min and 72°C for 90 seconds, followed by 72°C for 95 seconds for a complete extension and kept at 4 ° C. Agarose gel electrophoresis of the resulting PCR amplified fragment revealed the amplification of a PCR fragment at the same position as the positive control, confirming that the base sequence wherein the PBS-degrading enzyme gene had been ligated downstream of the glucoamylase promoter was inserted in the genomic DNA, and that the transformed *Aspergillus oryzae* comprising the above base sequence was obtained.

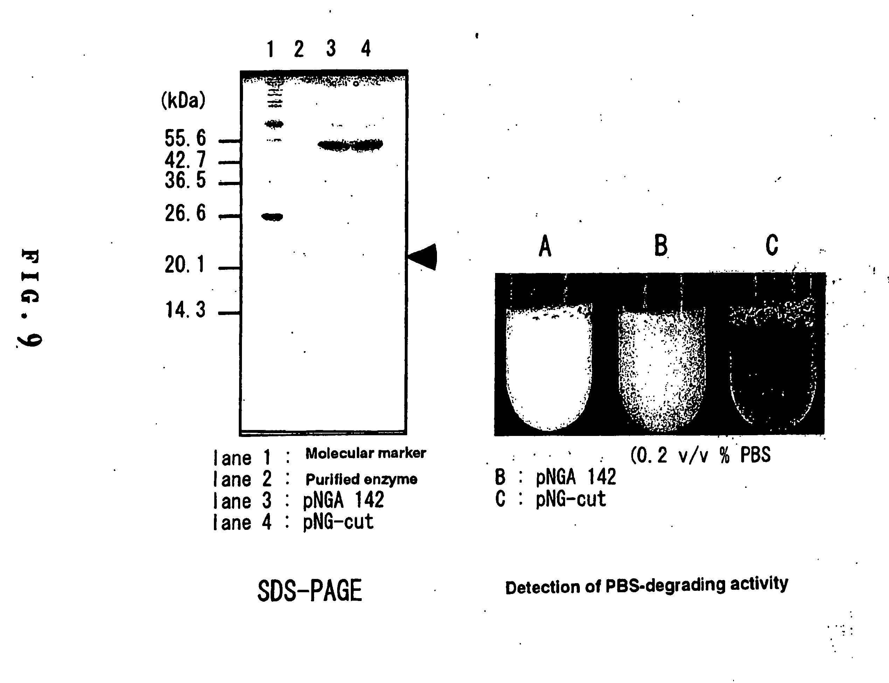

Spore suspension of the thus transformed strain of *Aspergillus oryzae* having the PBS-degrading enzyme gene inserted in the genomic DNA, and that of the transformed strain of *Aspergillus oryzae* having no inserted gene (i.e., transformed with pNGA142) were inoculated into 100 ml of YPM culture medium (1 (w/v)% yeast extract, 2 (w/v)% peptone, 2 (w/v)% maltose) containing maltose as an inducing substance for the glucoamylase promoter at a concentration of 0.5 x 10⁶ spores/ml, respectively, cultured for 16 hours at 30° C and 125 rpm, filtered with MIRACLOTH (CALBIOCHEM^{™}) to give culture supernatant. PBS emulsion was added to the supernatant at a final concentration of 0;1 (v/v)% and kept at 37°C to detect decrease in turbidity (degradation of PBS), observing the expression of the PBS-degrading enzyme. As a result, activity of the PBS-degrading enzyme was confirmed only in the supernatant of the transformed strain of *Aspergillus oryzae* having the PBS-degrading enzyme gene inserted in the genomic DNA. The supernatant (100 µl) was mixed with 50µl of cold trichloroacetic acid, and kept in ice for 20 min. The mixture was centrifuged at 15,000 x g, for 15 min at 4°C. After the supernatant was completely removed, the resulting precipitate was dissolved in 15µl of SDS solution (0.063M Tris-HCl buffer, pH6.8, 2 (w/v)% SDS, 1 (v/v)% 2-mercaptoethanol, 10 (w/v)% glycerol, 0.05 (w/v)% Bromo Phenol Blue) and allowed to stand in a boiling bath for 5 min for SDS treatment to give a sample. SDS-PAGE of the resulting sample and the above-mentioned purified sample of PBS-degrading enzyme revealed expression of a protein at the same position as the purified sample only with respect to the supernatant of the transformed strain of *Aspergillus oryzae* having the PBS-degrading enzyme gene inserted in the genomic DNA (Fig. 9).

The *Aspergillus* strain highly expressing the biodegradable plastic-degrading enzyme, cutinase, which was transformed by the plasmid DNA (pNG-cut) was deposited at the International Patent Organism Depository of National Institute of Advanced Industrial Science and Technology (1-1, Higashi 1-chome, Tsukuba-shi, Ibaraki Japan) on 4 October, 2002 with an accession No. FERM P-19054.

### Example 4

### (4) Experiment of degradation of plastic in a liquid culture system by single culture of Aspergillus oryzae highly expressing hydrophobin or cutinase, and by their co-culture

Each of *Aspergillus* strain highly expressing hydrophobin (pNG-enoP-hyp) and cutinase (pNG-cut), and the strain (pNGA142) prepared by transformation of a host strain RIB 40 with the vector (control) were inoculated at a concentration of 1 x 10⁶ spores/ml, respectively, in Czapek-Dox minimum medium in Example 1 containing PBS emulsion (0.2 %) and maltose as an inducing material (0.5%) and cultured for 2 days at 30°C. Co-culture of the *Aspergillus* strains highly expressing hydrophobin and cutinase (pNG-cut + pNG-enoP-hyp) was also done in the same way. On the second day of the culture, the culture medium was filtered through MIRACLOTH to remove fungi, and turbidity (630 nm) of the filtered culture medium was measured. The results are shown in Table 3. The starting turbidity was 8.73 (OD₆₃₀). It was shown that the degree of decrease in turbidity was as follows: co-culture>cutinase-expressing strain>hydrophobin-expressing strain>control.

**[Table 3]**

| Strain | Remaining turbidity of emulsion PBS (OD₆₃₀) |
|---|---|
| Control (pNGA142) | 4.99 |
| pNG-cut+pNG-enoP-hyp | 1.53 |
| pNG-cut | 3.26 |
| pNG-enoP-hyp | 4.16 |

### Example 5

### (5) Experiment of degradation of plastic in a solid culture system by single culture of Aspergillus oryzae highly expressing hydrophobin or cutinase, and by their co-culture

Each of the strains used in Example 4 was inoculated at a concentration of 1 x 10⁷ spores/ml, respectively, in Czapek-Dox minimum medium containing PBS powder (0.5 (w/v) %) and maltose (0.5%) and cultured for 8 days at 30°C. The PBS fine powder taken by the fungi were washed by being mixed with about 25 ml of sterilized water in 50 ml Falcon tube and stirred by means of a vortex mixer so as to completely separate the fungi and the PBS fine powder. The washing solution was filtered through MIRACLOTH and the resulting filtrate was centrifuged (10,000 rpm, 15 min). The resulting supernatant was then diluted with the minimum medium up to the same amount as that under the starting conditions of the culture. The resulting suspension was further diluted 300 times and its turbidity was measured at 630 nm. The results are shown in Table 4.

It was shown from the results that the growth rate of both the *Aspergillus* strains highly expressing hydrophobin (pNG-enoP-hyp) and cutinase (pNG-cut) in the culture using solid powder PBS was significantly increased compared with that of RIB 40 strain control, confirming the increase of a PBS-converting activity of the above high expression strains.

**[Table 4]**

| Strain | Remaining turbidity of solid PBS (OD₆₃₀) | Degradation ratio rate (%) |
|---|---|---|
| Control (pNGA142) | 0.893 | 7.7 |
| pNG-cut+pNG-enoP-hyp | 0.717 | 26 |
| pNG-cut | 0.519 | 46 |
| pNG-enoP-hyp | 0.243 | 75 |

### Example 6

### (6) Production of a useful substance by co-culture of the Aspergillus oryzae highly expressing hydrophobin and cutinase, and a strain highly expressing an enzyme (useful substance) involved in biosynthesis of the useful substance

As an example of the useful substance, α-amylase for industry was produced in conjugation with the degradation of biodegradable plastic, PBS. Co-culture of the *Aspergillus* strains highly expressing hydrophobin (pNG-enoP-hyp), cutinase (pNG-cut), respectively, and co-culture of the *Aspergillus* strains highly expressing hydrophobin (pNG-enoP-hyp), cutinase (pNG-cut) and α-amylase (pNG-amy), respectively, were carried out in the same culture medium as in Example 4. The strain (pNGA142) prepared by transformation of a host strain RIB 40 with the vector having no inserted gene was used as a control. The RIB 40 transformant having pNG-amy was prepared by transforming RIB 40 strain by pNGA142 comprising the α-amylase gene (S. Tada et al.: Agric. Biol. Chem., 53, 593-599 (1989)) inserted therein. Each of the strains was inoculated at a concentration of 1 x 10⁶ spores/ml, respectively, in Czapek-Dox minimum medium used in Example 4 and cultured for 2 days at 30°C. The culture medium was filtered through MIRACLOTH and the resulting filtrate was centrifuged (15,000 x g, 10 min, 4°C) to completely remove the remaining PBS from the medium. The resulting supernatant was diluted 100 times with water to be used in the determination of α-amylase activity by iodine color reaction. Solution A: 50 mM Tris buffer (pH 6.8) containing soluble starch (0.3 %) dissolved therein; and Solution B: 1N HCl containing 2 × 10⁻⁴ % iodine and 2 × 10⁻³ % potassium iodine were prepared as a reagent solution for the determination. Solution A (400 µl) was mixed the diluted culture supernatant (50 µl), reacted for 10 min at 37°C, then mixed well with Solution B (50 µl) to immediately measure absorbance at 620 nm. The degree of decrease in absorbance compared to blank (non-inoculated) was shown as a relative activity of α-amylase in Table 5. The degree of decrease of PBS was as follows: triple-culture=co-culture>control, and the degree of induction of α-amylase was as follows: triple-culture=co-culture>control, showing that the α-amylase for industry could be produced in the above triple-culture utilizing PBS monomers provided by the PBS degradation as energy source.

**[Table 5]**

| Strain | Remaining turbidity of PBS (OD₆₃₀) | Relative activity of α-amylase (OD₆₂₀) |
|---|---|---|
| Blank | 10.44 | 0 |
| Control (pNGA142) | 3.18 | 0 |
| pNG-cut+pNG-enoP-hyp | 0.47 | 0.020 |
| pNG-cut+pNG-enoP-hyp + pNG-amy | 0.36 | 0.134 |

### Example 7

### (7) Preparation and growth of a strain highly co-expressing cutinase and hydrophobin

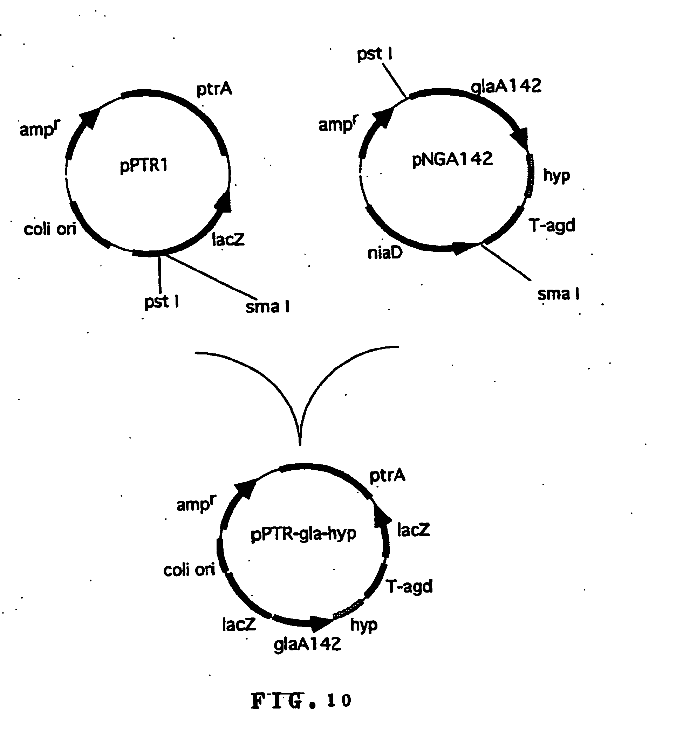

For the preparation of a strain highly co-expressing cutinase and hydrophobin using the strain highly expressing cutinase obtained in Example 3-4, a plasmid highly expressing hydrophobin, pPTR-gla-hyp, was constructed by fusing a glaA 142 promoter that could be strongly induced by maltose upstream of a target gene with the use of an expression vector for Aspergillus fungi, pPTR 1 vector (Fig.10).

### (7-1) Construction of pPTR-gla-hyp

### [Materials]

The promoter pPTR-gla-hyp was constructed by using the two vectors, pNGA 142 and pPTR 1. The plasmid pNG-enoP-hyp constructed in Example 2 was used to prepare a product for insertion..

### [Method]

### 1. Cut of DNA by a restriction enzyme

pNGA 142 and pNG-enoP-hyp were digested by *Xbal* and *Sal*I, respectively.
2. Preparation of a vector DNA and inserted DNA
3. Ligation
4. Transformation of *E*. *coli*
5. Preparation of a plasmid DNA

The above steps 2.-5. were carried out in the same manner as in Example 2. The resulting plasmid was cut by *Xba*I and *Sal*I and subjected to agarose electrophoresis to confirm the existence of an inserted fragment and the plasmid, and the completion of pNG-gla-hyp.

### 6. Cut of DNA by a restriction enzyme

pPTR1 and pNG-gla-hyp were digested by *Pst*I and *Sma*I, respectively.
7. Preparation of a vector DNA and inserted DNA
8. Ligation
9. Transformation of *E*. *coli*
10. Preparation of a plasmid DNA

The above steps 7.-10. were carried out in the same manners as in Example 2. The resulting plasmid was cut by *Pst*I and *Sma*I and subjected to agarose electrophoresis to confirm the existence of an inserted fragment and the plasmid, and the completion of pPTR-gla-hyp.

### (7-2) Growth of a strain highly co-expressing cutinase and hydrophobin

The *Aspergillus oryzae* strain (the strain highly expressing cutinase obtained in Example 3-4) was transformed by the above plasmid, pPTR-gla-hyp according to the modified protoplast PEG method. The plasmid (10 µg) was completely digested with Sacll, extracted with phenol and precipitated with ethanol according to a standard method, and dissolved in 10 µl of TE to serve as a DNA transformation solution. The following procedures for the transformation of the *Aspergillus oryzae* were done in accordance with those in Example 2-2.

However, a protoplast regeneration medium, Czapek-Dox soft agar medium contained 100µg/ml pyrithiamine (TaKaRa). The spore formed on the medium was inoculated on Czapek-Dox agar medium containing pyrithiamine in order to separate monospore.

The following PCR primers for the spore of pPTR-gla-hyp were synthesized:
5'-ATTCGCGAAAATGGTAGCTCGAGGA-3'and
5'-CTGTGTCCCGTATGTAACGGTG -3'

PCR was carried out using the plasmid DNA for the transformation as a template in a positive control by means of TaKaRa PCR Thermal Cycler PERSONAL (TAKARA SHUZO CO., LTD.) by denaturing the template DNA for 3 min 95°C and repeating 30 cycles of 94°C for 1 min, 55.5°C for 1 min and 72° C for 90 seconds, followed by 72°C for 95 seconds for a complete extension and kept at 4°C for amplification. Agarose gel electrophoresis of the resulting PCR amplified fragment revealed its amplification at the same position as the positive control, confirming the existence of the hydrophobin gene inserted downstream of the gla142 promoter in the genomic DNA of the *Aspergillus oryzae.*

The thus transformed high co-expression *Aspergillus* strain of cutinase and hydrophobin was inoculated into 200 ml of YPD culture medium (Example2 (2-2)) at a concentration of 1 x 10⁶ spores/ml, cultured for 24 hours at 30°C with shaking, filtered with a glass filter to give culture supernatant. The supernatant (200 µl) was mixed well with 100µl of 100 (w/v)% cold trichloroacetic acid, and kept in ice for 12-16 hours. The mixture was centrifuged at 15,000 x g, for 20 min at 4°C. After the supernatant was completely removed, the resulting precipitate was dissolved in 15 µl of SDS solution (Example 2 (2-2)) and allowed to stand in a boiling bath for 5 min for SDS treatment to give a sample. The following SDS-PAGE procedures were done in accordance with those in Example 2 (2-2). A band with 14 kDa that was not observed in the high-expression strain of cutinase was confirmed by SDS-PAGE and CBB staining, showing that the *Aspergillus* strain highly co-expressing cutinase and hydrophobin was prepared.

### Example 8

### (8) Experiment of degradation of plastic in a liquid culture system by the Aspergillus oryzae strain highly expressing hydrophobin or cutinase, and the strain highly co-expressing hydrophobin and cutinase

Each of the *Aspergillus* strain highly expressing hydrophobin prepared in Example 2, the *Aspergillus* strain highly expressing cutinase prepared in Example 3, and the *Aspergillus* strain highly co-expressing cutinase and hydrophobin prepared in Example 7, and the strain of pNGA142 as a control were inoculated at a concentration of 1 × 10⁶ spores/ml, respectively, in YPM medium (1 (w/v) % yeast extract, 2(w/v)% peprone, 4 (w/v) % maltose) containing PBS emulsion (0.1 %) and cultured for 24 hours at 30°C. The culture medium was filtered through MIRACLOTH to remove fungi, and turbidity (630 nm) of the filtered culture medium was measured. The results are shown in Table 6. It was shown that the degree of decrease in turbidity was as follows: the *Aspergillus* strain highly co-expressing cutinase and hydrophobin > the *Aspergillus* strain highly expressing cutinase > the *Aspergillus* strain highly expressing hydrophobin > control (pNGA 142 strain).

**[Table 6]**

| Strain | Turbidity (OD630) | Degradation rate (%) |
|---|---|---|
| Blank | 1.103 | |
| PNGA142 | 1.023 | 7.25 |
| Strain highly expressing hydrophobin | 0.831 | 24.66 |
| Strain highly expressing | | |
| Cutinase | 0.436 | 60.47 |
| Strain highly co-expressing cutinase and hydrophobin | 0.383 | 65.28 |

### Example 9

### (9-1) Preparation and growth of a strain triple-co-expressing cutinase-hydrophobin-amylase

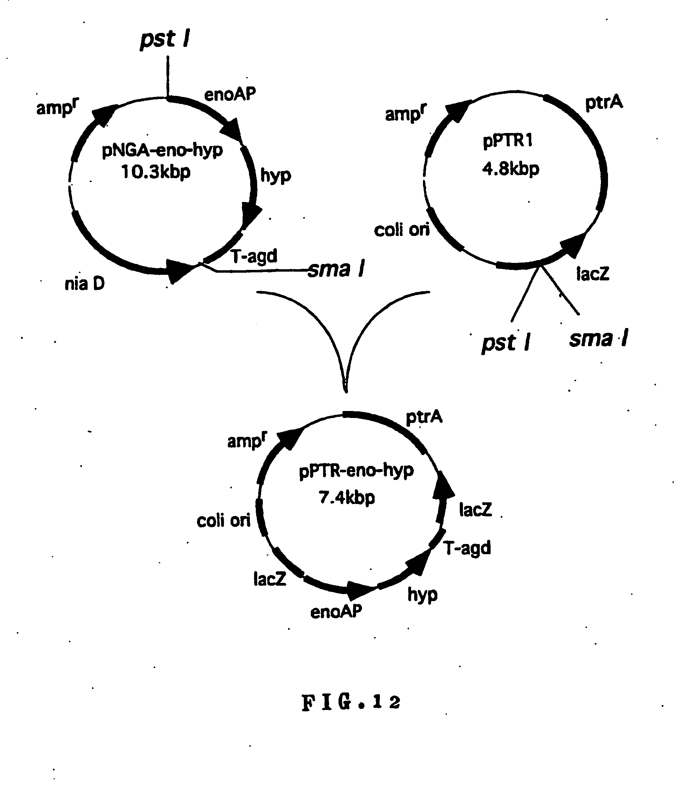

For the preparation of a strain highly triple-co-expressing cutinase, hydrophobin and amylase using the strain highly expressing cutinase obtained in Example 3-4, a plasmid highly expressing hydrophobin, pPTR-eno-hyp, was constructed (Fig.12). The plasmid, pPTR-eno-hyp was constructed by fusing eno A promoter that could be constitutively induced upstream of a target gene. On the other hand, a plasmid highly expressing amylase, pNG-amy, which was constructed by fusing the glaA 142 promoter strongly induced by maltose, was provided from Tohoku University, Graduate School of Agricultural Science, Applied Life Science Technology.

### [Materials]

The promoter pPTR-enoP-hyp was constructed by using the vector pPTR 1. The promoter pNG-enoP-hyp prepared in Example 2 was used to prepare a product for insertion.

### [Method]

### 1. Cut of DNA with a restriction enzyme

pPTR 1 and pNG-enoP-hyp were digested by *Pst*I and *Sma*I, respectively.
2. Preparation of a vector DNA and inserted DNA
3. Ligation
4. Transformation of *E*. *coli*
5. Preparation of a plasmid DNA

The above steps 2.-5. were carried out in the same manners as in Example 2. The resulting plasmid was cut by *Pst*I and *Sma*I and subjected to agarose electrophoresis to confirm the existence of an inserted fragment and the plasmid, and the completion of pPTR-enoP-hyp.

### (9-2) Growth of the strain highly triple-co-expressing cutinase-hydrophobin-amylase

The *Aspergillus oryzae* strain was transformed by the above plasmids, pPTR-enoP-hyp and pNG-amy according to the modified protoplast PEG method. The plasmid pPTR-enoP-hyp (10 µg) was completely digested with *Sac*II*,* extracted with phenol and precipitated with ethanol according to a standard method, and dissolved in 10 µl of TE to serve as a DNA transformation solution. On the other hand, the plasmid pNG-amy (10 µg) was directly dissolved in 10 µl of TE to serve as a DNA transformation solution as well. The following procedures for the transformation of the *Aspergillus oryzae* were done in accordance with those in Example 7-1-2.

The following PCR primers for spore of pPTR-enoP-hyp were synthesized:
5'-ATTCGCGAAAATGGTAGCTCGAGGA-3' , and
5'-CTGTGTCCCGTATGTAACGGTG -3'.

The following PCR primers for the spore of pNG-amy were synthesized:
5'-GGTTCGCTTCGTAAGTCTTCCCTT-3', and
5'-GTAGAATCACGAATGAGACCTTTGACGACC-3'

PCR was carried out using the plasmid DNA for the transformation as a template in a positive control by means of TaKaRa PCR Thermal Cycler PERSONAL (TAKARA SHUZO CO., LTD.) by denaturing the template DNA for 3 min 95°C and repeating 30 cycles of 94°C for 1 min, 55.5°C for 1 min and 72° C for 90 seconds, followed by 72°C for 95 seconds for a complete extension and kept at 4°C for amplification. Agarose gel electrophoresis of the resulting PCR amplified fragments revealed their amplification at the same position as the positive controls, confirming the existence of the hydrophobin gene inserted downstream of the enoA promoter and the amylase gene downstream of the glaA 142 promoter in the genomic DNA of the *Aspergillus oryzae.*

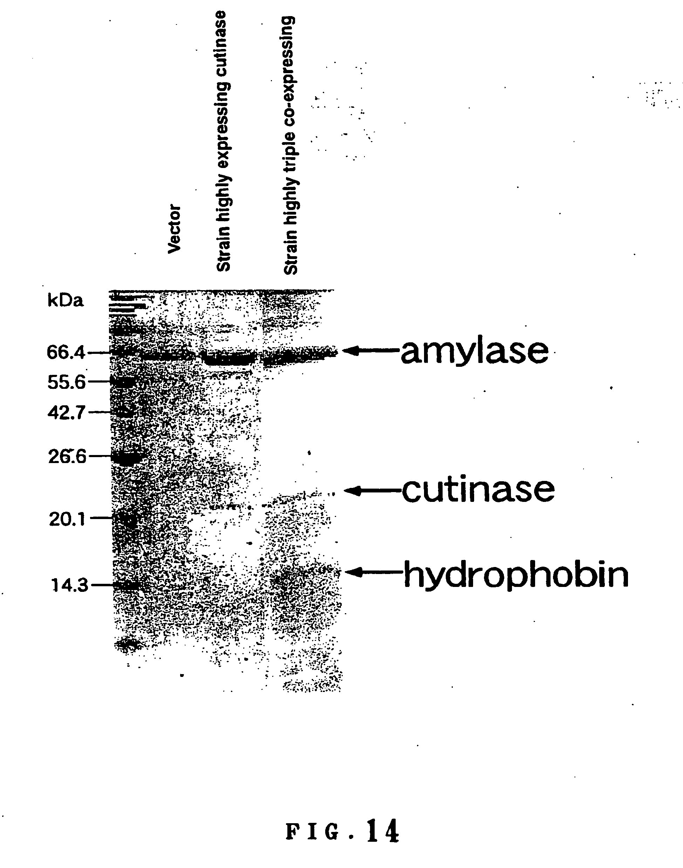

The thus transformed *Aspergillus* strain highly triple-co-expressing cutinase-hydrophobin-amylase was inoculated into 200 ml of YPD culture medium (Example2 (2-2)) at a concentration of 1 x 10⁶ spores/ml, cultured for 24 hours at 30° C with shaking, filtered with a glass filter to give culture supernatant. The supernatant (200 µl ) was mixed well with 100µl of 100 (w/v)% cold trichloroacetic acid, and kept in ice for 12-16 hours. The mixture was centrifuged at 15,000 x g, for 20 min at 4°C. After the supernatant was completely removed, the resulting precipitate was dissolved in 15µl of SDS solution (Example 2 (2-2)) and allowed to stand in a boiling bath for 5 min for SDS treatment to give a sample. The following SDS-PAGE procedures were done in accordance with those in Example 2 (2-2). A band with 14 kDa that was not observed in the strain highly expressing cutinase was confirmed by SDS-PAGE and CBB staining, showing that the *Aspergillus* strain highly triple-co-expressing cutinase-hydrophobin-amylase (*A*. *oryzae* pN-cha) highly expressed hydrophobin (Fig. 14).

### (9-3) Confirmation of α-amylase expression

As an example of the useful substance in conjugation with the degradation of PBS, α-amylase for industry was produced. The strain highly co-expressing hydrophobin and cutinase prepared in Example 7, and the strain highly triple-co-expressing cutinase-hydrophobin-amylase prepared in this example were cultured in the same culture medium as in Table 5. The strain (pNGA142) prepared by transformation of a host strain RIB 40 with the vector having no inserted gene was used as a control. Each of the strains was inoculated at a concentration of 1 x 10⁶ spores/ml, respectively, in the medium and cultured for 24 hours at 30°C. The culture medium was filtered through MIRACLOTH and the resulting filtrate was centrifuged (15,000 x g, 10 min, 4°C) to completely remove the remaining PBS from the medium. The resulting supernatant was diluted 100 times with water to be used in the determination of α-amylase activity by iodine color reaction by the same method as in Example 6.

Solution A: 50 mM Tris buffer (pH 6.8) containing starch (0.2 %) dissolved therein; and Solution B: 1N HCl containing 2 x 10⁻⁴ % iodine and 2x10⁻³ % potassium iodine were prepared as reagent solution for the determination. Solution A (400 µl) was mixed the diluted culture supernatant (50 µl), reacted for 10 min at 37°C, then Solution B (50 µl) was added to the mixture and mixed well, and immediately subjected to the measurement of absorbance at 620 nm. The amount of decrease in the absorbance compared to blank (non-inoculated) was shown as a relative activity of α-amylase in Table 7. The preparation of the strain highly triple co-expressing cutinase-hydrophobin-amylase was confirmed by the fact that it showed the highest α-amylase activity.

**[Table 7]**

| Strain | Relative activity of α-amylase (OD₆₂₀) |
|---|---|
| Blank | 0 |
| Control (pNGA142) | 0.078 |
| Gla-cut-eno-hyp | 0.1 |
| Gla-cut-eno-hyp-amylase | 0.185 |

### Example 10

### (10) Hydrophobin homologue

### (10-1) Search of Hydrophobin homologue by means of Aspergillus oryzae EST data base

Clone information having a high homology (identity) with the amino acids or base sequences of hydrophobins of Filamentous fungi and *Basidiomycetes* were searched in *A. oryzae* EST data base by using BLAST network service.

As a result, the information concerning the following two clones were obtained: *Aspergillus oryzae* hydrophobin, hyp B (Sequence ID No.2) (matching results in the *A. oryzae* data base: e-value of base sequence:0.0, e-value of amino acid sequence:6e-36), and a homologue to *Pholiota nameko* hydrophobin, hydrophobin-315 (Sequence ID No.3) (matching results in the *A*. *oryzae* data base: e-value of base sequence:0.029, e-value of amino acid sequence:1e-19). These two clones had eight cysteine residues preserved in the hydrophobin, showing that these homologues were the hydrophobin of A. oryzae.

### (10-2) Cloning of the hydrophobin homologue gene of A. oryzae and preparation of a strain highly expressing it

### (10-2-1) Cloning of hyp B

Based on the information about an ORF (Open Reading Frame) of hyp B deduced from the BLAST network service, the following two primers for cloning were synthesized so that the above ORF should be contained in an amplified fragment:
5'- CAACCCAACCGTCGACATGAAGTTCT-3', and
5'- GCCAAATGGCGTCTAGATTACAGACC-3'.

PCR was carried out using the genomic DNA of *A. oryzae* RIB 40 prepared in Example 3-3 as a template by means of TaKaRa PCR Thermal Cycler PERSONAL (TAKARA SHUZO CO., LTD.) by denaturing the template DNA for 3 min 95°C and repeating 30 cycles of 95°C for 1 min, 57.2°C 30 seconds and 72°C for 90 seconds, followed by 72°C for 90 seconds for a complete extension and kept at 4°C for amplification. Agarose gel electrophoresis was carried out in order to confirm that the above DNA fragment was actually amplified. And the resulting PCR-amplified fragment was further subjected to base sequencing in order to observe whether any mutation might be introduced. The PCR-amplified fragment was subjected to the agarose gel electrophoresis, and the DNA was excised under a long wave-length (363 nm) UV and collected by prep A Systems (Bio-Rad-Laboratories). The PCR fragment collected by means of PGEM-T Easy Vector Systems (Promega) was subjected to TA cloning in accordance with a protocol attached to the PGEM-T Easy Vector Systems. The base sequence of the DNA inserted in the plasmid DNA (TA-hyp B) was analyzed by means of ABI PRISM^{™} 377 DNA sequencing system (PE Biosystem) according to a protocol of ABI PRISM^{™} 377 DNA sequencer Long Read (PE Biosystem). As a result, there was no mutation in the base sequence of the PCR amplified fragment.

### (10-2-2) Construction of a vector highly expressing hyp B

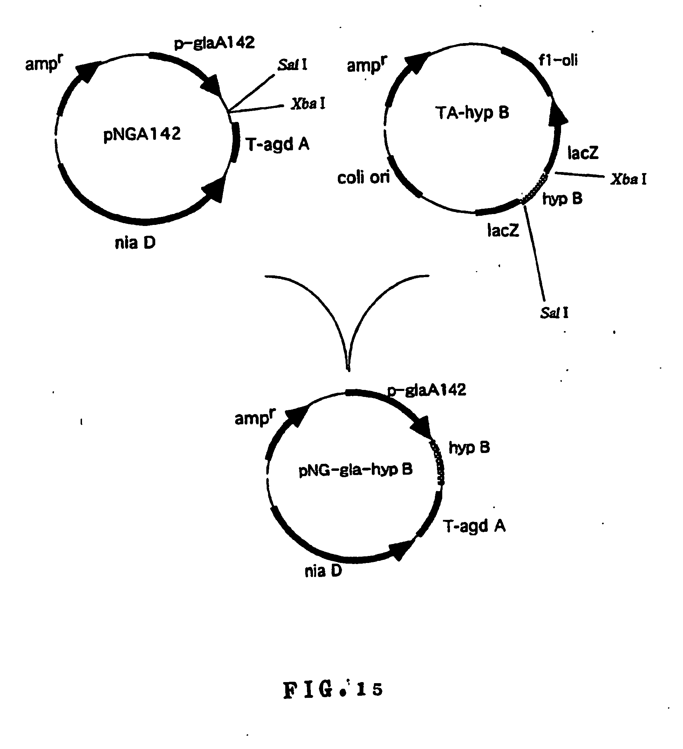

For the preparation of a strain highly expressing hyp B, pNG-gla-hyp B was constructed with the use of an expression vector for *Aspergillus* fungi, pNGA142 vector. A glaA 142 promoter that could be strongly induced by maltose was fused upstream of a target gene(Fig.15).

### [Materials]

The pNG-gla-hyp B was constructed by using the vector, pNGA 142, and TA-hyp B for insertion.

### [Method]

1. Cut of DNA by restriction enzyme pNGA 142 and TA-hyp B were digested by *Xbal* and *Sa*/I, respectively.
2. Preparation of a vector DNA and an inserted DNA
3. Ligation
4. Transformation of *E*. *coli*
5. Preparation of a plasmid

The above steps 2.-5. were carried out in the same manners as in Example 2. The resulting plasmid was cut by *Xba*I and *Sal*I and subjected to agarose electrophoresis to confirm the existence of an inserted fragment and the completion of the plasmid, pNG-gla-hypB.

### (10-2-3) Growth of a strain highly expressing hyp B

The *Aspergillus oryzae* strain was transformed by the above plasmid, pNG-gla-hyp B according to the modified protoplast PEG method. The plasmid (10 µg) was completely digested by *Mun*I*,* extracted with phenol and precipitated with ethanol according to a standard method, and dissolved in 10 µl of TE to serve as a DNA transformation solution. The following procedures for the transformation of the *Aspergillus oryzae* were done in accordance with those in Example 2-2.

The following PCR primers were synthesized for the spore of pNG-gla-hyp B:
5'-CAACCCAACCGTCGACATGAAGTTCT-3', and
5'-GTAGAATCACGAATGAGACCTTTGACGACC -3'.

PCR was carried out using the plasmid DNA for the transformation as a template in a positive control by means of TaKaRa PCR Thermal Cycler PERSONAL (TAKARA SHUZO CO., LTD.) by denaturing the template DNA for 3 min 95°C and repeating 30 cycles of 94°C for 1 min, 57.0°C for 1 min and 72° C for 90 seconds, followed by 72°C for 95 seconds for a complete extension and kept at 4°C for amplification. Agarose gel electrophoresis of the resulting PCR amplified fragment revealed its amplification at the same position as the positive control, confirming the existence of the hypB gene inserted downstream of the gla142 promoter in the genomic DNA of the *Aspergillus oryzae.*

### (10-2-4) Cloning of hydrophobin-315

Based on the information about an ORF (Open Reading Frame) of hydrophobin-315 deduced from the BLAST network service, the following two primers for cloning were synthesized so that the above ORF should be contained in an amplified fragment:
5'-CTGCTTCCTTTGTCGACATGAAGGT-3' and
5'- TCAATGGTCTAGAAGCCCTTGGC-3'

PCR was carried out using the genomic DNA of *A. oryzae* RIB 40 prepared in Example 3-3 as a template by means of TaKaRa PCR Thermal Cycler PERSONAL (TAKARA SHUZO CO., LTD.) by denaturing the template DNA for 3 min 95°C and repeating 30 cycles of 95°C for 1 min, 55.4°C 30 seconds and 72°C for 90 seconds, followed by 72°C for 90 seconds for a complete extension and kept at 4°C for amplification. Agarose gel electrophoresis was carried out in order to confirm that the above DNA fragment was actually amplified. And the resulting PCR-amplified fragment was further subjected to the above base sequencing in accordance with that of Example 10-2-1 in order to observe whether any mutation might be introduced. As a result, there was no mutation in the base sequence of the PCR-amplified fragment.

### (10-2-5) Construction of a vector highly expressing hydrophobin-315

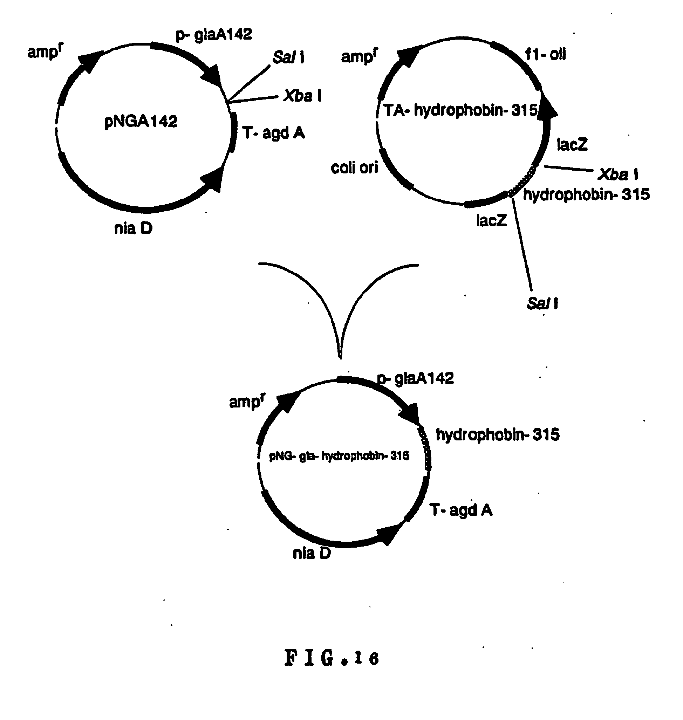

For the preparation of a strain highly expressing hydrophobin-315, pNG-gla-hydrophobin-315 was constructed with the use of an expression vector for *Aspergillus* fungi, pNG142 vector. The glaA 142 promoter that could be strongly induced by maltose was fused upstream of a target gene (Fig.16)..

### [Materials]

The promoter pNG-gla-hydrophobin-315 was constructed by using the vector, pNGA 142, and TA-hydrophobin-315 for insertion.

### [Method]

### 1. Cut of DNA by restriction enzyme

pNGA 142 and TA-hydrophobin-315 were digested by *Xba*I and *Sa*/I, respectively.
2. Preparation of a vector DNA and an inserted DNA
3. Ligation
4. Transformation of *E*. *coli*
5. Preparation of a plasmid

The above steps 2.-5. were carried out in the same manners as in Example 2. The resulting plasmid was cut by *Xba*I and *Sal*I and subjected to agarose electrophoresis to confirm the existence of an inserted fragment and the completion of the plasmid, pNG-gla-hydrophobin-315.

### (10-2-6) Growth of a strain highly expressing hyp B

The *Aspergillus oryzae* strain was transformed by the above plasmid, pNG-gla-hydrophobin-315 according to the modified protoplast PEG method. The plasmid (10 µg) was completely digested with *Mun*I, extracted with phenol and precipitated with ethanol according to a standard method, and dissolved in 10 µl of TE to serve as a DNA transformation solution. The following procedures for the transformation of the *Aspergillus oryzae* were done in accordance with those in Example 2-2.

The following PCR primers were synthesized for the spore of pNG-gla-hydrophobin-315:
5'- CTGCTTCCTTTGTCGACATGAAGGT-3'and
5'-GTAGAATCACGAATGAGACCTTTGACGACC -3'.

PCR was carried out using the plasmid DNA for the transformation as a template in a positive control by means of TaKaRa PCR Thermal Cycler PERSONAL (TAKARA SHUZO CO., LTD.) by denaturing the template DNA for 3 min 95°C and repeating 30 cycles of 94°C for 1 min, 57.0°C for 1 min and 72° C for 90 seconds, followed by 72°C for 95 seconds for a complete extension and kept at 4°C for amplification. Agarose gel electrophoresis of the resulting PCR amplified fragment revealed its amplification at the same position as the positive control, confirming the existence of the hydrophobin-315 gene inserted downstream of the gla142 promoter in the genomic DNA of the *Aspergillus oryzae.*

### (10-2-7) Fungus homologue genes to hydrophobin

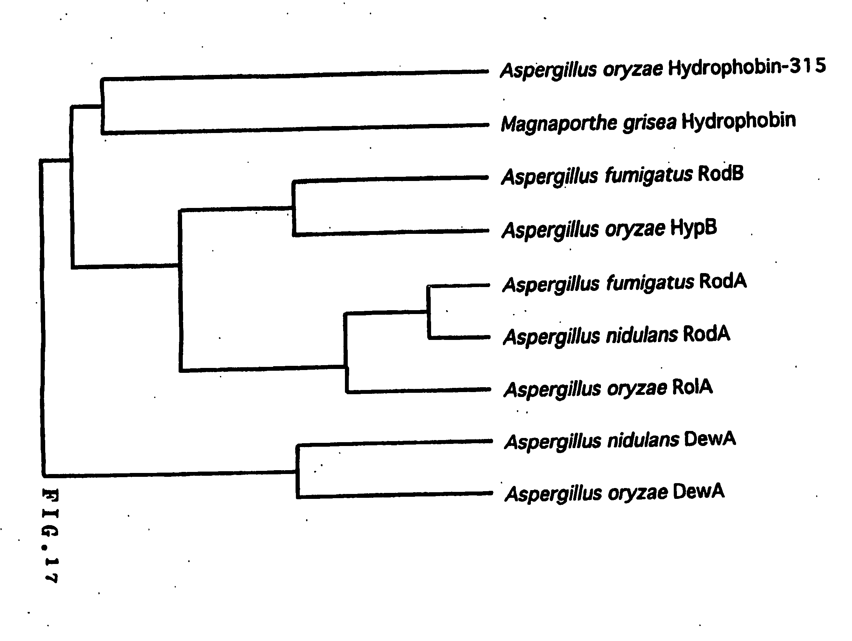

Fungus homologue genes to the *Aspergillus* hydrophobin were searched in known DNA and protein data base with the use of the DNA and amino acid sequences of the above plastic-attaching hydrophobin genes of *Aspergillus* strains. The results are shown as a phylogenic tree in Fig.17, showing that there are homologue genes *in Magnaporthe grisea, Aspergillus fumigatus* and *Aspregillus nidulans.* As these genes had cysteine residues that are characteristic to the hydrophobin gene, it was confirmed that they were hydrophobin as well. And it is expected that their genetic products will attach to a hydrophobic surface of plastic, and function as a factor for promoting the degradation of plastic together with the plastic-degrading enzyme.

The present information about the annotation of the hydrophobin genes in Fig. 17 is as follows:
*Magnaporthe grisea* MPG1
   →an important binding factor for infection to plants; cloned
   ACCESSION NO. L20685(Nucleotide), AAA20128(Protein)
*Aspergillus fumigatus* Rod A
   → an important factor for *Aspergillus* infection; cloned
   ACCESSION NO. AF057335(Nucleotide), AAB60712(Protein)
*Aspergillus fumigatus* Rod B
   →having an activity of inhibiting virus infection to spore; cloned AEM .Mar. 2003. p1581-1588
*Aspergillus nidulans* Rod A
   →expressed in the stage of spore formation and make the surface of fungus hydrophobic; cloned
   ACCESSION NO. M61113(Nucleotide), AAA33321 (Protein)
*Aspergillus nidulans* DewA
   →expressed in the stage of spore formation and make the surface of fungus
   ACCESSION NO. U07935 (Nucleotide), S67924(Protein)
*Aspergillus oryzae* DewA
   →found as homologous to hydrophobin (*Aspergillus nidulans* Dew A) of *A. nidulans*
*Aspergillus oryzae* RolA
   LOCUS AB094496 861 bp DNA linear PLN 07-MAR-2003
   DEFINITION *Aspergillus oryzae* RolA gene for hydrophobin putative, complete cds.
   ACCESSION AB094496
   VERSION AB094496.1 GI:28875528
   SOURCE Aspergillus oryzae
   ORGANISM *Aspergillus oryzae*
   *Eukaryota; Fungi; Ascomycota; Pezizomycotina; Eurotiomycetes;*
   *Eurotiales; Trichocomaceae; mitosporic Trichocomaceae;*
Aspergillus.
   REFERENCE 1
   AUTHORS Takahashi,T., Yoneda,S., Maeda,H., Yamagata,Y., Abe,K., Machida,M., Gomi,K. and Nakajima,T.
   TITLE Hyper induction of a Hydrophobin-like protein, RolA, of *Aspergillus oryzae by* polybutylenesuccinate in liquid culture
   JOURNAL Unpublished
*Aspergillus oryzae* nameko (hydrophobin-315)
   A novel gene (Example 10)
*Aspergillus oryzae* HypB
   This gene and its expressed protein are known, and registered with the following numbers:
   ACCESSION NO. AB097448(Nucleotide), BAC77248(Protein)

This gene was cloned isolated from the genomic DNA of *A. oryzae* based on the known information (Example 10). It was further confirmed that it was specifically expressed in the presence of plastic.

### Example 11

### (11) Attachment of Rol A to the biodegradable plastic

In the process of biodegradation of plastic in the presence of hydrophobin (Rol A), it was thought that Rol A could attach to the surface of the biodegradable plastic due to its amphipathic physical property. It was therefore checked whether Rol A could attach to a biodegradable plastic film (1 mm thickness) made of PBS, PBSA and PLA.

### (11-1) Purification of hydrophobin (Rol A) from the Aspergillus strain highly expressing that

Spore solution of the *Aspergillus* strain highly expressing hydrophobin prepared in Example 2 was inoculated at a final concentration of 1 x 10⁶ spores/ml to a minimum medium containing 1 % of maltose in a shaking flask (3 L) and cultured for 48 hours at 30°. The culture medium was filtered by means of MIRACLOTH (Calbiochem). The resulting filtrate was brought to 40 % saturation with ammonium sulfate and centrifuged at 8,000 g for 20 min at 4°C to give a supernatant fraction. The resulting supernatant fraction was applied to Octyl-Cellulofine column (SEIKAGAKU CORPORATION) equilibrated with 10 mM Tris-NCI buffer, pH 8.0 and 40% saturated ammonium sulfate and an adsorbed fraction was eluted with a linear concentration gradient of 40 - 0 % saturated ammonium sulfate. All of the eluted fractions were subjected to SDS PAGE, and a fraction showing a band at an expected molecular weight was collected. The collected fraction was then dialysed against 10 mM Citrate-NaOH buffer, pH 4.0 and applied to S-Sepharose FF column equilibrated with the same buffer. An adsorbed fraction was eluted with a linear concentration gradient of 0 - 0.5 M NaCl. All of the collected fractions were subjected to SDS-PAGE so that the homogeneity of a desired band was confirmed with CBB staining, which would be then used as purified Rol A.

### (11-2) Preparation of anti-Rol A antibody

By using the purified Rol A protein prepared in Example (11-1), a mouse anti Rol A antibody was prepared (T.K Craft Ltd.).

### (11-3) Confirmation of the attachment of Rol A to the biodegradable plastic

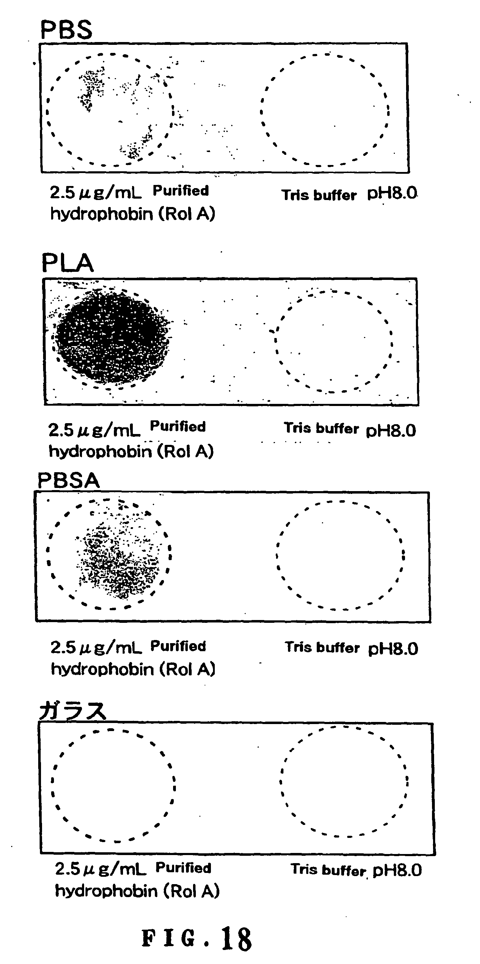

Ten µl of solution of the purified Rol A (2.5 protein/ml Tris-HCl buffer, pH 8.0) was spotted in I cm² area formed by cutting out filter paper (Whatman) on PBS, PBSA (SHOWA HIGHPOLYMER Co., LTD.) and PLA (Mitsui Chemical Co., Ltd.) as well as a slide glass as a control and kept for 13 hours at 30°C with 95 % humidity. A 1,000 times-diluted solution (10 ml) of a first antibody prepared in Example 11-2 (1 % BSA, 0.05 % Tween 20, 10 mM phosphate buffer, pH 7.2, 0.9 (w/v) NaCl) was added to the above film in a container, shaken for one hour, and washed three times with 10 mM phosphate buffer pH 7.2. A 5,000 times-diluted solution (10 ml) of a second anti-mouse antibody (SEIKAGAKU CORPORATION) was added into the container, shaken gently for 30 min and washed three times with10 mM phosphate buffer pH 7.2. A substrate solution was made by mixing 66 µl of NBT (nitro blue tetrazdium 50 mg/ml in 70 % dimethylformamide), 10 ml of alkaline phosphatase buffer (100 mM Tris-HCl, pH 9.5) and 100 mM NaCl, 5 mM MgCl₂. followed by the addition of 66 µl of BIPC (50 mg/ml in 70 % dimethylformamide). The substrate solution was added to the container to detect Rol A attaching to the biodegradable plastic. It was observed that Rol A protein was attaching to all of PBS, PBSA and PLA, but not to the slide glass (Fig. 18).

### Example 12

### (12) Search of novel plastic-degrading enzyme genes

PCR fragments to be used as a probe in Southern hybridization were prepared in the following PCR.

A library of genes expressed during the culture in the presence of PBS (referred to as "cDNA library" thereafter) was obtained in order to prepare a probe sequence. A spore solution was inoculated at a final concentration of 0.5x10⁶ spores/ml to Czapek-Dox medium (100 ml) in a conical flask (500 ml) and cultured for 24 hours at 30 °C. The fungi were collected by means of MIRACLOTH (Calbiochem) and washed with sterilized water, followed by removal of extra water. The collected fungi were transferred to a minimal medium containing PBS emulsion as an only carbon source, cultured with shaking at 30°C for 3 days. After the culture, the fungi were collected by means of MIRACLOTH. The total RNA and mRNA were prepared from the thus collected fungi in accordance with Example 1. A reverse transcription was then done using the resulting mRNA as a template.

Five µl of mRNA (92 ng/µl), 1 µl of oligo(dT) primer (0.5 µg/µl), 4 µl of 2.5 mM dNTP MIX (2.5 mM each) and 2 µl of DEPC-treated water were added to an Eppendorf tube. The mixture was incubated for 10 min at 70°C, allowed to stand for more than 1 min on ice so as to anneal mRNA with the oligo(dT) primers. To this mixture, 4 µl of 5 x First Strand Buffer (250 mM Tris-HCl, pH 8.3, 375 mM KCI, 15 mM MgCl₂) and 2 µl of 0.1 M DTT were added, incubated fro 5 min at 42°C. To the resulting mixture was added 1 µl of Super Script II RT (200 U/µl), mixed with a pipette and allowed to stand for 50 min at 42°C, followed by another addition of 1 µl of Super Script II RT (200 U/µl), mixing with a pipette and incubation for 50 min at 42°C for the reverse transcription. The reaction solution was incubated for 15 min at 70°C to inactivate the reverse transcriptase, followed by addition of RNase H (10 U/µl) and incubation for 20 min at 37°C to decompose unreacted mRNA. The thus prepared solution was used as cDNA library solution.

The following two mix oligonucleotides having 18 bases and 15 bases, respectively, were synthesized based on the comparison of homology between the PBS-degrading enzyme (cutinase) identified in Example 3 and known analogous enzymes derived from *Aspergillus fumigatus:*
{5'-GT(T/C/A/G)GC(T/C/A/G)TG(T/C)CA(A/G)GG(T/C/A/G)GT(T/C/A/G)-3'} and,
{5'-(G/A)TA(C/T/G/A)CC(C/T/G/A)CC(C/T/G/A)GC(C/T/G/A)AC(T/G/A)AT-3'}.

PCR fragments to be used as a probe in Southern hybridization were amplified in the PCR using the above primer pair.

PCR was carried out using the cDNA library as a template by means of TaKaRa PCR Thermal Cycler PERSONAL (TAKARA SHUZO CO., LTD.) by denaturing the template DNA for 3 min 95°C and repeating 30 cycles of 95°C for 1 min, 53° C for 1 min and 72°C for 30 seconds, followed by 72°C for 1 min for a complete extension and kept at 4°C. Agarose gel electrophoresis of the resulting PCR amplified fragment confirmed the amplification of a fragment having about 160 base pairs.

Southern hybridization was carried out in the same manner as in Example 3 (3-3) using NEN RandomPrimer Fluorescein Labeling Kit with Antifluorescein-AP (Eizo Diagnostics, Inc.). Fragments of the genomic DNA (10 µg) completely digested by 50 units of *Hinc*III (TAKARA SHUZO CO., LTD.), or Sacl and *Hin*dIII (TAKARA SHUZO CO., LTD.) were used as a target. The complete digestion by *Hinc* III showed a strongly-hybridizing band with about 2,700 base pairs, and the complete digestion by *Sac*I and *Hin*dIII showed a strongly-hybridizing band with about 1,800 base pairs.

Then the colony hybridization was carried out in accordance with Example 3 (3-3). The genomic DNA (10 µg) was completely digested by 50 units of Hinclll (TAKARA SHUZO CO., LTD.), or Sacl and *Hin*dIII (TAKARA SHUZO CO., LTD.), and subjected to agarose gel electrophoresis. After staining with ethidium bromide, parts of gel around 2,700 base pairs and 1,800 base pairs were excised under UV irradiation. DNA was extracted from the gel using prep A gene (BioRad) to be used as an insertion DNA fragment.

A plasmid, pBluescript II KS+DNA (Stratagene), 2.5 µg, was digested completely by *Hinc*III (TAKARA SHUZO CO., LTD.), or Sacl and Hindlll (TAKARA SHUZO CO., LTD.), and subjected to conventional phenol extraction and ethanol precipitation, followed by removal of phosphoric acid at the 5' end by alkaline phosphatase (TAKARA SHUZO CO., LTD.). The resulting solution was subjected to conventional phenol extraction and ethanol precipitation and dissolved in TE to be used as a vector DNA solution. The insertion DNA fragment was ligated with the vector DNA to give a ligated DNA solution. Competent *E*. *coli* DH5α strain (TAKARA SHUZO CO., LTD.) was transformed with the ligated DNA solution and subjected to the colony hybridization to give a positive signal.

*E. coli* strain having a plasmid showing the positive signal was collected from the agar medium, and a plasmid DNA solution was prepared by the same way as in Example 3 (3-3) from the above strain. As a result, an open reading frame (SEQ ID No.4) comprising the base sequence of the probe was found in the *Hinc*III-digested fragment (2713 base pairs) of the genomic DNA, and an open reading frame (SEQ ID No.5) comprising the base sequence of the probe was found in the *Sac*I/*Hin*dIII-digested fragment (1801 base pairs) of the genomic DNA. It was also revealed that these two open reading frames comprised two and three intron base sequences, respectively, and that they also had "ATG" encoding an initiation methionine and stop codon. Accordingly, each of these open reading frames contained a full length of the gene of PBS-degrading enzyme analogues.

### (12-2)

PCR was done using the cDNA library comprising the genes of the PBS-degrading enzyme of Example 3, and the above two PBS-degrading enzyme analogues as a template in order to prepare PCR fragments to be inserted into a plasmid for the expression in *E. coli* (pET-12b: Novagen). The following oligomer primer sets were used:
5'-TGCAGTGGCGGATCCGGTGGAC-3', and
5'-GACCGGATGGATCCCGAAAATTTATCC-3' for the PBS-degrading enzyme;
5`-GGCAGCAGGGGATCCCATCGCTG-3', and
-CGTAGCCCACACTCGGATCCTAAGCTGAC-3 ' for the gene of PBS-degrading enzyme analogue having the open reading frame of 2,713 base pairs; and
5'-GGCGGCTGCGGATCCAGTAGATATC-3', and
5'-CAGTTCAGGGGGATCCTATAGAGTCC-3' for the gene of PBS-degrading enzyme analogue having the open reading frame of 1,801 base pairs.

PCR was carried out using 1 µl of the cDNA library as a template by means of TaKaRa PCR Thermal Cycler PERSONAL (TAKARA SHUZO CO., LTD.) by denaturing the template DNA for 3 min 95°C and repeating 30 cycles of 95°C for 1 min, 57°C for 1 min and 72°C for 1 min, followed by 72°C for 1 min for a complete extension and kept at 4°C. The resulting PCR-amplified fragments were completely digested by *Bam*HI (TAKARA SHUZO CO., LTD, and subjected to agarose gel electrophoresis. After staining with ethidium bromide, fragments of 625 base pairs, 650 base pairs and 759 base pairs were excised under UV irradiation. DNA was extracted from the gel using prep A gene (BioRad) to be used as an insertion DNA fragment.

A plasmid, pET-12b (Novagen) containing T7 promoter sequence and a signal sequence (OmpT-leader sequence) 5 µg, was digested sequence by *Bam*H (TAKARA SHUZO CO., LTD.) at the *Bam*HI site immediately down stream of the signal, and subjected to conventional phenol extraction and ethanol precipitation, followed by removal of phosphoric acid at the 5' end by alkaline phosphatase (TAKARA SHUZO CO., LTD.). The resulting solution was subjected to conventional phenol extraction and ethanol precipitation and dissolved in TE to be used as a vector DNA solution. The insertion DNA fragment (1.5 µg) was ligated with the vector DNA (1 µg) to give a ligated DNA solution. Competent *E. coli* DH5α strain (TAKARA SHUZO CO., LTD.) was transformed with the ligated DNA solution to give *E. coli* transformant harboring the plasmid comprising each of the above genes. Plasmid DNA was extracted from the culture of each *E*. *coli* transformant as mentioned above to give plasmids (pET-cut, pET-cuthom1, pET-cuthom2) for *E*. *coli* transformation. *E*. *coli* BL21-SI strain (Invitrogen) was transformed by these plasmids and cultured on LB agar culture medium comprising 50 µg/ml of ampicillin so as to obtain each transformant.

Colonies of the transformants harboring the plasmids of pET-cut, pET-cuthom1, pET-cuthom2, and pET-12b were scraped away from the agar medium by means of a platinum needle, transferred in to LB liquid culture medium (50 µg of ampicillin) without NaCl and pre-cultured with shaking for 16 hours at 37°C. The pre-culture medium (500 µl) was transferred into 50 ml of LB liquid culture medium containing NaCl in a shaking flask with a volume of 500 ml. An enzyme in interest was induced and expressed by the culture with shaking for 12 hours at 30°C. The resulting culture medium (50 ml) was transferred into a micro tube (50 ml) and centrifuged at 8,000 x g, 10 min to obtain a culture supernatant. To the resulting supernatant was added PBS emulsion at a final concentration of 0.1 (v/v) %. The expression of the PBS-degrading enzyme was measured by decrease in turbidity (decomposition of PBS). The activity of the PBS-degrading enzyme was observed only in the culture supernatant of the *E. coli* transformants of the PBS-degrading enzyme and its analogues.

**[Table 8]**

| Plasmid Name | pET-12b Blank | pET-cut Cut (L1 ) | pET-cuthom1 Homologue 1 | pET-cuthom2 Homologue 2 |
|---|---|---|---|---|
| Substrate | | | | |
| PBSA | ND | 83 | 57 | 19 |
| PLA | ND | 3 | 6 | 4 |

| | | | | |
|---|---|---|---|---|
| ND; not detected | | | | |

To the resulting supernatant was then added PLA emulsion at a final concentration of 0.1 (w/v) % and incubated at 37°C. The expression of the PBS-degrading enzyme was measured by decrease in turbidity (decomposition of PLA). The activity of the PLA-degrading enzyme was observed only in the culture supernatant of the *E. coli* transformants of the PBS-degrading enzyme and its analogues. Thus, it was revealed that the PBS-degrading enzyme prepared in Example 3, and the two PBS-degrading enzyme analogues had both PBS- and PLA - degrading activities.

### (12-3) Biodegradable plastic-degrading enzymes derived from Magnaporthe grisea

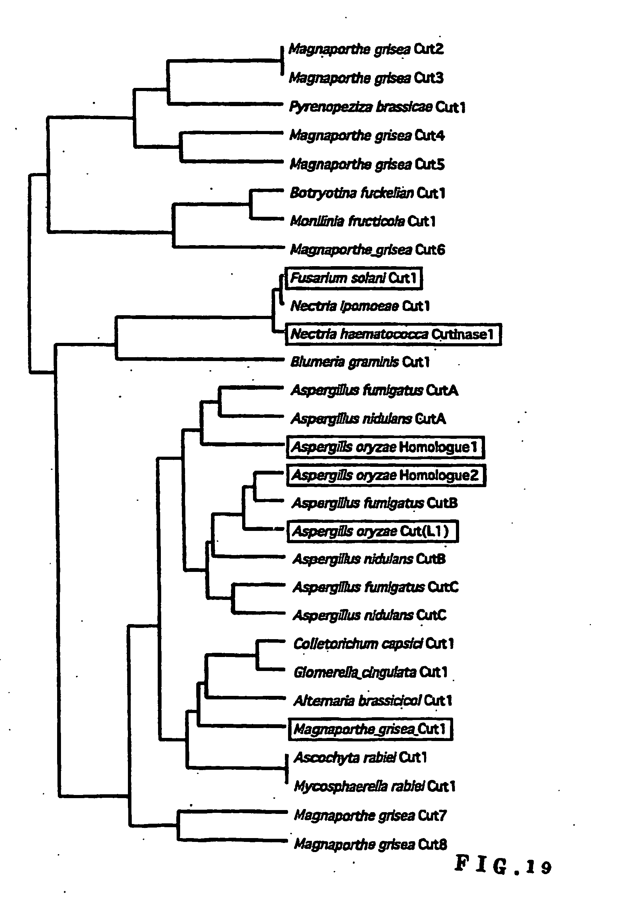

Homologue genes to *Aspergillus* PBS-degrading enzyme were searched in known DNA and protein data base with the use of the DNA and amino acid sequences of the above PBS-degrading enzyme genes of *Aspergillus* strains. The results are shown as a phylogenic tree in Fig.19. Many of the detected homologue genes are those of filamentous fungi. Especially, 8 homologous genes of *Magnaporthe grisea* were identified. *Magnaporthe grisea* 70-15 strain was then cultured in PBS emulsion and PLA emulsion, and a degrading activity was measured in each culture supernatant (Table 9).

Thus, The above strain was cultured with shaking for 4 days at 24°C in Vogel-N medium (2% sucrose). The fungi were collected by filtering and transferred to Vogel-N medium containing only PBSA or PLA emulsion (1 w/v %) as carbon source, and cultured with shaking for 2 days at 24 ° C. Each culture supernatant was obtained by filtration and centrifugation. To the resulting culture supernatant was added PBSA or PLA emulsion at a final concentration of 0.1 (w/v) % and incubated for 24 hours at 37°C to measure degradation ratio at O.D. 630. As a result, it was confirmed that the *Magnaporthe grisea* strain had PLA- and PBS-degrading activities.

**[Table 9]**

| Carbon source | Substance | Degradation ratio (%) |
|---|---|---|
| PBSA | PBSA | ND |
| PLA | PBSA | 12 |
| PBSA | PLA | 63 |
| PLA | PLA | 30 |

| | | |
|---|---|---|
| ND; not detected | | |

The information available at present about the annotation of the plastic-degrading enzyme genes in Fig. 19 is as follows:

### Aspergillus oryzae

- Cut (L1): BAA07428.1 (protein), Gl (gene): 949813
- Homologue 1, 2: →not registered as a gene or a protein

### Aspergillus fumigatus

Homologues that were found by tblastn *search* in *Aspergillus fumigatus* genome data base. As they are not annotated genes, they are not registered as a gene or a protein, either.
- CutA: a gene expression product found in contigue 4882
- CutB: a gene expression product found in contigue 4865.
- CutC: a gene expression product found in contigue 4812.

### Aspergillus nidulans

Homologues that were found by tblastn *search in Aspergillus nidulans* genome data base. Although they are registered as genomic DNA in each contigue, the following three genes are not reistered as an annotated gene or cutinase.
- CutA: AACD01000122.1 (protein), Gl (gene): 29570975
- CutB: AACD01000129.1 (protein), Gl (gene): 29570982
- CutC: AACD01000093.1 (protein), Gl (gene): 29570946

### Magnaporthe grisea

Cut1-8 was already annotated in all of the genomic data bases.

Cut1 was confirmed as cutinase in view of protein.

Cut2-8 is treated as hypothetical protein in any data base.
- Cut1: P29292 (protein), Gl (gene): 1345869, Locus: MG 1943.3
- Cut2: Locus: MG 02301.3
- Cut3: Locus: MG 11108.3
- Cut4: Locus: MG 03792.3
- Cut5: Locus: MG 05798.3
- Cut6: Locus: MG 02393.3
- Cut7: Locus: MG 00734.3
- Cut8: Locus: MG 09100.3

The following 4 cutinases are known genes and proteins, and their functions were already confirmed as well.
- *Monilinia fructicola* Cut1: Q8TGB8 (protein), Gl (gene): 29839372
- *Fusarium solani* Cut1: K02640.1 (protein), Gl (gene): 168145
- *Nectria ipomoeae* Cut1: Q99174 (protein), Gl (gene): 2493916
- *Nectria haematococca* Cutinase: Q96UT0 (protein), Gl (gene): 20137890

The functions of the following known genes and proteins are not yet confirmed.
- *Pyrenopeziza brassicae* Cut1: Q9Y7G8 (protein), Gl (gene): 29839423
- *Botryotina fuckelian* Cut1: W00298 (protein), Gl (gene): 2493915
- *Blumeria graminis* Cut1: Q8X1P1 (protein), Gl (gene): 29839380
- *Colletorichum capsici* Cut1: P10951 (protein), Gl (gene): 117650
- *Glomerella cingulata* Cut1: P11373 (protein), Gl (gene): 117651
- *Alternaria brassicicol* Cut1: P41744 (protein), Gl (gene): 1169141
- *Ascochyta rabiei* Cut1: P29292 (protein), Gl (gene): 117649
- *Mycosphaerella rabiei* Cut1: P29292 (protein), Gl (gene): 117649

### Example 13

### (13) Isolation of a biodegradable plastic-binding protein from Aspergillus oryzae, and growth of a strain highly expressing the same enzyme

### (13-1) Isolation of the gene of a biodegradable plastic-binding protein (PbpA, PbpB)

The genes of biodegradable plastic-binding protein (PbpA, PbpB) were cloned from the genomic DNA of *Aspergillus oryzae,* and a strain highly expressing PbpA was grown as described below.

### [Method]

RIB40 spore solution was inoculated at a final concentration of 1.0 x 10⁶ spores/ml to Czapek-Dox medium in a conical flask (3L) containing PBS emulsion (1 (w/v)%) as an only carbon source, and cultured with shaking for 5 days. The culture medium was filtered by means of MIRACLOTH (Calbiochem) and the resulting filtrate was brought to saturation with 20 % ammonium sulfate, cooled on ice for one hour, and centrifuged at 10,000 g for 30 min at 4°C so as to discard the pellet. The resulting supernatant fraction was applied to Octyl-Cellulofine column (SEIKAGAKU CORPORATION) equilibrated with 10 mM Tris-NCl buffer, pH6.8 and 20% saturated ammonium sulfate and an adsorbed fraction was eluted with a linear concentration gradient of 20 - 0 % ammonium sulfate saturation. The eluted fractions were subjected to SDS-PAGE to confirm eluted proteins. The gel after SDS-PAGE was treated with Silveststain (Nacalai Tesque, Inc.) for PAGE protein in accordance with its manual.

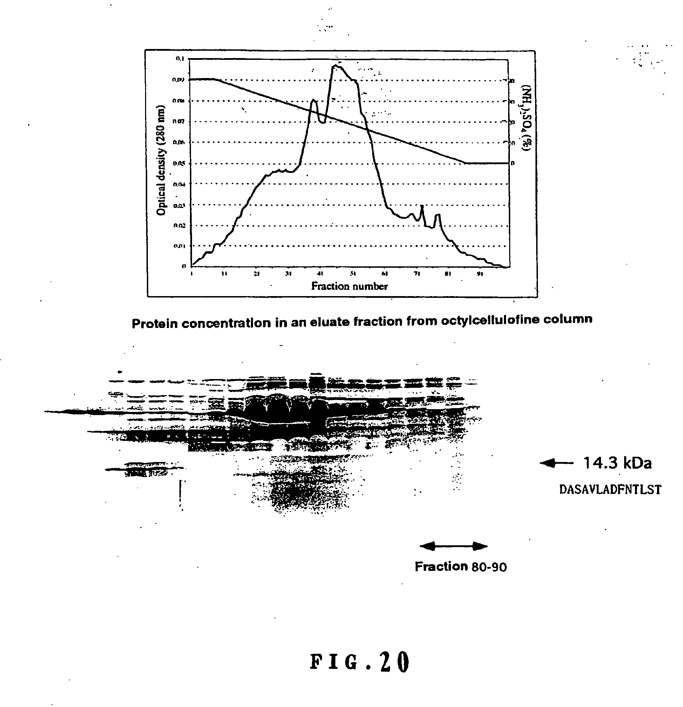

The existence of a protein with about 14 kDa was confirmed at around 0 % ammonium sulfate (Fig.20). Since this protein was eluted from the above hydrophobic column at confirmed at around 0 % ammonium sulfate saturation, it seemed to be a highly hydrophobic protein. Amino acid sequence at N-end was determined to be "DASAVLADFNTLST."

The resulting amino acid sequence was subjected to homology search in *Aspergillus oryzae* EST blast (http://www.nrib.go.jp/ken/EST/db/blast.html) so as to reveal its homology to a 138-amino acid sequence. This amino acid sequence was subjected to homology search in Japan DNA data bank (http://www.ddbj.nig.ac.jp/Welcome-j.html) to show its homology to 4MeS protein produced by *Metarhizium anisopliae.* Further, this amino acid sequence was subjected to homology search in the genomic data base of *Aspergillus fumigatus* (http://www.tigr.org/tdb/e2k1/afu1/) to show its homology to sequences contained in contig 4875 and contig 4820. Specific values concerning the above homology are shown in Table 10.

**[Table 10]**

| | PbpA | 4875 | 4MeS | 4820 | PbpB |
|---|---|---|---|---|---|
| PbpA | 100 | 44.5 | 31.4 | 29.7 | 27.4 |
| 4875 | | 100 | 32.7 | 25.0 | 29.4 |
| 4MeS | | | 100 | 26.6 | 26.5 |
| 4820 | | | | 100 | 60.2 |
| PbpB | | | | | 100 |

Cloning was then tried from the genomic DNA of *Aspergillus oryzae* based on the above sequences.

The following two sets of oligonucleotides were synthesized with reference to the homologous sequence of *Aspergillus fumigatus* and a codon usage of *Aspergillus oryzae* (http://www.kazusa.or.jp/codon/):
5'-ATGCTCGCCAAGCACGTC-3'and 5'-GGCCTTCTTGTACTCGGC-3. and
5'-GACGCAATCTCCACCAC-3' and 5'-TCAAACGCATCCGCAATCTG-3'.

PCR was carried out using the genomic DNA (100 ng) as a template, Ex taq polymerase (TAKARA SHUZO CO., LTD.) and the resulting two pairs as a primer set with TaKaRa PCR Thermal Cycler PERSONAL (TAKARA SHUZO CO., LTD.) to amplify a probe for Southern hybridization. The amplification was done by denaturing the template DNA for 3 min at 95°C, and repeating 30 cycles of 95°C for 1 min, 50°C for 1 min, 72°C for 30 seconds, and 72°C for 1 min for a complete extension, followed by being kept at 4°C. Agarose gel electrophoresis of the resulting PCR amplified fragments revealed amplification of PCR fragments having about 570 base pairs and about 300 base pairs, respectively.

Southern hybridization was carried out using NEN RandomPrimer Fluorescein Labeling Kit with Antifluorescein-AP (Eizo Diagnostics, Inc.) in a similar way to that of Example 3 (3-3). The genomic DNA (10µg) completely digested by 50 units of EcolR and *Bam*HI (TAKARA SHUZO CO., LTD.) was used as a target. As a rersult, a band having about 3,000 base pairs was detected when a probe of about 300 base pairs was used for the EcoIR-digested fragments. And a band having about 2,000 base pairs was detected when a probe of about 570 base pairs was used for the *Bam*HI-digested fragments.

The genomic DNA (10 µg) was completely digested by 50 units of *Eco*IR and *Bam*HI, respectively and subjected to agarose gel electrophoresis. After staining with ethidium bromide, a part of gel around 3,000 base pairs and 2,000 base pairs were excised under UV irradiation. DNA was extracted from the gel using prep A gene (BioRad) to be used as an insertion DNA fragment.

A plasmid, pBluescript II KS+DNA (Stratagene), 2.5 µg, was digested completely by EcoIR and *Bam*HI (TAKARA SHUZO CO., LTD.), respectively, and subjected to conventional phenol extraction and ethanol precipitation, followed by removal of phosphoric acid at the 5' end by alkaline phosphatase (TAKARA SHUZO CO., LTD.). The resulting solution was subjected to conventional phenol extraction and ethanol precipitation and dissolved in TE to be used as a vector DNA solution. The vector DNA was ligated with the above insertion DNA fragment to give a ligated DNA solution. *E. coli* DH5α (TAKARA SHUZO CO., LTD.) was transformed by the ligated DNA solution and subjected to colony hybridization to give a positive signal.

A plasmid solution was prepared from *E. coli* having a plasmid showing the positive signal in accordance with the method of Example 3 (3-3). The base sequence of the insertion DNA fragment comprised in the plasmid DNA was analyzed. As a result, open reading frames comprising the base sequence of the probes of about 570 base pairs and about 300 base pairs, respectively, were found in the *Eco*IR and *Bam*HI-digested fragments (3008 base pairs and 2004 base pairs, respectively) of the genomic DNA. The former open reading frame consisted of 584 base pairs encoding 174 amino acids (PbpA- SEQ ID No.6) and the latter open reading frame consisted of 561 base pairs encoding 186 amino acids (PbpB- SEQ ID No.7). The former one contained one intron base sequence, and the latter contained no intron base sequence.

It was shown that the former deduced amino acid sequence (PbpA) had 100 % homology (identity) to the 138 amino acid-sequence obtained from EST data base, indicating that the former amino acid sequence was that of the desired protein.

### (13-2) Growth of a Aspergillus strain highly expressing the PBS-binding protein

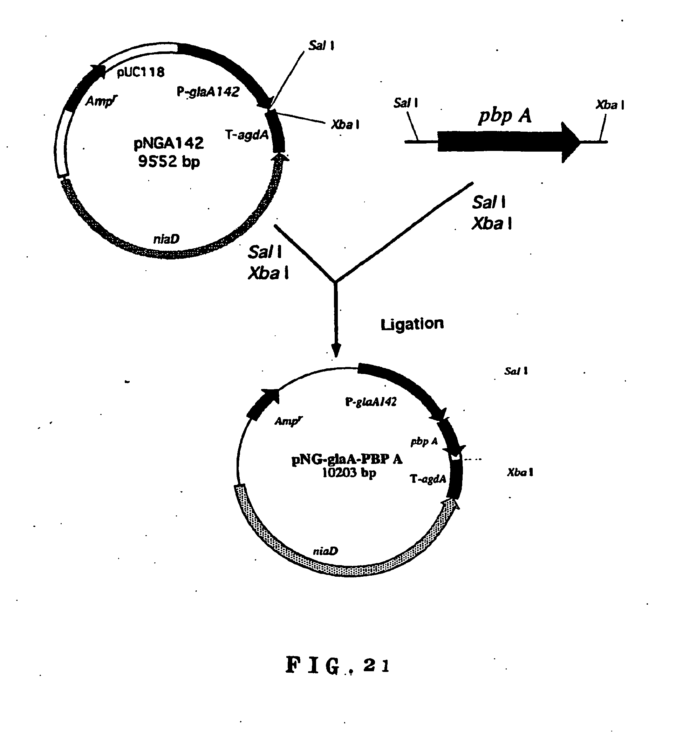

### Construction of a high expression system of the PBS-binding protein (Fig.21)

PCR was carried out using the genomic DNA of *Aspergillus aryzae* RIB40 strain as a template and the following pair of oligonucleotides designed based on the base sequences around the desired gene cloned above:
5'-CTTGCATTCAAGTCGACCTGAACAC-3'and,
5'-CTATTGAACTATGCTTCTAGAAGGCCTAATC-3'.

The amplification was done by denaturing the template DNA for 3 min at 95°C, and repeating 30 cycles of 95°C for 1 min, 60°C for 1 min, 72°C for 30 seconds, and 72°C for 1 min for a complete extension, followed by being kept at 4°C. Agarose gel electrophoresis of the resulting PCR amplified fragments revealed the amplification of a PCR fragment having about 730 base pairs. The PCR fragment was digested by *Sal*I and *Xba*I (TAKARA SHUZO CO., LTD.) and extracted using prep A gene (BioRad) to be used as an insertion DNA fragment. A plasmid, pNGA 142 DNA comprising an glucoamylase promoter (PglaA 142) sequence (TAKARA SHUZO CO., LTD.), 5 µg, was digested by *Sal* I and Xba *I,* and subjected to conventional phenol extraction and ethanol precipitation, followed by removal of phosphoric acid at the 5' end by alkaline phosphatase (TAKARA SHUZO CO., LTD.). The resulting solution was subjected to conventional phenol extraction and ethanol precipitation and dissolved in TE to be used as a vector DNA solution.

The insertion DNA fragment (1.5 µg) was ligated with the vector DNA (1 µg) with T4 DNA ligase (TAKARA SHUZO CO., LTD.) to give a ligated DNA solution. Competent *E. coli* DH5α (TAKARA SHUZO CO., LTD.) was transformed using the ligated DNA solution to give *E. coli* transformant harboring the plasmid comprising the above gene. Plasmid DNA was extracted from the culture of *E*. *coli* transformant in accordance with the method described in Example 3 (3-3) to give a plasmid DNA for transformation of *Aspergillus* strain.

The plasmid (10 µg) was completely digested with *Bam*HI, extracted with phenol and precipitated with ethanol according to a standard method, and dissolved in 10 µl of TE to serve as a DNA transformation solution for *Aspergillus* fungus. The following procedures for the transformation of the *Aspergillus oryzae* were done in accordance with those in Example 7 (7-1-2) to obtain a transformant.

The following PCR primers for spore were synthesized:
5'- CTTGCATTCAAGTCGACCTGAACAC -3' ,and
5'-GTAGAATCACGAATGGAGCCTTTGACGACC-3'.

PCR was carried out using the plasmid DNA for the transformation as a template in a positive control by means of TaKaRa PCR Thermal Cycler PERSONAL (TAKARA SHUZO CO., LTD.) by denaturing the template DNA for 3 min 94°C and repeating 30 cycles of 94°C for 1 min, 55.5°C for 1 min and 72° C for 90 seconds, followed by 72°C for 95 seconds for a complete extension and kept at 4°C. Agarose gel electrophoresis of the resulting PCR amplified fragment revealed its amplification at the same position as the positive control, confirming the existence of the desired gene inserted downstream of the gla142 promoter in the genomic DNA, and the preparation of a *Aspergillus oryzae* transformant containing the same gene.

Spore suspension of the thus transformed strain of *Aspergillus oryzae* having the above desired gene inserted in the genomic DNA, and that of the transformed strain of *Aspergillus oryzae* having no inserted gene (i.e., transformed with pNGA142) was inoculated into 100 ml of YPM culture medium containing maltose as an inducing substance for the glucoamylase promoter at a concentration of 0.5 x 10⁶ spores/ml, respectively, cultured for 16 hours at 30° C and 125 rpm, and filtered with MIRACLOTH (CALBIOCHEM^{™}) to give a culture supernatant.

The resulting supernatant (100 µl) was mixed well with 50µl of cold trichloroacetic acid, and kept in ice for 20 min. The mixture was centrifuged at 15,000 x g, for 15 min at 4°C. After the supernatant was completely removed, the resulting precipitate was dissolved in 15µl of SDS solution (Example 2 (2-2)) and allowed to stand in a boiling bath for 5 min for SDS treatment to give a sample. SDS-PAGE was carried out in accordance with the method described in Example 2 (2-2).

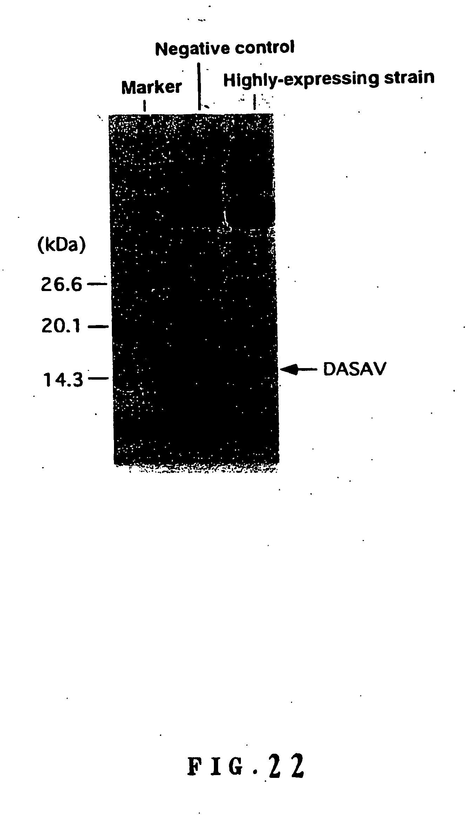

SDS-PAGE of the resulting sample revealed a band at the position of the desired protein (ca. 14 kDa) only with respect to the supernatant of the transformed strain of *Aspergillus oryzae* having the desired gene inserted in the genomic DNA (Fig. 22). The protein was transferred from the electrophoresis gel to a PVDF membrane, from which a band of ca. 14 kDa was excised and subjected to terminal amino acid sequence analysis. The analysis showed the N-end amino acid sequence of "DASAV", confirming the protein obtained at the position of ca. 14 kDa was the desired one.

### (13-3) Growth of a strain highly expressing PbpA on a hydrophobic surface

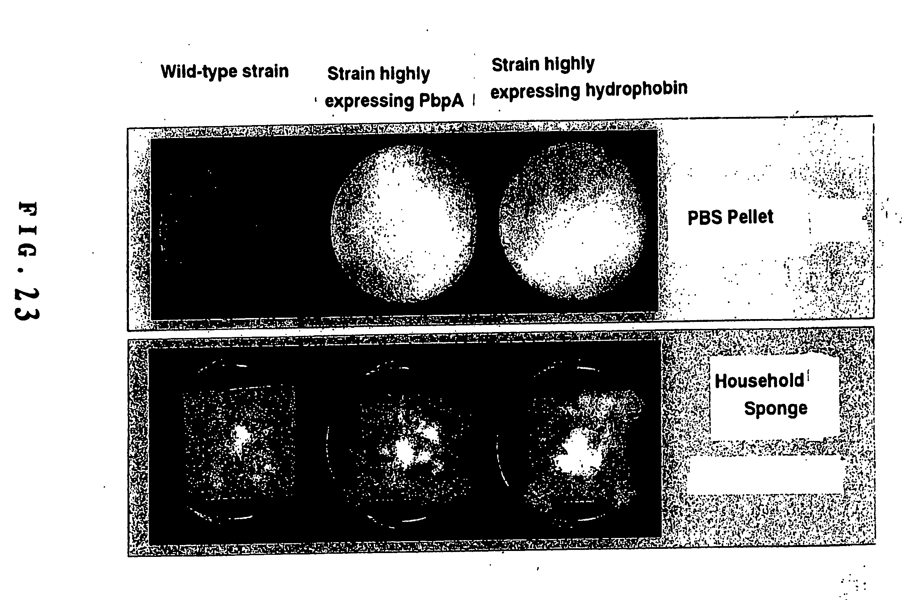

PBS particles (1 mm³) 20 g and YPM culture medium (15 ml) were put into a plastic petri dish (φ 90 mm) and mixed well to give a homogeneous YPM liquid medium. Spores (10⁸) of the strain highly expressing the protein of ca. 14 kDa, the strain highly expressing hydrophobin and the wild strain were inoculated in the medium and mixed well to homogenize the spore in the medium. Household urethane sponge cut into a piece of 6 cm x 6 cm in length and 1 cm in height was soaked sufficiently with the resulting YPM liquid medium. The spores (10⁸) of each of the above three strains were inoculated on the center of the sponge. After the culture for 7 days at 30°C, the strain highly expressing hydrophobin and PbpA grew much more actively on the hydrophobic surface than the wild strain (Fig.23). It was thought that this highly expressed protein was a cofactor necessary for mold to develop on a hydrophobic surface such as PBS, helping them attach to the hydrophobic surface. The protein of14 kDa was therefore named "PBS-binding protein (PbpA)." The other homologous sequence obtained in the cloning of the PbpA was similarly named PbpB.

### (13-4) Attachment of PbpA to PBS in vitro

Spores of the *Aspergillus* strain highly expressing PbpA were inoculated at a final concentration of 1 x 10⁶ spores/ml to YPM liquid medium and cultured for 2 days. The culture medium was filtered by means of MIRACLOTH (Calbiochem). The resulting filtrate was brought to 60 % saturation with ammonium sulfate, cooled on ice for 1one hour and centrifuged at 10,000 g at 4 °C to give a supernatant fraction. The resulting supernatant fraction purified to a single band in SDS-PAGE by means of Octyl-Cellulofine column (SEIKAGAKU CORPORATION), DEAE-Cellulofine column (SEIKAGAKU CORPORATION), S-Sepharose column (FF Pharmacia).

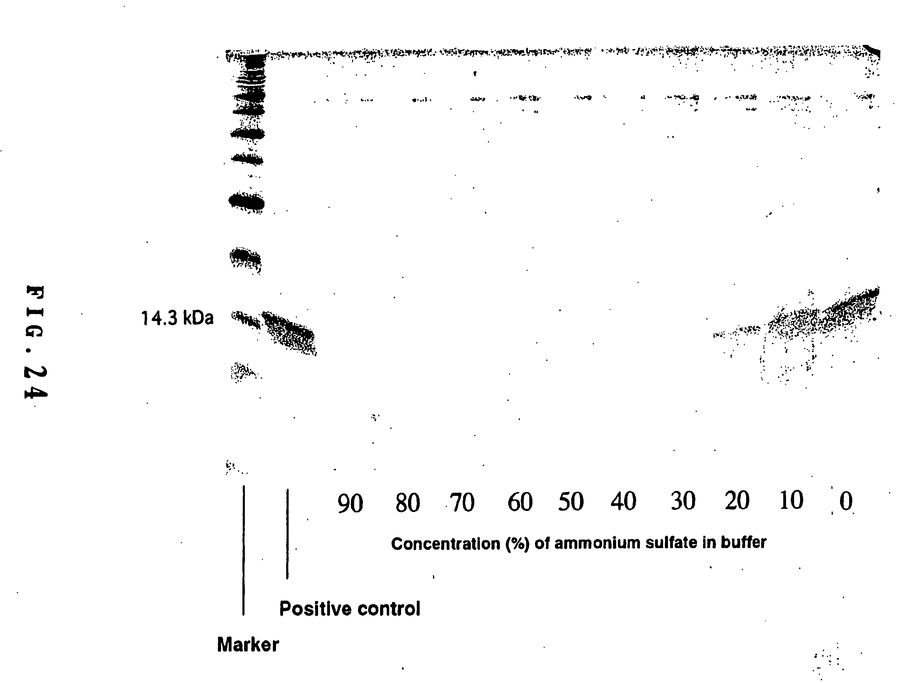

Ammonium sulfate was added to 10 mM Tris-HCl buffer (pH 8.0) to give ammonium sulfate solution of 0, 10, 20, 30, 40, 50, 60, 70, 80 and 90 % saturation, respectively. To each 1 ml buffer was added PBS powder (1 mm³) and suspended. PbpA purified sample (0.1 mg/ml) 100 µl was added to the resulting suspension and incubated with shaking (125 rpm) for 3 hours at 30°C for attachment. The mixture was passed through a filter (Millipore; 0.2µ pore size) to completely remove PBS, and the resulting filtrate was subjected to SDS-PAGE (Fig. 24).

The results showed that the presence of PbpA was confirmed in the filtrate of the ammonium sulfate solution of 0, 10 and 20 % saturation, but not in the filtrate of the solution of other saturations, indicating that PbpA attached to PBS in the presence of ammonium sulfate solution of 30 % saturation or more.

### (13-5) Genes of PbpA homologues

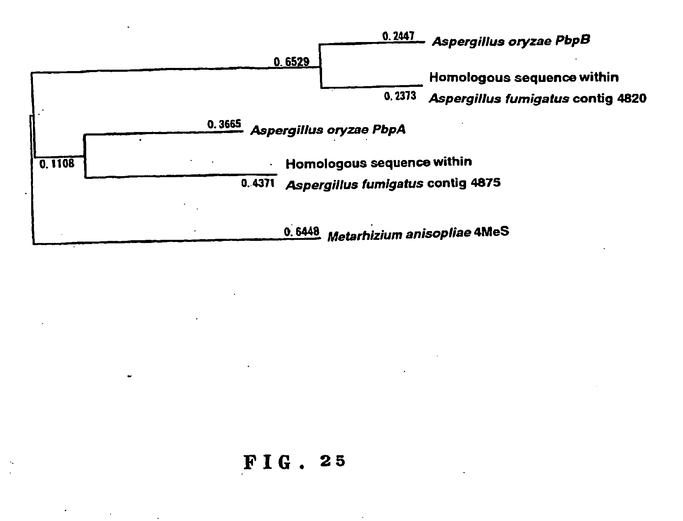

The homology research conducted on known DNA data bases revealed homologous genes in *Aspergillus fumigatus* and *Metarhizium anisopliae* (Fig.25). Although the function of these genes has never been even estimated, the above results on PbpA suggest that they also have the plastic-attaching function.

The information available at present about the annotation of the genes of the homologues of PbpA and PbpB in Fig. 25 is as follows:
*Aspergillus oryzae* PbpA
*Aspergillus oryzae* PbpB

These are described in the present specification.

The sequence within *Aspergillus fumigatus* contig 4875
Having homology to PbpA in base and amino acid sequences, and obtained from the data base of *Aspergillus fumigatus.* Its expression has not yet been confirmed, and its function is unknown.

The sequence within *Aspergillus fumigatus* contig 4820
Having homology to PbpB in base and amino acid sequences, and obtained from the data base of *Aspergillus fumigatus.* Its expression has not yet been confirmed, and its function is unknown.

### Metarhizium anisopliae 4MeS

ACCESSION No. AF012092 (Nucleotide), ACCESSION No. AAB69311

### (Protein)

Obtained from the cDNA in the culture of *Metarhizium anisopliae.* The culture conditions or its function are unknown.

### Example 14

### (14-1) Selective degradation of biodegradable plastic from its composite with other materials

Biodegradable plastic material is selectively degraded in its composite by the present degrading enzyme. The biodegradable plastic may be recovered as monomer and oligomer in a soluble fraction, and the other materials may be collected as well.

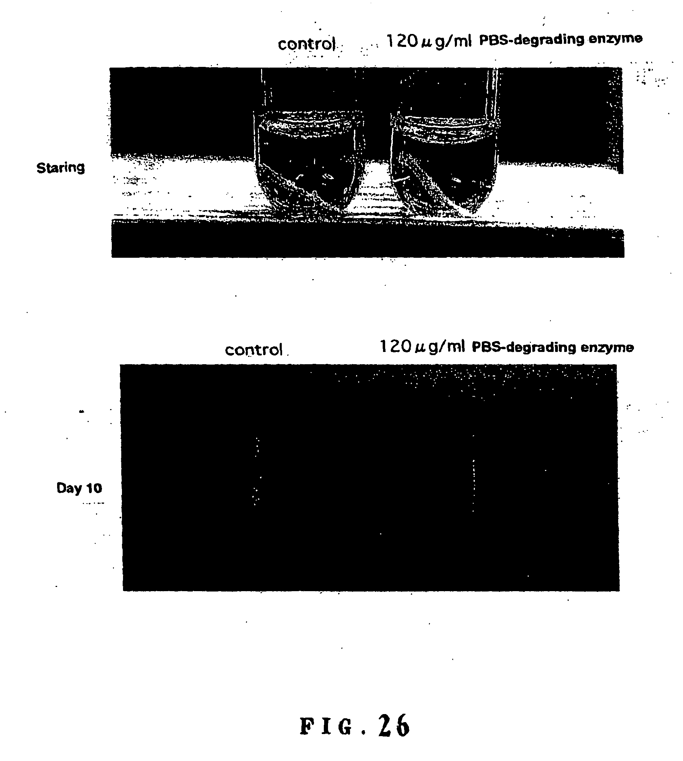

The composite material consisted of PBS as the biodegradable plastic and platinum string. The purified PBS-degrading enzyme sample (cutinase) described in Example 3 was used. PBS (4 g) was dissolved in 20 ml of chloroform in a 100 ml beaker to give a final concentration of 20 (w/v)%. A platinum string (φ 0.5 mm) was soaked in the resulting solution, and then dried to give a platinum string covered by PBS membrane. The platinum string covered by PBS membrane (0.5 mm x 8mm) was mixed with 600 µl of PBS-degrading enzyme solution (120 µg/ml) in 50 mM Tris-HCl buffer (pH 9.0) and incubated for 7 days at 37°C while being kept at pH 9.0 with 1 N NaOH. As a result, the PBS membrane was selectively degraded in the mixture containing the enzyme, and the metal piece was collected as solid substance (Fig.26). This result showed that the biodegradable plastic and the other component may be separately collected from their composite material.

### Example 15

### (15-1) Promoting effect of RoIA for the degradation of PBS film by cutinase

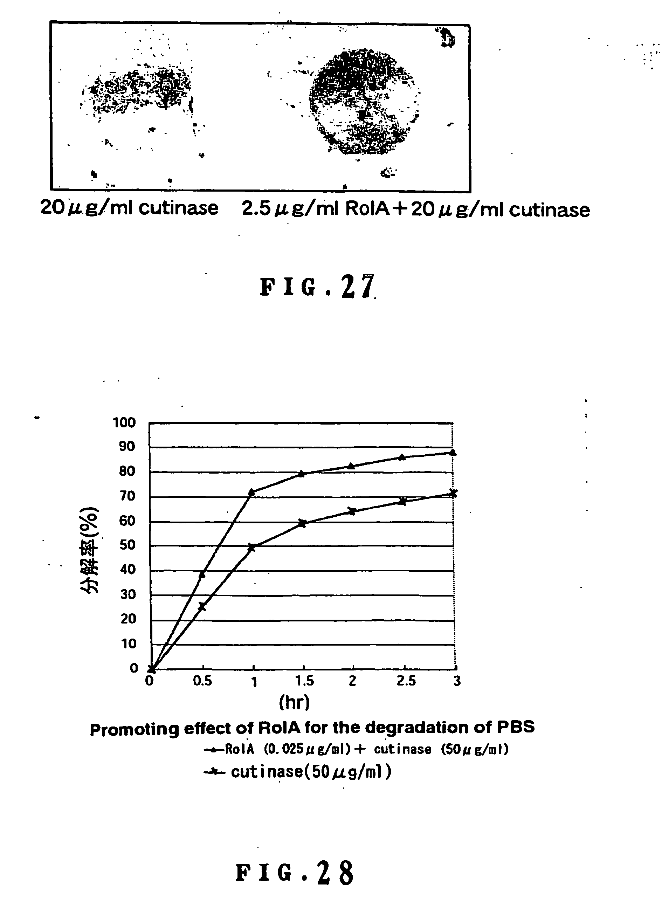

A piece of filter paper (Whatman:1 cm²) soaked with 10 µl of 10 mM Tris-HCl buffer, pH 8.0 containing RoIA (2.5 µg/ml) was placed on a piece of PBS film (76 x 26 mm) layered on a slide glass (MMATSUNAMI) of the same size, and incubated for 12 hours at 30°C. Another piece of filter paper soaked with 10 µl of the above buffer only was placed on the PBS film and used as a control in the same procedure. Ten µl of cutinase solution (20 µg/ml) was added to the filter paper, and incubated for 6 hours at 37°C for degrading reaction. After the degrading reaction, it was observed that a spot on which the filter paper had been placed had become turbid in white. The filter paper to which RoIA had attached showed a relatively high degree of turbidity, and the filter paper that had not been treated with RoIA showed a relatively low one. From a preliminary experiment, it had been already revealed that the higher the efficiency of degradation of PBS film by cutinase was, the more turbid in white their contact surface became. These results demonstrated that the degradation of PBS by cutinase could be promoted by RoIA (Fig.27). In Fig. 27, the black and white parts are shown vice versa, and the black parts show the degradation.

### (15-2) Promoting effect of RolA for the degradation of PBS emulsion by cutinase

### PBS emulsion (0.1 w/v) in an amount of 89.5 µl was put into a 96-well microtiter plate, 10 µl of RoIA solution (250 ng/ml) in 10 mM Tris-HCl buffer, pH 8.0 was added to each well and incubated for 12 hours at 30°C for RoIA to attach to PBS. 10 mM Tris-HCl buffer, pH 8.0 was added to 10 µl of PBS emulsion was used as a control.

After the attaching reaction, 0.5 µl of cutinase solution (1 mg/ml) in 10 mM Tris-HCl buffer, pH 8.0 was added and incubated for a degrading reaction at 40° C. Rate of PBS-degradation was calculated based on the turbidity of PBS by measuring absorbance at 630 nm every 30 min (Fig.28). As a result, a significant difference in PBS-degradation efficiency was confirmed, which in turn demonstrated a promoting effect of RoIA for the degradation of PBS in its emulsion.

### (15-3) Promoting effect of PbpA for the degradation of PBS film by cutinase

PbpA attached to PBS in a low water activity condition where ammonium sulfate concentration was 30 % or more. PbpA was therefore dried on PBS film in order to lower its water activity, so that PbpA could attach to the PBS film. PBS degradation was then carried out using the resulting PbpA-attaching PBS film.

A purified PbpA sample was diluted with water to a final concentration of 1.0, 5.0, 10, 50, 100 (µg/ml), respectively. Ten µl of PbpA solution with each concentration was spotted on the PBS film and completely dried at 37°C to promote the attachment of PbpA to PBS. Filter paper (φ 6mm) was placed on the PbpA attaching to the PBS film, and soaked with 10 µl of cutinase solution (20 µg/ml). The PBS film was then incubated for 6 hours at 37°C to observe the degradation of the PBS film.

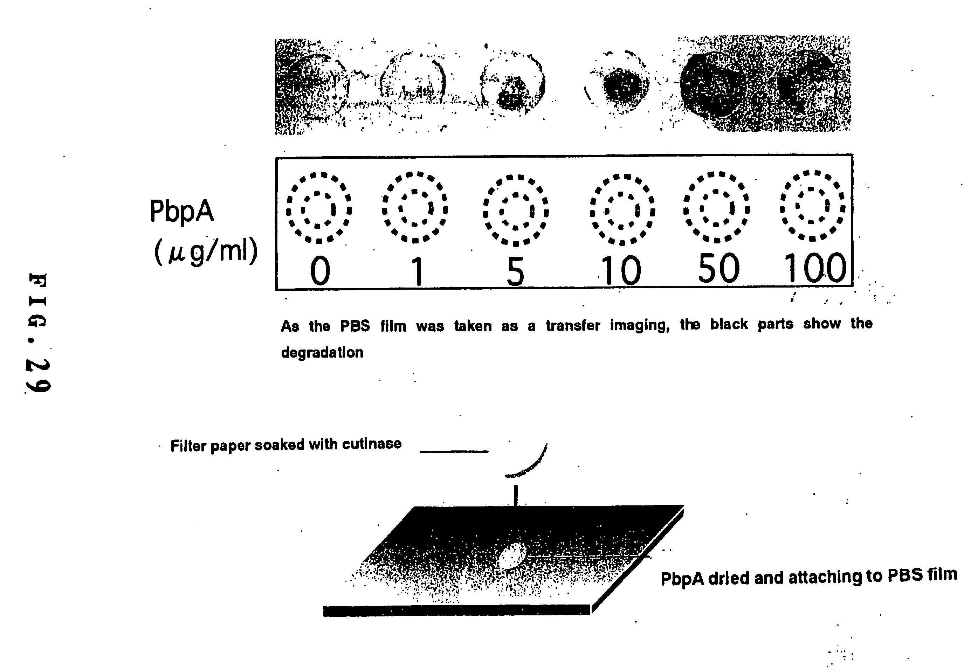

The significant degradation of PBS was observed in the whole parts of the filter paper where 100 and 50 (µg/ml) of PbpA had been spotted. Further, the significant degradation of PBS was observed in only the part of the filter paper where 10 and 5.0 (µg/ml) of PbpA had been just spotted (Fig.29). It was therefore demonstrated that the degradation by cutinase was promoted by the attachment of PbpA to PBS.

### (15-4) Comparison of a promoting activity for the degradation of PBS by cutinase between hydrophobin, PbpA and synthetic surfactant

It was confirmed that hydrophobin and PbpA promoted the degradation of PBS by cutinase. Their promoting activity was then compared with that of a synthetic surfactant, PLYSURF A210G (DAI-ICHI KOGYO SEIYAKU CO., LTD.).

Biosurfactant with a concentration that had been proven to show the promoting activity in Examples 15-1 and 15-3 (2.5 µg/ml for hydrophobin and 10 µg/ml for PbpA) was made attach onto PBS film in accordance with the methods of Examples 15-1 and 15-3. PLYSURF A210G of 2.5 µg/ml and 10 µg/ml was similarly made attach onto the PBS film. As a control comprising no biosurfactant, 10 mM Tris-HCl buffer (pH 8.0) and purified water were used for hydrophobin and PbpA, respectively, for the attaching treatment.

Filter paper (φ 6mm) was placed on the biosurfactant and PLYSURF A210G attaching to the PBS film, and soaked with 10 µl of cutinase solution (20 µg/ml). The PBS film was then incubated in accordance with the conditions in Examples 15-1 and 15-3 (12 hours at 30°C for hydrophobin, and 6 hours at 37° C for PbpA). After the reaction was completed, the PBS film was washed with purified water, dried and subjected to observation of the degradation of the PBS film.

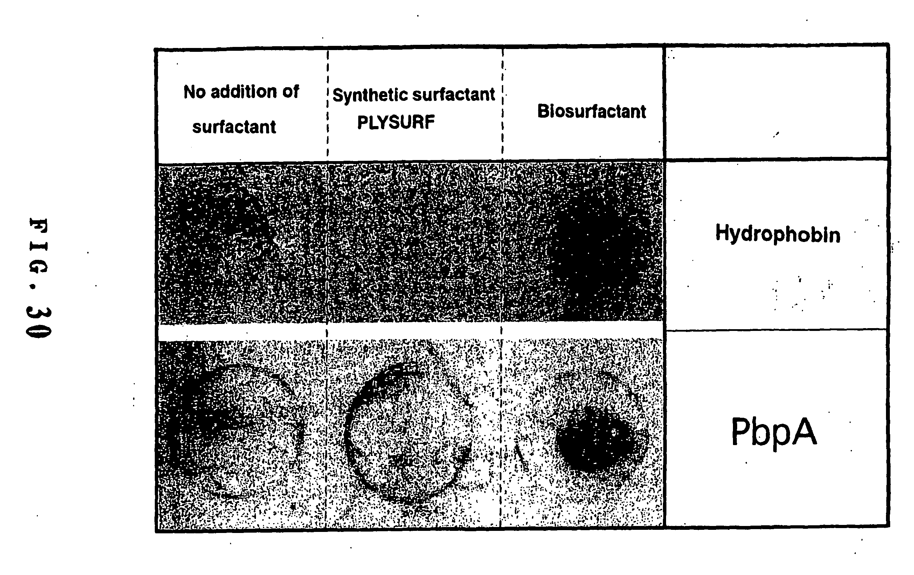

The significant degradation of PBS was observed in the parts of the filter paper where both the biosurfactants had been spotted (Fig.30), compared to the control. On the other hand, no significant degradation of PBS was observed in the part of the filter paper where PLYSURF A210G had been spotted (Fig.30). As the PBS film was taken as a transfer imaging, black and white parts are shown vice versa in Fig. 30, and the black parts show the degradation. It was demonstrated that the biosurfactant such as hydrophobin and PbpA could promote the degradation of PBS by cutinase more strongly than the synthetic surfactant such as PLYSURF A210G.

### [Industrial Applicability]

This invention is based on the findings that the biosurfactant such as the above plastic-binding protein attaches to a hydrophobic surface of the plastic and effectively promotes the degradation of the plastic in cooperation with the plastic-degrading enzyme.

Thus, the present invention provides a method of effectively degrading the plastic in the presence of the biosurfactant such as the plastic-binding protein. Especially, the present invention provides a method that makes it possible to effectively perform the degradation of high-density plastic in a large scale utilizing the microorganisms expressing the biosurfactant and the plastic-degrading enzyme, or the biosurfactant and/or plastic-degrading enzyme themselves, Furthermore, it provides a method, which may simultaneously produce a useful substance such as enzyme or protein and antibiotics with the use of the microorganism such as filamentous microorganisms and *Actinomycetes* that have a high productivity of the useful substance.

## Claims

1. A transformant prepared by recombination with the use of a DNA comprising a gene encoding a biosurfactant which is a plastic binding protein and a DNA comprising a gene encoding a plastic-degrading enzyme which is a cutinase.

2. A transformant of claim 1 wherein the biosurfactant is hydrophobin or a hydrophobin homologue derived from *Aspergillus oryzae.*

3. A transformant of claim 2 wherein said DNA is a DNA comprising a base sequence encoding the following:
(a) a polypeptide having an amino acid sequence that is the same or substantially the same as that represented by SEQ No.1, No.2 or No.3; or
(b) a polypeptide having an amino acid sequence of (a) wherein a part of amino acid residues are replaced, deleted, or added, and having substantially the same function as the hydrophobin.

4. A transformant of claim 2 wherein said DNA is:
(a) DNA comprising a base sequence represented by SEQ ID No.1, No.2 or No.3 or its partial sequence; or
(b) DNA being hybridized with a base sequence complementary to the DNA comprising the base sequence in (a) under stringent conditions, and having substantially the same function as the DNA (a).

5. A transformant of claim 1 wherein the biosurfactant is a plastic-binding protein derived from *Aspergillus oryzae.*

6. A transformant of claim 5 wherein said DNA is a DNA comprising a base sequence encoding the following:
(a) a polypeptide having an amino acid sequence that is the same or substantially the same as that represented by SEQ No.6 or No.7; or
(b) a polypeptide having an amino acid sequence of (a) wherein a part of amino acid residues are replaced, deleted, or added, and having substantially the same function as the plastic-binding protein.

7. A transformant of claim 5 wherein said DNA is:
(a) DNA comprising a base sequence represented by SEQ ID No. 6 or No.7 or its partial sequence; or
(b) DNA being hybridized with a base sequence complementary to the DNA comprising the base sequence in (a) under stringent conditions, and having substantially the same function as the DNA (a).

8. A transformant of claim 1 wherein the plastic-degrading enzyme is a cutinase from *Aspergillus oryzae.*

9. A transformant of claim 1 wherein said DNA is a DNA comprising a base sequence encoding the following:
(a) a polypeptide having an amino acid sequence that is the same or substantially the same as that represented by SEQ ID No.4 or No.5; or
(b) a polypeptide having an amino acid sequence of (a) wherein a part of amino acid residues are replaced, deleted, or added, and having substantially the same function as the plastic-degrading enzyme.

10. A transformant of claim 1 wherein said DNA is:
(a) DNA comprising a base sequence represented by SEQ ID No.4 or No.5 or its partial sequence; or
(b) DNA being hybridized with a base sequence complementary to the DNA comprising the base sequence in (a) under stringent conditions, and having substantially the same function as the DNA (a).

11. A transformant of any one of claims 1-10 which is further prepared by recombination with the use of DNA comprising a gene encoding a useful substance.

12. A transformant of claim 1 prepared by recombination with the use of the DNA comprising the gene encoding the hydrophobin derived from *Aspergillus oryzae,* the DNA comprising the gene encoding the cutinase derived from *Aspergillus oryzae,* and a DNA comprising a gene encoding α amylase.

13. A transformant of any one of claims 1-12 wherein at least one of the DNA comprising the gene encoding the biosurfactant and the DNA comprising the gene encoding the plastic-degrading enzyme is expressed under the control of a promoter derived from another gene.

14. A transformant of any one of claims 1-13, which is an eukaryotic filamentous fungus selected from the genera *of Aspergillus, Penicillium, Trichodera, Rhizopus, Magnaporthe, Metarhisium, Neurospora, Monascus, Acremonium* and *Mucor.*

15. A transformant of claim 14 which is of the genus *Aspergillus.*

16. A transformant of claim 15 which is *Aspergillus oryzae.*

17. A method of degrading biodegradable plastic using the transformant of any one of claims 1-16.

18. A method of degrading biodegradable plastic which is **characterized by** co-culture of a transformant prepared by recombination with the use of a DNA comprising a gene encoding a biosurfactant which is a plastic binding protein, and a transformant prepared by recombination with the use of a DNA comprising a gene encoding a plastic-degrading enzyme which is a cutinase.

19. A method of producing a useful substance from biodegradable plastic comprising degrading the plastic using the transformant of any one of claims 1-16 and further converting a component of the degraded plastic into a useful substance.

20. A method of producing a useful substance from biodegradable plastic comprising degrading the plastic and further converting a component of the degraded plastic into a useful substance, the method being **characterized by** using triple co-culture of
(i) a transformant prepared by recombination with the use of a DNA comprising a gene encoding a biosurfactant which is a plastic binding protein;
(ii) a transformant prepared by recombination with the use of a DNA comprising a gene encoding a plastic-degrading enzyme which is a cutinase;
(iii) a transformant prepared by recombination with the use of a DNA comprising a gene encoding an enzyme involved in biosynthesis.

21. A method of claim 19 wherein the useful substance is selected from protein, a first metabolite, a second metabolite and a biosurfactant.

22. A method of claim 19 wherein the useful substance is an extracellularly secreted substance.

23. Use of a transformant prepared by recombination with the use of a DNA comprising a gene encoding a biosurfactant which is a plastic binding protein or a DNA comprising a gene encoding a plastic degrading enzyme which is a cutinase in a method of degrading biodegradable plastic with the plastic degrading enzyme in the presence of the biosurfactant.

## Patentansprüche

1. Transformant, hergestellt durch Rekombination mit der Verwendung einer DNA, die ein Gen umfasst, welches ein biologisches grenzflächenaktives Mittel kodiert, das ein Kunststoff-bindendes Protein ist, und einer DNA, die ein Gen umfasst, welches ein Kunststoff-abbbauendes Enzym kodiert, das eine Cutinase ist.

2. Transformant nach Anspruch 1, wobei das biologische grenzflächenaktive Mittel Hydrophobin oder ein Hydrophobinhomologes, abgeleitet von *Aspergillus oryzae,* ist.

3. Transformant nach Anspruch 2, wobei die DNA eine DNA ist, welche eine Basensequenz umfasst, die das Folgende kodiert:
(a) ein Polypeptid mit einer Aminosäuresequenz, welche gleich oder im Wesentlichen gleich ist mit diejenigen, welche durch SEQ Nr. 1, Nr. 2 oder Nr. 3 dargestellt wird; oder
(b) ein Polypeptid mit einer Aminosäuresequenz von (a), wobei ein Teil der Aminosäurereste ersetzt, deletiert oder hinzugefügt ist und mit im Wesentlichen der gleichen Funktion wie das Hydrophobin.

4. Transformant nach Anspruch 2, wobei die DNA ist:
(a) DNA, welche eine Basensequenz, die durch SEQ ID Nr. 1, Nr. 2 oder Nr. 3 dargestellt wird, oder eine Teilsequenz davon umfasst; oder
(b) DNA, welche unter stringenten Bedingungen mit einer Basensequenz hybridisiert, die komplementär zu der DNA, welche die Basensequenz in (a) umfasst, ist, und mit im Wesentlichen der gleichen Funktion wie die DNA (a).

5. Transformant nach Anspruch 1, wobei das biologische grenzflächenaktive Mittel ein Kunststoff-bindendes Protein, abgeleitet von *Aspergillus oryzae,* ist.

6. Transformant nach Anspruch 5, wobei die DNA eine DNA ist, welche eine Basensequenz umfasst, die das Folgende kodiert:
(a) ein Polypeptid mit einer Aminosäuresequenz, welche gleich oder im Wesentlichen gleich ist mit diejenigen, welche durch SEQ Nr. 6 oder Nr. 7 dargestellt wird; oder
(b) ein Polypeptid mit einer Aminosäuresequenz von (a), wobei ein Teil der Aminosäurereste ersetzt, deletiert oder hinzugefügt ist und mit im Wesentlichen der gleichen Funktion wie das Kunststoff-bindende Protein.

7. Transformant nach Anspruch 5, wobei die DNA ist:
(a) DNA, welche eine Basensequenz, die durch SEQ ID Nr. 6 oder Nr. 7 dargestellt wird, oder eine Teilsequenz davon umfasst; oder
(b) DNA, welche unter stringenten Bedingungen mit einer Basensequenz hybridisiert, die komplementär zu der DNA, welche die Basensequenz in (a) umfasst, ist, und mit im Wesentlichen der gleichen Funktion wie die DNA (a).

8. Transformant nach Anspruch 1, wobei das Kunststoff-abbauende Enzym eine Cutinase von *Aspergillus oryzae* ist.

9. Transformant nach Anspruch 1, wobei die DNA eine DNA ist, welche eine Basensequenz umfasst, die das Folgende kodiert:
(a) ein Polypeptid mit einer Aminosäuresequenz, welche gleich oder im Wesentlichen gleich ist mit diejenigen, welche durch SEQ ID Nr. 4 oder Nr. 5 dargestellt wird; oder
(b) ein Polypeptid mit einer Aminosäuresequenz von (a), wobei ein Teil der Aminosäurereste ersetzt, deletiert oder hinzugefügt ist und mit im Wesentlichen der gleichen Funktion wie das Kunststoff-abbauende Enzym.

10. Transformant nach Anspruch 1, wobei die DNA ist:
(a) DNA, welche eine Basensequenz, die durch SEQ ID Nr. 4 oder Nr. 5 dargestellt wird, oder eine Teilsequenz davon umfasst; oder
(b) DNA, welche unter stringenten Bedingungen mit einer Basensequenz hybridisiert, die komplementär zu der DNA, welche die Basensequenz in (a) umfasst, ist, und mit im Wesentlichen der gleichen Funktion wie die DNA (a).

11. Transformant nach einem der Ansprüche 1 bis 10, welcher ferner durch Rekombination mit der Verwendung von DNA, die ein Gen umfasst, welches eine nützliche Substanz kodiert, hergestellt wird.

12. Transformant nach Anspruch 1, hergestellt durch Rekombination mit der Verwendung der DNA, die das Gen umfasst, welches das von *Aspergillus oryzae* abgeleitete Hydrophobin kodiert, der DNA, die das Gen umfasst, welches die von *Apergillus oryzae* abgeleitete Cutinase kodiert, und einer DNA, die ein Gen umfasst, welches α-Amylase kodiert.

13. Transformant nach einem der Ansprüche 1 bis 12, wobei mindestens eine der DNA, die das Gen umfasst, welches das biologische grenzflächenaktive Mittel kodiert, und der DNA, die das Gen umfasst, welches das Kunststoff-abbauende Enzym kodiert, unter der Kontrolle eines Promotors, welcher von einem anderen Gen abgeleitet ist, exprimiert wird.

14. Transformant nach einem der Ansprüche 1 bis 13, welcher ein eukaryotischer Fadenpilz, ausgewählt aus den Gattungen *Aspergillus, Penicillium, Trichodera, Rhizopus, Magnaporthe, Metarhisium, Neurospora, Monascus, Acremonium* und *Mucor,* ist.

15. Transformant nach Anspruch 14, welcher von der Gattung *Aspergillus* ist.

16. Transformant nach Anspruch 15, welcher *Aspergillus oryzae* ist.

17. Verfahren zum Abbauen von bioabbaubarem Kunststoff unter Verwendung des Transformanten nach einem der Ansprüche 1 bis 16.

18. Verfahren zum Abbauen von bioabbaubarem Kunststoff, welches durch Cokultivierung eines Transformanten, hergestellt durch Rekombination mit der Verwendung einer DNA, die ein Gen umfasst, welches ein biologisches grenzflächenaktives Mittel kodiert, das ein Kunststoff-bindendes Protein ist, und eines Transformanten, hergestellt durch Rekombination mit der Verwendung einer DNA, die ein Gen umfasst, welches ein Kunststoff-abbauendes Enzym kodiert, das eine Cutinase ist, **gekennzeichnet** ist.

19. Verfahren zum Herstellen einer nützlichen Substanz aus bioabbaubarem Kunststoff, umfassend das Abbauen des Kunststoffes unter Verwendung des Transformanten nach einem der Ansprüche 1 bis 16 und ferner das Umwandeln einer Komponente des abgebauten Kunststoffes in eine nützliche Substanz.

20. Verfahren zum Herstellen einer nützlichen Substanz aus bioabbaubarem Kunststoff, umfassend das Abbauen des Kunststoffes und ferner das Umwandeln einer Komponente des abgebauten Kunststoffes in eine nützliche Substanz, wobei das Verfahren **gekennzeichnet ist durch** die Verwendung einer Dreifach-Cokultivierung
(i) n: einem Transformanten, hergestellt **durch** Rekombination mit der Verwendung einer DNA, die ein Gen umfasst, welches ein biologisches grenzflächenaktives Mittel kodiert, das ein Kunststoff-bindendes Protein ist;
(ii) einem Transformanten, hergestellt **durch** Rekombination mit der Verwendung einer DNA, die ein Gen umfasst, welches ein Kunststoff-abbauendes Enzym kodiert, das eine Cutinase ist;
(iii) einem Transformanten, hergestellt **durch** Rekombination mit der Verwendung einer DNA, die ein Gen umfasst, welches ein Enzym kodiert, das in Biosynthese einbezogen ist.

21. Verfahren nach Anspruch 19, wobei die nützliche Substanz aus Protein, einem ersten Metaboliten, einem sekundären Metaboliten und einem biologischen grenzflächenaktiven Mittel ausgewählt ist.

22. Verfahren nach Anspruch 19, wobei die nützliche Substanz eine extrazellulär sekretierte Substanz ist.

23. Verwendung eines Transformanten, hergestellt durch Rekombination mit der Verwendung einer DNA, die ein Gen umfasst, welches ein biologisches grenzflächenaktives Mittel kodiert, das ein Kunststoff-bindendes Protein ist, oder einer DNA, die ein Gen umfasst, welches ein Kunststoff-abbauendes Enzym kodiert, das eine Cutinase ist, in einem Verfahren zum Abbauen von bioabbaubarem Kunststoff mit dem Kunststoff-abbauenden Enzym in der Gegenwart des biologischen grenzflächenaktiven Mittels.

## Revendications

1. Organisme transformé, préparé par recombinaison au moyen d'un ADN qui comprend un gène codant un biosurfactant qui est une protéine fixant les plastiques et d'un ADN qui comprend un gène codant une enzyme qui dégrade les plastiques et qui est une cutinase.

2. Organisme transformé conforme à la revendication 1, dans lequel le biosurfactant est de l'hydrophobine ou un homologue d'hydrophobine, dérivé(e) *d'Aspergillus oryzae.*

3. Organisme transformé conforme à la revendication 2, dans lequel ledit ADN est un ADN qui comprend une séquence de bases codant ce qui suit :
a) un polypeptide dont la séquence d'acides aminés est la même ou sensiblement la même que celle qui est présentée en tant que Séquence N° 1, N° 2 ou N° 3 ;
b) ou un polypeptide présentant une séquence d'acides aminés indiquée en (a), où il y a remplacement, délétion ou addition d'une partie des résidus d'acides aminés, mais qui possède sensiblement la même fonction que l'hydrophobine.

4. Organisme transformé conforme à la revendication 2, dans lequel ledit ADN est :
a) un ADN comprenant une séquence de bases présentée en tant que Séquence N° 1, N° 2 ou N° 3, ou une partie d'une telle séquence ;
b) ou un ADN qui s'hybride, dans des conditions stringentes, avec une séquence de bases complémentaire de l'ADN comprenant une séquence de bases indiquée en (a) et qui possède sensiblement la même fonction que l'ADN indiqué en (a).

5. Organisme transformé conforme à la revendication 1, dans lequel le biosurfactant est une protéine fixant les plastiques, dérivée *d'Aspergillus oryzae.*

6. Organisme transformé conforme à la revendication 5, dans lequel ledit ADN est un ADN qui comprend une séquence de bases codant ce qui suit :
a) un polypeptide dont la séquence d'acides aminés est la même ou sensiblement la même que celle qui est présentée en tant que Séquence N° 6 ou N° 7 ;
b) ou un polypeptide présentant une séquence d'acides aminés indiquée en (a), où il y a remplacement, délétion ou addition d'une partie des résidus d'acides aminés, mais qui possède sensiblement la même fonction que la protéine fixant les plastiques.

7. Organisme transformé conforme à la revendication 5, dans lequel ledit ADN est :
a) un ADN comprenant une séquence de bases présentée en tant que Séquence N° 6 ou N° 7, ou une partie d'une telle séquence ;
b) ou un ADN qui s'hybride, dans des conditions stringentes, avec une séquence de bases complémentaire de l'ADN comprenant une séquence de bases indiquée en (a) et qui possède sensiblement la même fonction que l'ADN indiqué en (a).

8. Organisme transformé conforme à la revendication 1, dans lequel l'enzyme dégradant les plastiques est une cutinase dérivée *d'Aspergillus oryzae.*

9. Organisme transformé conforme à la revendication 1, dans lequel ledit ADN est un ADN qui comprend une séquence de bases codant ce qui suit :
a) un polypeptide dont la séquence d'acides aminés est la même ou sensiblement la même que celle qui est présentée en tant que Séquence N° 4 ou N° 5 ;
b) ou un polypeptide présentant une séquence d'acides aminés indiquée en (a), où il y a remplacement, délétion ou addition d'une partie des résidus d'acides aminés, mais qui possède sensiblement la même fonction que l'enzyme dégradant les plastiques.

10. Organisme transformé conforme à la revendication 1, dans lequel ledit ADN est :
a) un ADN comprenant une séquence de bases présentée en tant que Séquence N° 4 ou N° 5, ou une partie d'une telle séquence ;
b) ou un ADN qui s'hybride, dans des conditions stringentes, avec une séquence de bases complémentaire de l'ADN comprenant une séquence de bases indiquée en (a) et qui possède sensiblement la même fonction que l'ADN indiqué en (a).

11. Organisme transformé conforme à l'une des revendications 1 à 10, qui est en outre préparé par recombinaison à l'aide d'un ADN qui comprend un gène codant une substance utile.

12. Organisme transformé conforme à la revendication 1, préparé par recombinaison à l'aide d'un ADN qui comprend le gène codant l'hydrophobine dérivée *d'Aspergillus oryzae,* d'un ADN qui comprend le gène codant la cutinase dérivée *d'Aspergillus oryzae,* et d'un ADN qui comprend un gène codant une alpha-amylase.

13. Organisme transformé conforme à l'une des revendications 1 à 12, dans lequel au moins l'un de l'ADN qui comprend un gène codant un biosurfactant et de l'ADN qui comprend un gène codant une enzyme qui dégrade les plastiques est exprimé sous le contrôle d'un promoteur dérivé d'un autre gène.

14. Organisme transformé conforme à l'une des revendications 1 à 13, qui est un champignon filamentaire eucaryote choisi parmi les genres *Aspergillus, Penicillium, Trichodera, Rhizopus, Magnaporthe, Neurospora, Metarhisium, Monascus, Acremonium* et *Mucor.*

15. Organisme transformé conforme à la revendication 14, appartenant au genre *Aspergillus.*

16. Organisme transformé conforme à la revendication 15, qui est une souche *d'Aspergillus oryzae.*

17. Procédé de dégradation de plastiques biodégradables à l'aide d'un organisme transformé conforme à l'une des revendications 1 à 16.

18. Procédé de dégradation de plastiques biodégradables, **caractérisé en ce qu'**on cultive conjointement un organisme transformé préparé par recombinaison au moyen d'un ADN qui comprend un gène codant un biosurfactant qui est une protéine fixant les plastiques, et un organisme transformé préparé par recombinaison au moyen d'un ADN qui comprend un gène codant une enzyme qui dégrade les plastiques et qui est une cutinase.

19. Procédé de production d'une substance utile à partir d'un plastique biodégradable, qui comporte le fait de dégrader le plastique à l'aide d'un organisme transformé conforme à l'une des revendications 1 à 16, et le fait de convertir en une substance utile un composant du plastique dégradé.

20. Procédé de production d'une substance utile à partir d'un plastique biodégradable, qui comporte le fait de dégrader le plastique et le fait de convertir en une substance utile un composant du plastique dégradé, lequel procédé est **caractérisé en ce qu'**on a recours à une triple co-culture où l'on cultive conjointement :
i) un organisme transformé préparé par recombinaison au moyen d'un ADN qui comprend un gène codant un biosurfactant qui est une protéine fixant les plastiques,
ii) un organisme transformé préparé par recombinaison au moyen d'un ADN qui comprend un gène codant une enzyme qui dégrade les plastiques et qui est une cutinase,
iii) et un organisme transformé préparé par recombinaison au moyen d'un ADN qui comprend un gène codant une enzyme impliquée dans une biosynthèse.

21. Procédé conforme à la revendication 19, dans lequel la substance utile est choisie parmi une protéine, un premier métabolite, un deuxième métabolite et un biosurfactant.

22. Procédé conforme à la revendication 19, dans lequel la substance utile est une substance sécrétée hors de la cellule.

23. Utilisation d'un organisme transformé préparé par recombinaison au moyen d'un ADN qui comprend un gène codant un biosurfactant qui est une protéine fixant les plastiques, ou au moyen d'un ADN qui comprend un gène codant une enzyme qui dégrade les plastiques et qui est une cutinase, dans un procédé de dégradation de plastiques biodégradables à l'aide de l'enzyme qui dégrade les plastiques et en présence du biosurfactant.
